(19) **Europäisches Patentamt European Patent Office Office européen des brevets**

(11) **EP 3 825 417 A2**

(12) **EUROPEAN PATENT APPLICATION**

(43) Date of publication:
26.05.2021 Bulletin 2021/21

(21) Application number: 20193121.9

(22) Date of filing: 22.05.2015

(51) Int Cl.:
C12Q 1/6881 (2018.01)          C12Q 1/6883 (2018.01)
G01N 33/53 (2006.01)           G01N 33/68 (2006.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 22.05.2014  US 201462001909 P
22.05.2014  US 201462001889 P
22.05.2014  US 201462001902 P
25.07.2014  US 201462029038 P
09.09.2014  US 201414481167
09.09.2014  PCT/US2014/054735

(62) Document number(s) of the earlier application(s) in
accordance with Art. 76 EPC:
15795618.6 / 3 146 077

(71) Applicants:
• **The Scripps Research Institute**
**La Jolla, California 92037 (US)**
• **Northwestern University**
**Evanston, IL 60208 (US)**

(72) Inventors:
• **SALOMON, Daniel**
**San Diego, California 92130 (US)**
• **KURIAN, Sunil**
**San Diego, California 92126 (US)**
• **ABECASSIS, Michael**
**Highland Park, Illinois 60035 (US)**

(74) Representative: **Hutter, Anton et al**
**Venner Shipley LLP**
**5 Stirling House**
**Stirling Road**
**The Surrey Research Park**
**Guildford GU2 7RF (GB)**

Remarks:
•This application was filed on 27.08.2020 as a
divisional application to the application mentioned
under INID code 62.
•Claims filed after the date of filing of the application
/ after the date of receipt of the divisional application
(Rule 68(4) EPC).

(54) **TISSUE MOLECULAR SIGNATURES OF KIDNEY TRANSPLANT REJECTIONS**

(57)     By a genome-wide gene analysis of expression profiles of known or putative gene sequences in kidney biopsy samples, the present inventors have identified a consensus set of gene expression-based molecular biomarkers for distinguishing kidney transplantation patients who have Acute Rejection (AR), Acute Dysfunction No Rejection (ADNR), Chronic Allograft Nephropathy (CAN), or Transplant Excellent/Normal (TX). These molecular biomarkers are useful for diagnosis, prognosis and monitoring of transplantation patients.

**Figure 1**

EP 3 825 417 A2

**Description**

**CROSS-REFERENCE TO RELATED APPLICATIONS**

This application claims the benefit of priority to U.S. Application No. 14/481,167, filed September 9, 2014; to International Application No. PCT/US2014/054735, filed September 9, 2014; to U.S. Provisional Application No. 62/029,038, filed July 25, 2014; to U.S. Provisional Application No. 62/001,889, filed May 22, 2014; to U.S. Provisional Application No.62/001,902, filed May 22, 2014; and to U.S. Provisional Application No.62/001,909, filed May 22, 2014, each of which is incorporated by reference herein in their entirety.

**COPYRIGHT NOTIFICATION**

**STATEMENT CONCERNING GOVERNMENT SUPPORT**

This invention was made in part with the U.S. government support by the National Institutes of Health Grant No. A1063603. The U.S. Government therefore may have certain rights in the invention.

**BACKGROUND OF THE INVENTION**

Kidney transplantation offers a significant improvement in life expectancy and quality of life for patients with end stage renal disease. Unfortunately, graft losses due to allograft dysfunction or other uncertain etiologies have greatly hampered the therapeutic potential of kidney transplantation. Currently, immune reactivity of kidney transplant recipients is estimated by monitoring the levels of immunosuppressive drugs, and by functional and/or histological evaluation of the allograft. Diagnosis of acute rejection relies on clinical data (e.g., patient signs and symptoms) and laboratory data such as tissue biopsy. The laboratory pathologist generally seeks three main histological signs: (1) infiltrating T cells, perhaps accompanied by infiltrating eosinophils, plasma cells, and neutrophils, particularly in telltale ratios, (2) structural compromise of tissue anatomy, varying by tissue type transplanted, and (3) injury to blood vessels.
There is a need in the art for alternative and more effective means that can diagnose and directly quantify the extent of the recipient's immune response towards the allograft. Such means would help clinicians to customize the prescription of immunosuppressive drugs to individual patients. The present invention addresses this and other unfulfilled needs in the art.

**SUMMARY OF THE INVENTION**

In one aspect, the invention provides methods of prognosing, detecting, diagnosing or monitoring a kidney transplant rejection or injury, or lack thereof in a subject. These methods may involve obtaining nucleic acids of interest, and then (a) determining expression levels in a subject of at least 5 genes selected from the genes listed in Table 7, Table 8, Table 9, Table 10, or Table 11; and (b) detecting, prognosing, diagnosing or monitoring from the expression levels of the genes an ongoing transplant rejection or injury, or lack thereof in the subject. In some embodiments, the nucleic acids of interest comprise mRNA extracted from a sample from a subject or nucleic acids derived from the mRNA extracted from the sample from the subject. In some embodiments, the nucleic acids are contacted with probes, wherein the probes are specific for the at least five genes. In some preferred embodiments, step (a) is performed on a biopsy sample of the subject, particularly a kidney biopsy of the subject.
Some of the methods are directed to subjects who have acute rejection (AR), acute dysfunction no rejection (ADNR), chronic allograft nephropathy (CAN), or well-functioning normal transplant (TX). In some of the methods, for each of the at least five genes, step (b) entails comparing the expression level of the gene in the subject to one or more reference expression levels of the gene associated with AR, ADNR, CAN, or TX. In some methods, step (b) further involves for each of the at least five genes assigning the expression level of the gene in the subject a value or other designation which can provide an indication whether the subject has AR, ADNR, CAN, or TX. In some of these methods, the expression level of each of the at least five genes is assigned a value on a normalized scale of values associated with a range of expression levels in kidney transplant patients with AR, ADNR, CAN, or TX. In some methods, the expression level of each of the at least five genes is assigned a value or other designation in order to provide an indication that the subject has or is at risk of AR, ADNR, CAN, has well-functioning normal transplant, or that the expression level is uninformative. In some methods, step (b) further entails combining the values or designations for each of the genes to provide a combined value or designation which can provide an indication whether the subject has or is at risk of AR, ADNR, CAN, or has well-functioning normal transplant (TX).

In some methods of the invention, the steps are repeated at different times on the subject. Some of these methods are directed to subjects who are receiving a drug. In some of these methods, a change in the combined value or designation over time provides an indication of the effectiveness of the drug. In some methods of the invention, the subject has undergone a kidney transplant within 1 month, 3 months, 1 year, 2 years, 3 years or 5 years of performing step (a). In some methods, step (a) is performed on at least 10, 20, 40, or 100 genes. Some of the methods further include changing the treatment regime of the subject responsive to the prognosing, detecting, diagnosing or monitoring step. In some of these methods, the subject has received a drug before performing the methods, and the change involves administering an additional drug or administering a higher dose of the same drug, or administering a lower dose of the same drug, or stopping administering the same drug.

Some methods of the invention utilize the genes listed in Table 7 for prognosing or diagnosing subjects who have AR, ADNR, CAN, or TX. Some other methods of the invention utilize the genes listed in Table 8 for prognosing or diagnosing subjects who have AR, ADNR, or TX. Still some other methods of the invention utilize the genes listed in Table 9 for prognosing or diagnosing subjects who have CAN or TX. Still some other methods of the invention utilize the genes listed in Table 10 for prognosing or diagnosing subjects who have AR or TX. Still some other methods of the invention utilize the genes listed in Table 11 for prognosing or diagnosing subjects who have CAN/IFTA or TX. In some methods, expression levels of the genes are determined at the mRNA level or at the protein level. In some methods, step (b) is performed by a computer.

In another aspect, the invention provides an array which contains a support or supports bearing a plurality of nucleic acid probes complementary to a plurality of mRNAs fewer than 5000 in number. Typically, the plurality of mRNAs includes mRNAs expressed by at least five genes selected from Table 7, Table 8, Table 9, Table 10, or Table 11. Some of the arrays contain a plurality of mRNAs that are fewer than 1000 or fewer than 100 in number. In some arrays, the plurality of nucleic acid probes are attached to a planar support or to beads. In a related aspect, the invention provides an array which contains support or supports bearing a plurality of ligands that specifically bind to a plurality of proteins fewer than 5000 in number. Typically, the plurality of proteins include at least five proteins encoded by genes selected from Table 7, Table 8, Table 9, Table 10, or Table 11. Some of these arrays contain ligands that specifically bind to a plurality of proteins that are fewer than 1000 or fewer than 100 in number. In some arrays, the plurality of ligands are attached to a planar support or to beads. In some of the arrays, the ligands are different antibodies that bind to different proteins of the plurality of proteins.

In another aspect, the invention provides methods of expression analysis. The methods involve determining expression levels of up to 5000 genes in a sample from a subject having a kidney transplant. Typically, the genes include at least 5 genes selected from Table 7, Table 8, Table 9, Table 10, or Table 11. In some methods, the expression levels of up to 100 or 1000 genes are determined. In some methods, the expression levels are determined at the mRNA level or at the protein level. In some methods, the expression levels are determined by quantitative PCR or hybridization to an array or RNA sequencing.

In still another aspect, the invention provides methods of screening a compound for activity in inhibiting or treating a kidney transplant rejection or injury. The methods involve (a) administering the compound to a subject having or at risk of developing a kidney transplant rejection; (b) determining expression levels of at least five genes in the subject selected from Tables 1-11 and species variants thereof before and after administering the compound to the subject, (c) determining whether the compound has activity in inhibiting or treating the kidney transplant rejection from a change in expression levels of the genes after administering the compound. In some of these methods, the kidney transplant rejection or injury to be treated or inhibited is AR, ADNR, or CAN. In some methods, step (c) involves for each of the at least five changes assigning a value or designation depending on whether the change in the expression level of the gene relative to one or more reference levels indicating presence or absence of the kidney transplant rejection. Some of these methods further entail determining a combined value or designation for the at least five genes from the values or designations determined for each gene. Some of the methods employ subjects who are human or nonhuman animal models of the kidney transplant rejection.

In another aspect, the methods disclosed herein have an error rate of less than about 40%. In some embodiments, the method has an error rate of less than about 40%, 35%, 30%, 25%, 20%, 15%, 10%, 5%, 3%, 2%, or 1%. For example, the method has an error rate of less than about 10%. In some embodiments, the methods disclosed herein have an accuracy of at least about 60%, 65%, 70%, 75%, 80%, 85%, 90%, 95%, or 99%. For example, the method has an accuracy of at least about 70%. In some embodiments, the methods disclosed herein have a sensitivity of at least about 60%, 65%, 70%, 75%, 80%, 85%, 90%, 95%, or 99%. For example, the method has a sensitivity of at least about 80%. In some embodiments, the methods disclosed herein have a positive predictive value of at least about 60%, 65%, 70%, 75%, 80%, 85%, 90%, 95%, or 99%. In some embodiments, the methods disclosed herein have a negative predictive value of at least about 60%, 65%, 70%, 75%, 80%, 85%, 90%, 95%, or 99%.

In some embodiments, the gene expression products described herein are RNA (e.g., mRNA). In some embodiments, the gene expression products are polypeptides. In some embodiments, the gene expression products are DNA complements of RNA expression products from the transplant recipient.

In an embodiment, the algorithm described herein is a trained algorithm. In another embodiment, the trained algorithm is trained with gene expression data from biological samples from at least three different cohorts. In another embodiment, the trained algorithm comprises a linear classifier. In another embodiment, the linear classifier comprises one or more linear discriminant analysis, Fisher's linear discriminant, Naive Bayes classifier, Logistic regression, Perceptron, Support vector machine (SVM) or a combination thereof. In another embodiment, the algorithm comprises a Diagonal Linear Discriminant Analysis (DLDA) algorithm. In another embodiment, the algorithm comprises a Nearest Centroid algorithm. In another embodiment, the algorithm comprises a Random Forest algorithm or statistical bootstrapping. In another embodiment, the algorithm comprises a Prediction Analysis of Microarrays (PAM) algorithm. In another embodiment, the algorithm is not validated by a cohort-based analysis of an entire cohort. In another embodiment, the algorithm is validated by a combined analysis with an unknown phenotype and a subset of a cohort with known phenotypes.

In another aspect, the assay is a microarray, SAGE, blotting, RT-PCR, sequencing and/or quantitative PCR assay. In another embodiment, the assay is a microarray assay. In another embodiment, the microarray assay comprises the use of an Affymetrix Human Genome U133 Plus 2.0 GeneChip. In another embodiment, the mircroarray uses the Hu133 Plus 2.0 cartridge arrays plates. In another embodiment, the microarray uses the HT HG-U133+ PM array plates. In another embodiment, the assay is a sequencing assay. In another embodiment, the assay is a RNA sequencing assay.

In some embodiments, the transplant recipient has a normal serum creatinine level. In some cases, the transplant recipient has an elevated serum creatinine level. In some cases, the transplant recipient has a serum creatinine level of at least 0.4 mg/dL, 0.6 mg/dL, 0.8 mg/dL, 1.0 mg/dL, 1.2 mg/dL, 1.4 mg/dL, 1.6 mg/dL, 1.8 mg/dL, 2.0 mg/dL, 2.2 mg/dL, 2.4 mg/dL, 2.6 mg/dL, 2.8 mg/dL, 3.0 mg/dL, 3.2 mg/dL, 3.4 mg/dL, 3.6 mg/dL, 3.8 mg/dL, or 4.0 mg/dL. For example, the transplant recipient has a serum creatinine level of at least 1.5 mg/dL. In another example, the transplant recipient has a serum creatinine level of at least 3 mg/dL. In another example, the transplant recipient has a serum creatinine level less than 3 mg/dL, less than 2 mg/dL, less than 1.5 mg/dL, or less than 1.0 mg/dL.

A further understanding of the nature and advantages of the present invention may be realized by reference to the remaining portions of the specification and claims.

## BRIEF DESCRIPTION OF THE DRAWINGS

**Figure 1** shows a schematic overview of certain methods in the disclosure.
**Figure 2** shows a schematic overview of certain methods of acquiring samples, analyzing results, and transmitting reports over a computer network.
**Figure 3** shows a computer system for implementing the methods of the disclosure.

## DETAILED DESCRIPTION

The present invention is predicated in part on the development of molecular classifiers that can diagnose kidney transplantation patients who are Acute Rejection (AR), Acute Dysfunction No Rejection (ADNR), Chronic Allograft Nephropathy/Chronic Rejection (CAN/IFTA; CR), or Transplant Excellent/Normal (TX). The molecular classifiers were identified using RNA from kidney biopsies of the patients. These classifiers were successfully validated in an independent cohort, underscoring their applicability in significantly different racial/ethnic backgrounds as well as significantly different drug regimens. These signatures also correlated as well or better with assays based on creatinine levels and histology-based predictions. As detailed herein, they provide molecular insights into disease pathogenesis and functional molecular pathways including possible new drug targets.

The invention accordingly provides molecular diagnostic assays based on the identified molecular classifiers and additional studies of the inventors. The assays are cost effective, labor-saving and completely objective as compared to conventional light histology, the current "gold standard". It also provides a molecular score for phenotypes that are very difficult to accomplish with light histology. Thus, methods of the invention can be used as an alternative or complement to light histology in order to make more informed therapy decisions. They provide practical advantages of an automated, rapid molecular-based diagnostic over the current workflow for light histology involving pathologists making the interpretations. In addition to the diagnostic utilities, such assays could also provide a more in-depth understanding of the complex mechanisms of acute rejection, chronic injury, and tolerance in organ transplantation. This would allow the design of new and potentially more effective strategies for the minimization of immunosuppression, or even for the induction of immunological tolerance.

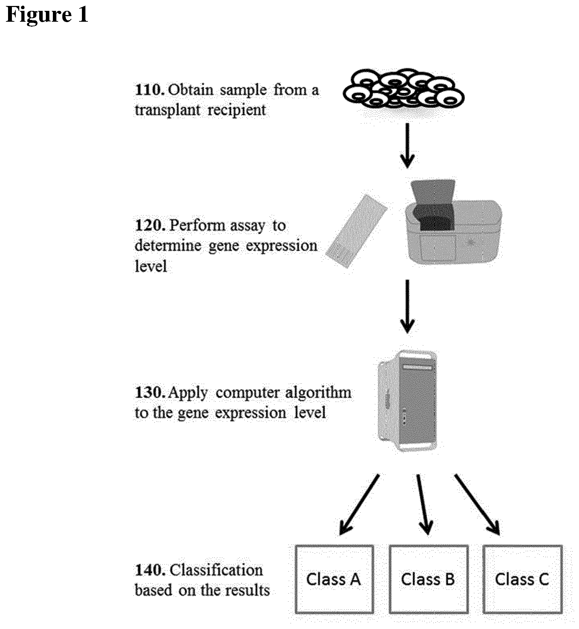

An overview of certain methods in the disclosure is provided in **Figure 1.** In some instances, a method comprises obtaining a sample from a transplant recipient in an invasive manner **(110),** such as via a biopsy, etc. The sample may comprise gene expression products (e.g., polypeptides, RNA, mRNA isolated from within cells or a cell-free source) associated with the status of the transplant (e.g., AR, ADNR, normal transplant function, etc.). In some instances, the method may involve reverse-transcribing RNA within the sample to obtain cDNA that can be analyzed using the methods described herein. The method may also comprise assaying the level of the gene expression products (or the corresponding DNA) using methods such as microarray or sequencing technology **(120).** The method may also comprise applying an algorithm to the assayed gene expression levels **(130).**

The following sections provide guidance for carrying out the methods of the invention.

## I. Definitions

Unless defined otherwise, all technical and scientific terms used herein have the same meaning as commonly understood by those of ordinary skill in the art to which this invention pertains. The following references provide one of skill with a general definition of many of the terms used in this invention: Academic Press Dictionary of Science and Technology, Morris (Ed.), Academic Press (1st ed., 1992); Illustrated Dictionary of Immunology, Cruse (Ed.), CRC Pr I Llc (2nd ed., 2002); Oxford Dictionary of Biochemistry and Molecular Biology, Smith et al. (Eds.), Oxford University Press (revised ed., 2000); Encyclopaedic Dictionary of Chemistry, Kumar (Ed.), Anmol Publications Pvt. Ltd. (2002); Dictionary of Microbiology and Molecular Biology, Singleton et al. (Eds.), John Wiley & Sons (3rd ed., 2002); Dictionary of Chemistry, Hunt (Ed.), Routledge (1st ed., 1999); Dictionary of Pharmaceutical Medicine, Nahler (Ed.), Springer-Verlag Telos (1994); Dictionary of Organic Chemistry, Kumar and Anandand (Eds.), Anmol Publications Pvt. Ltd. (2002); and A Dictionary of Biology (Oxford Paperback Reference), Martin and Hine (Eds.), Oxford University Press (4th ed., 2000). In addition, the following definitions are provided to assist the reader in the practice of the invention.

Transplantation is the transfer of tissues, cells or an organ from a donor into a recipient. If the donor and recipient as the same person, the graft is referred to as an autograft and as is usually the case between different individuals of the same species an allograft. Transfer of tissue between species is referred to as a xenograft. Unless otherwise noted, all graft samples described herein were allografts.

A biopsy is a specimen obtained from a living patient for diagnostic or prognostic evaluation. Kidney biopsies can be obtained with a needle.

An average value can refer to any of a mean, median or mode.

Chronic allograft nephropathy (CAN), also known as sclerosing/chronic allograft nephropathy, is the leading cause of kidney transplant failure and happens month to years after the transplant. It is characterized by a gradual decline in kidney function and, typically, accompanied by high blood pressure and hematuria. The histopathology is characterized by interstitial fibrosis, tubular atrophy, fibrotic intimal thickening of arteries and glomerulosclerosis.

A gene expression level is associated with a particular phenotype e.g., presence of acute graft rejection if the gene is differentially expressed in a patient having the phenotype relative to a patient lacking the phenotype to a statistically significant extent. Unless otherwise apparent from the context a gene expression level can be measured at the mRNA and/or protein level.

A target nucleic acids is a nucleic acid (often derived from a biological sample), to which a polynucleotide probe is designed to specifically hybridize. The probe can detect presence, absence and/or amount of the target. The term can refer to the specific subsequence of a larger nucleic acid to which the probe is directed or to the overall sequence (*e.g.,* cDNA or mRNA) whose expression level is to be detected. The term can also refer to a nucleic acid that is analyzed by a method, including sequencing, PCR, or other method known in the art.

The term subject or patient can include human or non-human animals. Thus, the methods and described herein are applicable to both human and veterinary disease and animal models. Preferred subjects are "patients," *i.e.,* living humans that are receiving medical care for a disease or condition. This includes persons with no defined illness who are being investigated for signs of pathology. The term subject or patient can include transplant recipients or donors or healthy subjects. The methods can be particularly useful for human subjects who have undergone a kidney transplant although they can also be used for subjects who have gone other types of transplant (e.g., heart, liver, lung, stem cell, etc.). The subjects may be mammals or non-mammals. Preferably, the subject is a human but in some cases, the subject is a non-human mammal, such as a non-human primate (e.g., ape, monkey, chimpanzee), cat, dog, rabbit, goat, horse, cow, pig, rodent, mouse, SCID mouse, rat, guinea pig, or sheep. The subject may be male or female; the subject may be and, in some cases, the subject may be an infant, child, adolescent, teenager or adult. In some cases, the methods provided herein are used on a subject who has not yet received a transplant, such as a subject who is awaiting a tissue or organ transplant. In other cases, the subject is a transplant donor. In some cases, the subject has not received a transplant and is not expected to receive such transplant. In some cases, the subject may be a subject who is suffering from diseases requiring monitoring of certain organs for potential failure or dysfunction. In some cases, the subject may be a healthy subject.

Often, the subject is a patient or other individual undergoing a treatment regimen, or being evaluated for a treatment regimen (e.g., immunosuppressive therapy). However, in some instances, the subject is not undergoing a treatment regimen. A feature of the graft tolerant phenotype detected or identified by the subject methods is that it is a phenotype which occurs without immunosuppressive therapy, e.g., it is present in a subject that is not receiving immunosuppressive therapy.

A transplant recipient may be a recipient of a solid organ or a fragment of a solid organ such as a kidney. Preferably, the transplant recipient is a kidney transplant or allograft recipient. In some instances, the transplant recipient may be a recipient of a tissue or cell. In some particular examples, the transplanted kidney may be a kidney differentiated in vitro from pluripotent stem cell(s) (e.g., induced pluripotent stem cells or embryonic stem cells).

The donor organ, tissue, or cells may be derived from a subject who has certain similarities or compatibilities with the recipient subject. For example, the donor organ, tissue, or cells may be derived from a donor subject who is age-matched, ethnicity-matched, gender-matched, blood-type compatible, or HLA-type compatible with the recipient subject.

In various embodiments, the subjects suitable for methods of the invention are patients who have undergone an organ transplant within 6 hours, 12 hours, 1 day, 2 days, 3 days, 4 days, 5 days, 10 days, 15 days, 20 days, 25 days, 1 month, 2 months, 3 months, 4 months, 5 months, 7 months, 9 months, 11 months, 1 year, 2 years, 4 years, 5 years, 10 years, 15 years, 20 years or longer of prior to receiving a classification obtained by the methods disclosed herein, such as detection of subAR.

Diagnosis refers to methods of estimating or determining whether or not a patient is suffering from a given disease or condition or severity of the condition. Diagnosis does not require ability to determine the presence or absence of a particular disease with 100% accuracy, or even that a given course or outcome is more likely to occur than not. Instead, the "diagnosis" refers to an increased probability that a certain disease or condition is present in the subject compared to the probability before the diagnostic test was performed. Similarly, a prognosis signals an increased probability that a given course or outcome will occur in a patient relative to the probability before the prognostic test.

A probe or polynucleotide probe is a nucleic acid capable of binding to a target nucleic acid of complementary sequence through one or more types of chemical bonds, usually through complementary base pairing, usually through hydrogen bond formation, thus forming a duplex structure. The probe binds or hybridizes to a "probe binding site." A probe can include natural (*e.g.,* A, G, C, U, or T) or modified bases (e.g., 7-deazaguanosine, inosine.). A probe can be an oligonucleotide which is a single-stranded DNA. Polynucleotide probes can be synthesized or produced from naturally occurring polynucleotides. In addition, the bases in a probe can be joined by a linkage other than a phosphodiester bond, so long as it does not interfere with hybridization. Thus, probes can include, for example, peptide nucleic acids in which the constituent bases are joined by peptide bonds rather than phosphodiester linkages (see, *e.g.,* Nielsen et al., Science 254, 1497-1500 (1991)). Some probes can have leading and/or trailing sequences of noncomplementarity flanking a region of complementarity.

A perfectly matched probe has a sequence perfectly complementary to a particular target sequence. The probe is typically perfectly complementary to a portion (subsequence) of a target sequence. The term "mismatch probe" refers to probes whose sequence is deliberately selected not to be perfectly complementary to a particular target sequence.

The term "isolated," "purified" or "substantially pure" means an object species (*e.g.,* a nucleic acid sequence described herein or a polypeptide encoded thereby) has been at least partially separated from the components with which it is naturally associated.

Differential expression refers to a statistically significant difference in expression levels of a gene between two populations of samples (e.g., kidney tissue samples with and without a specific type of kidney graft rejection such as AR). The expression levels can differ for example by at least a factor of 1.5 or 2 between such populations of samples. Differential expression includes genes that are expressed in one population and are not expressed (at least at detectable levels) in the other populations. Unique expression refers to detectable expression in one population and undetectable expression (i.e., insignificantly different from background) in the other population using the same technique (e.g., as in the present example for detection).

Control populations for comparison with populations undergoing a graft rejection or injury (e.g., AR) are usually referred to as being without the rejection or injury. Unless otherwise indicated, such a control population also means subjects without acute kidney rejection and/or chronic rejection.

Hybridization reactions are preferably performed under stringent conditions in which probes or primers hybridize to their intended target with which they have perfect complementarity and not to or at least to a reduced extent to other targets. An example of stringent hybridization conditions are hybridization in $6\times$sodium chloride/sodium citrate (SSC) at about 45° C., followed by one or more washes in $0.2\times$SSC, 0.1% SDS at 50° C, 55° C, 60° C, and even more or 65° C.

Statistical significance means $p < 0.05$, $< 0.01$, or $< 0.005$, or even $< 0.001$ level.

The term "about," as used herein and throughout the disclosure, generally refers to a range that may be 15% greater than or 15% less than the stated numerical value within the context of the particular usage. For example, "about 10" would include a range from 8.5 to 11.5.

The term "or" as used herein and throughout the disclosure, generally means "and/or".

II. Genes in profiles

Table 7 lists 199 genes whose expression changes significantly in kidney biopsies between transplant patients undergoing acute rejection (AR), acute dysfunction no rejection (ADNR), chronic allograft nephropathy (CAN), or are transplant excellent (*i.e.,* with normal functional transplant) (TX). The columns in the table have the following meanings: column 1 is a number assigned to a gene, column 2 is an Affymetrix number indicating a set of probes suitable for measuring expression of the gene, column 3 is a gene designation number, column 4 is a gene name (recognized names of HUGO or similar bodies are used when available), column 5 is a further description of the gene, column 6 is a measure of the statistical significance of change in gene expression between the above patient populations, and columns 7-10 respectively show mean expression levels of ADNR, AR, CAN, and TX patients. Expression profiles of genes selected from this list can be used to distinguish kidney transplant patients with one of the above-noted four graft conditions or phenotypes (4-way prediction).

Table 8 provides similar information on 197 genes that show differential expression between kidney transplant patients undergoing acute rejection (AR), acute dysfunction no rejection (ADNR), or are transplant excellent (TX). These are the most common phenotypes of kidney transplant during the early post-transplant period, while CAN is usually a late manifestation of graft injury which is progressive. Expression profiles of genes selected from this Table are thus useful for distinguishing kidney transplant patients who are undergoing acute rejection (AR), acute dysfunction no rejection (ADNR), or are transplant excellent (TX) (3-way prediction). Table 9 similarly lists information on 200 genes which show differential expression between transplant patients who have chronic allograft nephropathy (CAN) and patients who are transplant excellent (TX). Table 10 lists information on genes which show differential expression between transplant patients who have acute rejection (AR) and patients who are transplant excellent (TX). Table 11 lists information on genes which show differential expression between transplant patients who have chronic allograft nephropathy (CAN)/ interstitial fibrosis and tubular atrophy (IF/TA) and patients who are transplant excellent (TX). Expression profiles of genes selected from this list are typically suitable for making 2-way diagnosis between patients with these two phenotypes (4-way prediction).

The genes referred to in the above tables are human genes. In some methods, species variants or homologs of these genes are used in a non-human animal model. Species variants are the genes in different species having greatest sequence identity and similarity in functional properties to one another. Many species variants of the above human genes are listed in the Swiss-Prot database.

Raw gene expression levels are comparable between different genes in the same sample but not necessarily between different samples. As noted above, values given for gene expression levels can be normalized so that values for particular genes are comparable within and between the populations being analyzed. The normalization eliminates or at least reduces to acceptable levels any sample to sample differences arising from factors other than graft rejection (e.g., differences in overall transcription levels of patients due to general state of health and differences in sample preparation or nucleic acid amplification between samples) and also technical variation among the samples being analyzed. The normalization effectively applies a correction factor to the measured expression levels from a given array such that a profile of many expression levels in the array are the same between different patient samples. Software for normalizing overall expression patterns between different samples is both commercially (e.g., Partek Genomics Suite from Partek Systems) and publically available (e.g., XRAY from Biotique Systems or BRB ArrayTools from the National Cancer Institute). After applying appropriate normalizing factors to the measured expression value of a particular gene in different samples, an average or mean value of the expression level is determined for the samples in a population. The average or mean values between different populations are then compared to determine whether expression level has changed significantly between the populations. The changes in expression level indicated for a given gene represent the relative expression level of that gene in samples from a population of individuals with a defined condition (e.g., transplant patients with AR of specified Banff stage) relative to samples from a control population (kidney transplant patients not undergoing rejection). Similar principles apply in normalizing gene expression levels at the mRNA and protein levels. Comparisons between populations are made at the same level (e.g., mRNA levels in one population are compared with mRNA levels in another population or protein levels in one population with protein levels in another population).

III. Subject populations

The methods of the invention are suitable for detecting and distinguishing different types of graft rejections in kidney transplant patients. The methods are particularly useful on human subjects who have undergone a kidney transplant although can also be used on subjects who have gone other types of transplant (e.g., heart, liver, lungs, stem cell) or on non-humans who have undergone kidney or other transplant. As detailed herein, the methods can be employed to distinguish transplant patients who (1) have or are at risk of having acute rejection (AR), (2) have or are at risk of having acute dysfunction no rejection (ADNR), (3) have or are at risk of having chronic allograft nephropathy (CAN), or (4) have normal functioning transplant (TX). Other than patients for such a four way diagnosis, the subject population can also comprise only patients at early post-transplant period who are therefore likely to have AR, have acute dysfunction no rejection (ADNR), or are transplant excellent (TX). The patients are examined via a three-way analysis to identify one of these three graft phenotypes. Further, the subject population can also merely contain late post-transplant patients who likely either have chronic allograft nephropathy (CAN) or are transplant excellent (TX). Such a subject population can be examined with methods of the invention to diagnose or confirm that the patients have late-manifestation of graft injury.

Regardless of the specific subject population, gene expression levels in the patients can be measured, for example, within, one month, three months, six months, one year, two years, five years or ten years after a kidney transplant. In some methods, gene expression levels are determined at regular intervals, e.g., every 3 months, 6 months or every year post-transplant, either indefinitely, or until evidence of one of the noted phenotype is observed, in which case the frequency of monitoring is sometimes increased. In some methods, baseline values of expression levels are determined in a subject before a kidney transplant in combination with determining expression levels at one or more time points thereafter. Similar methods can be practiced in non-human species, in which cases, the expression levels measured are the species equivalent of the human genes referenced above.

Often the methods are used on a subject, preferably human, that is a transplant recipient. The methods may be used for detecting or predicting a condition of the transplant recipient such as acute rejection (AR), acute dysfunction with no rejection (ADNR), chronic allograft nephropathy (CAN), interstitial fibrosis and tubular atrophy (IF/TA), subclinical rejection acute rejection (SubAR), etc. In some cases, the condition may be AR. In some cases, the condition may be ADNR. In some cases, the condition may be SubAR. In some cases, the condition may be transplant dysfunction. In some cases, the condition may be transplant dysfunction with no rejection. In some cases, the condition may be acute transplant dysfunction.

Typically, when the patient does not exhibit symptoms or test results of organ dysfunction or rejection, the transplant is considered a normal functioning transplant (TX: Transplant eXcellent). An unhealthy transplant recipient may exhibit signs of organ dysfunction and/or rejection (e.g., an increasing serum creatinine). However, a subject (e.g., kidney transplant recipient) with subclinical rejection may have normal and stable organ function (e.g. normal creatinine level and normal eGFR). In these subjects, at the present time, rejection may be diagnosed histologically through a biopsy. A failure to recognize, diagnose and treat subclinical AR before significant tissue injury has occurred and the transplant shows clinical signs of dysfunction could be a major cause of irreversible organ damage. Moreover, a failure to recognize a chronic, subclinical immune-mediated organ damage and a failure to make appropriate changes in immunosuppressive therapy to restore a state of effective immunosuppression in that patient could contribute to late organ transplant failure. The methods disclosed herein can reduce or eliminate these and other problems associated with transplant rejection or failure.

Acute rejection (AR) occurs when transplanted tissue is rejected by the recipient's immune system, which damages or destroys the transplanted tissue unless immunosuppression is achieved. T-cells, B-cells and other immune cells as well as possibly antibodies of the recipient may cause the graft cells to lyse or produce cytokines that recruit other inflammatory cells, eventually causing necrosis of allograft tissue. In some instances, AR may be diagnosed by a biopsy of the transplanted organ. In the case of kidney transplant recipients, AR may be associated with an increase in serum creatinine levels. The treatment of AR may include using immunosuppressive agents, corticosteroids, polyclonal and monoclonal antibodies, engineered and naturally occurring biological molecules, and antiproliferatives. AR more frequently occurs in the first three to 12 months after transplantation but there is a continued risk and incidence of AR for the first five years post transplant and whenever a patient's immunosuppression becomes inadequate for any reason for the life of the transplant.

Acute dysfunction with no rejection (ADNR) is an abrupt decrease or loss of organ function without histological evidence of rejection from a transplant biopsy. Kidney transplant recipients with ADNR will often exhibit elevated creatinine levels. Unfortunately, the levels of kidney dysfunction based on serum creatinines are usually not significantly different between AR and ADNR subjects.

Another condition that can be associated with a kidney transplant is chronic allograft nephropathy (CAN), which is characterized by a gradual decline in kidney function and, typically, accompanied by high blood pressure and hematuria. Histopathology of patients with CAN is characterized by interstitial fibrosis, tubular atrophy, fibrotic intimal thickening of arteries and glomerulosclerosis typically described as IFTA. CAN/IFTA usually happens months to years after the transplant though increased amounts of IFTA can be present early in the first year post transplant in patients that have received kidneys from older or diseased donors or when early severe ischemia perfusion injury or other transplant injury occurs. CAN is a clinical phenotype characterized by a progressive decrease in organ transplant function. In contrast, IFTA is a histological phenotype currently diagnosed by an organ biopsy. In kidney transplants, interstitial fibrosis (IF) is usually considered to be present when the supporting connective tissue in the renal parenchyma exceeds 5% of the cortical area. Tubular atrophy (TA) refers to the presence of tubules with thick redundant basement membranes, or a reduction of greater than 50% in tubular diameter compared to surrounding non-atrophic tubules. In certain instances, finding interstitial fibrosis and tubular atrophy (IFTA) on the biopsy may be early indicators that predict the later organ dysfunction associated with the clinical phenotype of CAN. Immunologically, CAN/IFTA usually represents a failure of effective longterm immunosuppression and mechanistically it is immune-mediated chronic rejection (CR) and can involve both cell and antibody-mediated mechanisms of tissue injury as well as activation of complement and other blood coagulation mechanisms and can also involve inflammatory cytokine-mediated tissue activation and injury.

Subclinical rejection (SubAR) (also known as SCAR) is generally a condition that is histologically identified as acute rejection but without concurrent functional deterioration. For kidney transplant recipients, subclinical rejection (SubAR) is histologically defined acute rejection that is characterized by tubulointerstitial mononuclear infiltration identified from a biopsy specimen, but without concurrent functional deterioration (variably defined as a serum creatinine not exceeding about 10%, 20% or 25% of baseline values). A SubAR subject typically shows normal and/or stable serum creatinine levels. SubAR is usually diagnosed through biopsies that are taken at a fixed time after transplantation (e.g. protocol biopsies or serial monitoring biopsies) which are not driven by clinical indications but rather by standards of care. SubAR may be subclassified by some into acute SubAR or a milder form called borderline SubAR (suspicious for acute rejection) based on the biopsy histology.

In some instances, a normal serum creatinine level and/or a normal estimated glomerular filtration rate (eGFR) may indicate healthy transplant (TX) or subclinical rejection (SubAR). For example, typical reference ranges for serum creatinine are 0.5 to 1.0 mg/dL for women and 0.7 to 1.2 mg/dL for men, though typical kidney transplant patients have creatinines in the 0.8 to 1.5 mg/dL range for women and 1.0 to 1.9 mg/dL range for men. This may be due to the fact that most kidney transplant patients have a single kidney. In some instances, the trend of serum creatinine levels over time can be used to evaluate the recipient's organ function. This is why it may be important to consider both "normal" serum creatinine levels and "stable" serum creatinine levels in making clinical judgments, interpreting testing results, deciding to do a biopsy or making therapy change decisions including changing immunosuppressive drugs. For example, the transplant recipient may show signs of a transplant dysfunction or rejection as indicated by an elevated serum creatinine level and/or a decreased eGFR. In some instances, a transplant subject with a particular transplant condition (e.g., AR, ADNR, CAN, etc.) may have an increase of a serum creatinine level of at least 0.1 mg/dL, 0.2 mg/dL, 0.3 mg/dL, 0.4 mg/dL, 0.5 mg/dL, 0.6 mg/dL, 0.7 mg/dL 0.8 mg/dL, 0.9 mg/dL, 1.0 mg/dL, 1.1 mg/dL, 1.2 mg/dL, 1.3 mg/dL, 1.4 mg/dL, 1.5 mg/dL, 1.6 mg/dL, 1.7 mg/dL, 1.8 mg/dL, 1.9 mg/dL, 2.0 mg/dL, 2.1 mg/dL, 2.2 mg/dL, 2.3 mg/dL, 2.4 mg/dL, 2.5 mg/dL, 2.6 mg/dL, 2.7 mg/dL, 2.8 mg/dL, 2.9 mg/dL, 3.0 mg/dL, 3.1 mg/dL, 3.2 mg/dL, 3.3 mg/dL, 3.4 mg/dL, 3.5 mg/dL, 3.6 mg/dL, 3.7 mg/dL, 3.8 mg/dL, 3.9 mg/dL, or 4.0 mg/dL. In some instances, a transplant subject with a certain transplant condition (e.g., AR, ADNR, CAN, etc.) may have an increase of a serum creatinine level of at least 10%, 20%, 30%, 40%, 50%, 60%, 70%, 80%, 90%, or 100% from baseline. In some instances, a transplant subject with a certain transplant condition (e.g., AR, ADNR, CAN, etc.) may have an increase of a serum creatinine level of at least 1-fold, 2-fold, 3-fold, 4-fold, 5-fold, 6-fold, 7-fold, 8-fold, 9-fold, or 10-fold from baseline. In some cases, the increase in serum creatinine (e.g., any increase in the concentration of serum creatinine described herein) may occur over about .25 days, 0.5 days, 0.75 days, 1 day, 1.25 days, 1.5 days, 1.75 days, 2.0 days, 3.0 days, 4.0 days, 5.0 days, 6.0 days, 7.0 days, 8.0 days, 9.0 days, 10.0 days, 15 days, 30 days, 1 month, 2 months, 3 months, 4 months, 5 months, or 6 months, or more. In some instances, a transplant subject with a particular transplant condition (e.g., AR, ADNR, CAN, etc.) may have a decrease of a eGFR of at least 10%, 20%, 30%, 40%, 50%, 60%, 70%, 80%, 90%, or 100% from baseline. In some cases, the decrease in eGFR may occur over .25 days, 0.5 days, 0.75 days, 1 day, 1.25 days, 1.5 days, 1.75 days, 2.0 days, 3.0 days, 4.0 days, 5.0 days, 6.0 days, 7.0 days, 8.0 days, 9.0 days, 10.0 days, 15 days, 30 days, 1 month, 2 months, 3 months, 4 months, 5 months, or 6 months, or more. In some instances, diagnosing, predicting, or monitoring the status or outcome of a transplant or condition comprises determining transplant recipient-specific baselines and/or thresholds.

In some cases, the methods provided herein are used on a subject who has not yet received a transplant, such as a subject who is awaiting a tissue or organ transplant. In other cases, the subject is a transplant donor. In some cases, the subject has not received a transplant and is not expected to receive such transplant. In some cases, the subject may be a subject who is suffering from diseases requiring monitoring of certain organs for potential failure or dysfunction. In some cases, the subject may be a healthy subject.

A transplant recipient may be a recipient of a solid organ or a fragment of a solid organ. The solid organ may be a lung, kidney, heart, liver, pancreas, large intestine, small intestine, gall bladder, reproductive organ or a combination thereof. Preferably, the transplant recipient is a kidney transplant or allograft recipient. In some instances, the transplant recipient may be a recipient of a tissue or cell. The tissue or cell may be amnion, skin, bone, blood, marrow, blood stem cells, platelets, umbilical cord blood, cornea, middle ear, heart valve, vein, cartilage, tendon, ligament, nerve tissue, embryonic stem (ES) cells, induced pluripotent stem cells (IPSCs), stem cells, adult stem cells, hematopoietic stem cells, or a combination thereof.

The donor organ, tissue, or cells may be derived from a subject who has certain similarities or compatibilities with the recipient subject. For example, the donor organ, tissue, or cells may be derived from a donor subject who is age-matched, ethnicity-matched, gender-matched, blood-type compatible, or HLA-type compatible with the recipient subject.

The transplant recipient may be a male or a female. The transplant recipient may be patients of any age. For example, the transplant recipient may be a patient of less than about 10 years old. For example, the transplant recipient may be a patient of at least about 0, 5, 10, 20, 30, 40, 50, 60, 70, 80, 90, or 100 years old. The transplant recipient may be in utero. Often, the subject is a patient or other individual undergoing a treatment regimen, or being evaluated for a treatment regimen (e.g., immunosuppressive therapy). However, in some instances, the subject is not undergoing a treatment regimen. A feature of the graft tolerant phenotype detected or identified by the subject methods is that it is a phenotype which occurs without immunosuppressive therapy, e.g., it is present in a host that is not undergoing immunosuppressive therapy such that immunosuppressive agents are not being administered to the host.

In various embodiments, the subjects suitable for methods of the invention are patients who have undergone an organ transplant within 6 hours, 12 hours, 1 day, 2 days, 3 days, 4 days, 5 days, 10 days, 15 days, 20 days, 25 days, 1 month, 2 months, 3 months, 4 months, 5 months, 7 months, 9 months, 11 months, 1 year, 2 years, 4 years, 5 years, 10 years, 15 years, 20 years or longer of prior to receiving a classification disclosed herein (e.g., a classification obtained by the methods disclosed herein). Some of the methods further comprise changing the treatment regime of the patient responsive to the detecting, prognosing, diagnosing or monitoring step. In some of these methods, the subject can be one who has received a drug before performing the methods, and the change in treatment comprises administering an additional drug, administering a higher or lower dose of the same drug, stopping administration of the drug, or replacing the drug with a different drug or therapeutic intervention.

The subjects can include transplant recipients or donors or healthy subjects. The methods can be useful on human subjects who have undergone a kidney transplant although can also be used on subjects who have gone other types of transplant (e.g., heart, liver, lung, stem cell, etc.). The subjects may be mammals or non-mammals. The methods can be useful on non-humans who have undergone kidney or other transplant. Preferably, the subjects are a mammal, such as, a human, non-human primate (e.g., apes, monkeys, chimpanzees), cat, dog, rabbit, goat, horse, cow, pig, rodent, mouse, SCID mouse, rat, guinea pig, or sheep. Even more preferably, the subject is a human. The subject may be male or female; the subject may be a fetus, infant, child, adolescent, teenager or adult.

IV. Methods of measuring profiles

The preferred sample type for analysis with methods of the invention is a tissue biopsy, e.g., kidney biopsy for kidney transplant patients. In addition to biopsy samples, some other types of samples may also be used in the practice of the invention. These include, e.g., blood samples which can be whole blood or fractions thereof such as plasma or lymphocytes. Other samples that can be analyzed include urine, feces, and saliva. The samples are typically isolated from a subject and not returned to the subject. The analytes of interests in the samples can be analyzed with or without further processing of the sample, such as purification and amplification.

Expression profiles are preferably measured at the nucleic acid level, meaning that levels of mRNA or nucleic acid derived therefrom (e.g., cDNA or cRNA). An expression profile refers to the expression levels of a plurality of genes in a sample. A nucleic derived from mRNA means a nucleic acid synthesized using mRNA as a template. Methods of isolation and amplification of mRNA are well known in the art, e.g., as described in WO 97/10365, WO 97/27317, Chapter 3 of Laboratory Techniques in Biochemistry and Molecular Biology: Hybridization With Nucleic Acid Probes, Part I. Theory and Nucleic Acid Preparation, (P. Tijssen, ed.) Elsevier, N.Y. (1993). If mRNA or a nucleic acid therefrom is amplified, the amplification is performed under conditions that approximately preserve the relative proportions of mRNA in the original samples, such that the levels of the amplified nucleic acids can be used to establish phenotypic associations representative of the mRNAs.

A variety of approaches are available for determining mRNA levels including probe arrays and quantitative PCR. A number of distinct array formats are available. Some arrays, such as an Affymetrix GeneChip® array, have different probes occupying discrete known areas of a contiguous support. Other arrays, such as arrays from Illumina, have different probes attached to different particles or beads. In such arrays, the identity of which probe is attached to which particle or beads is usually determinable from an encoding system. The probes can be oligonucleotides. In such case, typically several match probes are included with perfect complementarity to a given target mRNA together, optionally together with mismatch probes differing from the match probes are a known number of oligonucleotides (Lockhart, et al., Nature Biotechnology 14:1675-1680 (1996); and Lipschutz, et al., Nature Genetics Supplement 21: 20-24, 1999). Other arrays including full length cDNA sequences with perfect or near perfect complementarity to a particular cDNA (Schena et al. (Science 270:467-470 (1995); and DeRisi et al. (Nature Genetics 14:457-460 (1996)). Such arrays can also include various control probes, such as a probe complementarity with a house keeping gene likely to be expressed in most samples. Regardless of the specifics of array design, an array contains one or more probes either perfectly complementary to a particular target mRNA or sufficiently complementarity to the target mRNA to distinguish it from other mRNAs in the sample, and the presence of such a target mRNA can be determined from the hybridization signal of such probes, optionally by comparison with mismatch or other control probes included in the array. Typically, the target bears a fluorescent label, in which case hybridization intensity can be determined by, for example, a scanning confocal microscope in photon counting mode. Appropriate scanning devices are described by e.g., U.S. 5,578,832, and U.S. 5,631,734. The intensity of labeling of probes hybridizing to a particular mRNA or its amplification product provides a raw measure of expression level.

In other methods, expression levels are determined by so-called "real time amplification" methods also known as quantitative PCR or Taqman (see, e.g., U.S. Pat Nos. 5,210,015 to Gelfand, 5,538,848 to Livak, et al., and 5,863,736 to Haaland, as well as Heid, C.A., et al., Genome Research, 6:986-994 (1996); Gibson, U.E.M, et al., Genome Research 6:995-1001 (1996); Holland, P. M., et al., Proc. Natl. Acad. Sci. USA 88:7276-7280, (1991); and Livak, K.J., et al., PCR Methods and Applications 357-362 (1995)). The basis for this method of monitoring the formation of amplification product is to measure continuously PCR product accumulation using a dual-labeled fluorogenic oligonucleotide probe. The probe used in such assays is typically a short (ca. 20-25 bases) polynucleotide that is labeled with two different fluorescent dyes. The 5' terminus of the probe is typically attached to a reporter dye and the 3' terminus is attached to a quenching dye The probe is designed to have at least substantial sequence complementarity with a site on the target mRNA or nucleic acid derived from. Upstream and downstream PCR primers that bind to flanking regions of the locus are also added to the reaction mixture. When the probe is intact, energy transfer between the two fluorophors occurs and the quencher quenches emission from the reporter. During the extension phase of PCR, the probe is cleaved by the 5' nuclease activity of a nucleic acid polymerase such as Taq polymerase, thereby releasing the reporter from the polynucleotide-quencher and resulting in an increase of reporter emission intensity which can be measured by an appropriate detector. The recorded values can then be used to calculate the increase in normalized reporter emission intensity on a continuous basis and ultimately quantify the amount of the mRNA being amplified. mRNA levels can also be measured without amplification by hybridization to a probe, for example, using a branched nucleic acid probe, such as a Quanti-Gene® Reagent System from Panomics.

In other methods, expression levels are determined by sequencing methods. Sequencing methods may include: Next Generation sequencing, high-throughput sequencing, pyrosequencing, classic Sangar sequencing methods, sequencing-by-ligation, sequencing by synthesis, sequencing-by-hybridization, RNA-Seq (Illumina), Digital Gene Expression (Helicos), next generation sequencing, single molecule sequencing by synthesis (SMSS) (Helicos), Ion Torrent Sequencing Machine (Life Technologies/Thermo-Fisher), massively-parallel sequencing, clonal single molecule Array (Solexa), shotgun sequencing, Maxim-Gilbert sequencing, primer walking, and any other sequencing methods known in the art.

Alternatively or additionally, expression levels of genes can be determined at the protein level, meaning that levels of proteins encoded by the genes discussed above are measured. Several methods and devices are well known for determining levels of proteins including immunoassays such as described in e.g., U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792. These assays include various sandwich, competitive, or non-competitive assay formats, to generate a signal that is related to the presence or amount of an protein analyte of interest. Any suitable immunoassay may be utilized, for example, lateral flow, enzyme-linked immunoassays (ELISA), radioimmunoassays (RIAs), competitive binding assays, and the like. Numerous formats for antibody arrays have been described proposed employing antibodies. Such arrays typically include different antibodies having specificity for different proteins intended to be detected. For example, usually at least one hundred different antibodies are used to detect one hundred different protein targets, each antibody being specific for one target. Other ligands having specificity for a particular protein target can also be used, such as the synthetic antibodies disclosed in WO/2008/048970. Other compounds with a desired binding specificity can be selected from random libraries of peptides or small molecules. US Patent No. 5,922,615 describes a device that utilizes multiple discrete zones of immobilized antibodies on membranes to detect multiple target antigens in an array. US Patent Nos. 5,458,852, 6,019,944, US 6,143,576. Microtiter plates or automation can be used to facilitate detection of large numbers of different proteins. Protein levels can also be determined by mass spectrometry as described in the examples.

The selection of genes for determination of expression levels depends on the particular application. In general, the genes are selected from one of the tables indicated above as appropriate for the application. In some methods, expression levels of at least 2, 3, 4, 5, 10, 20, 25, 50, 100, 150, or 200 (e.g., 10-100) genes shown in any of Table 7, 8, 9, 10, or 11 are determined. In some methods, expression levels of at least 2, 3, 4, 5, 10, 20, 25, 50, 100, 150 or all genes shown in Table 7 are determined. In some methods, expression levels of at least 2, 3, 4, 5, 10, 20, 25, 50, 100, 150 or all genes in Table 8 are determined. In some methods, expression levels of at least 2, 3, 4, 5, 10, 20, 25, 50, 100, 150 or all genes shown in Table 9 are determined. In some methods, expression levels of at least 2, 3, 4, 5, 10, 20, 25, 50, 100, 150 or all genes shown in Table 10 are determined. In some methods, expression levels of at least 2, 3, 4, 5, 10, 20, 25, 50, 100, 150 or all genes shown in Table 11 are determined. In some methods, genes from different tables are tested (e.g., at least 2, 3, 5, 10, 25, 50 or more genes from each of Table 7, Table 8, Table 9, Table 10, and/or Table 11). In some methods, genes are selected such that genes from several different pathways are represented (e.g.,

at least one gene from at least 2, 3, 5, or 10 pathways). The genes within a pathway tend to be expressed in a coordinated expression whereas genes from different pathways tend to be expressed more independently. Thus, changes in expression based on the aggregate changes of genes from different pathways can have greater statistical significance than aggregate changes of genes within a pathway. As noted above, expression levels can be measured at either mRNA levels or protein levels.

Expression levels of the present genes and/or proteins can be combined with or without determination of expression levels of any other genes or proteins of interest (e.g., genes or proteins associated with rejection of kidneys or other organs in WO 2007/104537, WO 2009/060035), Anglicheau et al., PNAS 106, 5330-5335 (2009)) and references, 16, 20, 21, 22, 23, 25, 26, 37 and 39. In some methods, the genes in the expression profiles to be measured do not include at least one or all of the genes known to be linked to graft rejection, e.g., genes described in Halloran et al., Am. J. Transplant. 2013, 13(11):2865-74; and Halloran et al., Am. J. Transplant. 2013, 13(9):2352-63.

Regardless of the format adopted, the present methods can (but need not) be practiced by detection expression levels of a relatively small number of genes or proteins compared with the whole genome level expression analysis described in the Examples. In some methods, the total number of genes whose expression levels are determined is less than 5000, 1000, 500, 200, 100, 50, 25, 10, 5 or 3. In some methods, the total number of genes whose expression level is determined is 100-1500, 100-250, 500-1500 or 750-1250. In some methods, the total number of proteins whose expression levels are determined is less than 5000, 1000, 500, 200, 100, 50, 25, 10, 5 or 3. In some methods, the total number of proteins whose expression level is determined is 100-1500, 100-250, 500-1500 or 750-1250. Correspondingly, when an array form is used for detection of expression levels, the array includes probes or probes sets for less than 5000, 1000, 500, 200, 100, 50, 25, 10, 5 or 3 genes. Thus, for example, an Affymetrix GeneChip® expression monitoring array contains a set of about 20-50 oligonucleotide probes (half match and half-mismatch) for monitoring each gene of interest. Such an array design would include less than 5000, 1000, 500, 200, 100, 50, 25, 10, 5 or 3 such probes sets for detecting less than 5000, 1000, 500, 200, 100, 50, 25, 10, 5 or 3 genes. By further example, an alternative array including one cDNA for each gene whose expression level is to be detected would contain less than 5000, 1000, 500, 200, 100, 50, 25, 10, 5 or 3 such cDNAs for analyzing less than 5000, 1000, 500, 200, 100, 50, 25, 10, 5 or 3 genes. By further example, an array containing a different antibody for each protein to be detected would containing less than 5000, 1000, 500, 200, 100, 50, 25, 10, 5 or 3 different antibodies for analyzing less than 5000, 1000, 500, 200, 100, 50, 25, 10, 5 or 3 gene products.

Methods for detecting molecules (e.g., nucleic acids, proteins, etc.) in a subject who has received a transplant (e.g., organ transplant, tissue transplant, stem cell transplant) in order to detect, diagnose, monitor, predict, or evaluate the status or outcome of the transplant are described in this disclosure. In some cases, the molecules are circulating molecules. In some cases, the molecules are expressed in blood cells. In some cases, the molecules are cell-free circulating nucleic acids.

The methods, kits, and systems disclosed herein may be used to classify one or more samples from one or more subjects. A sample may be any material containing tissues, cells, nucleic acids, genes, gene fragments, expression products, polypeptides, exosomes, gene expression products, or gene expression product fragments of a subject to be tested. Methods for determining sample suitability and/or adequacy are provided. A sample may include but is not limited to, tissue, cells, or biological material from cells or derived from cells of an individual. The sample may be a heterogeneous or homogeneous population of cells or tissues. In some cases, the sample is from a single patient. In some cases, the method comprises analyzing multiple samples at once, e.g., via massively parallel sequencing.

The methods, kits, and systems disclosed herein may comprise specifically detecting, profiling, or quantitating molecules (e.g., nucleic acids, DNA, RNA, polypeptides, etc.) that are within the biological samples. In some instances, genomic expression products, including RNA, or polypeptides, may be isolated from the biological samples. In some cases, nucleic acids, DNA, RNA, polypeptides may be isolated from a cell-free source. In some cases, nucleic acids, DNA, RNA, polypeptides may be isolated from cells derived from the transplant recipient.

The sample may be obtained using any method known to the art that can provide a sample suitable for the analytical methods described herein. In some instances, the sample is obtained by an invasive procedure including but not limited to: biopsy, alveolar or pulmonary lavage, or needle aspiration. The method of biopsy may include surgical biopsy, incisional biopsy, excisional biopsy, punch biopsy, shave biopsy, or skin biopsy. The sample may be formalin fixed sections. The method of needle aspiration may further include fine needle aspiration, core needle biopsy, vacuum assisted biopsy, or large core biopsy. In some embodiments, multiple samples may be obtained by the methods herein to ensure a sufficient amount of biological material. In some instances, the sample is not obtained by biopsy. In some instances, the sample is not a kidney biopsy.

The total sample population may comprise samples obtained by needle aspiration, fine needle aspiration, core needle biopsy, vacuum assisted biopsy, large core biopsy, incisional biopsy, excisional biopsy, punch biopsy, shave biopsy, skin biopsy, or a combination thereof. In some embodiments, the samples are not obtained by biopsy. The percent of the total sample population that is obtained by biopsy may be greater than about 1%, 5%, 10%, 15%, 20%, 25%, 30%, 35%, 40%, 50%, 55%, 60%, 65%, 70%, 75%, 80%, 85%, 90%, or 95%. The percent of the total sample population that is obtained by biopsy may be greater than about 1%. The percent of the total sample population that is obtained by biopsy may be greater than about 5%. The percent of the total sample population that is obtained by biopsy may be greater than about 10%.

## V. Analysis of Expression Levels

Analysis of expression levels initially provides a measurement of the expression level of each of several individual genes. The expression level can be absolute in terms of a concentration of an expression product, or relative in terms of a relative concentration of an expression product of interest to another expression product in the sample. For example, relative expression levels of genes can be expressed with respect to the expression level of a house-keeping gene in the sample. Relative expression levels can also be determined by simultaneously analyzing differentially labeled samples hybridized to the same array. Expression levels can also be expressed in arbitrary units, for example, related to signal intensity.

The individual expression levels, whether absolute or relative, can be converted into values or other designations providing an indication of presence or risk of a kidney transplant rejection or injury by comparison with one or more reference points. For different phenotypes of graft injuries (e.g., AR, ADNR, CAN; or TX), different gene sets are typically used in the analysis. For example, chronic allograft nephropathy (CAN) can be determined with gene sets selected from Table 9 or Table 7. Acute rejection (AR) and acute dysfunction no rejection (ADNR) can be determined with genes selected from Table 8 or Table 7. Acute rejection (AR) and chronic allograft nephropathy/interstitial fibrosis and tubular atrophy (IF/TA) can be determined with genes selected from Table 10 or Table 11.

For kidney transplant with each of the phenotypes noted above, the reference points can include a measure of an average or mean expression level of a gene in subjects having had a kidney transplant with the specific phenotype. The reference points can also include a scale of values found in kidney transplant patients including patients having that phenotype. The reference points can also or alternatively include a reference value in the subject before kidney transplant, or a reference value in a population of patients who have not undergone kidney transplant. Such reference points can be expressed in terms of absolute or relative concentrations of gene products as for measured values in a sample.

For comparison between a measured expression level and reference level(s), the measured level sometimes needs to be normalized for comparison with the reference level(s) or vice versa. The normalization serves to eliminate or at least minimize changes in expression level unrelated to the specific kidney transplant injury or phenotype (e.g., from differences in overall health of the patient or sample preparation). Normalization can be performed by determining what factor is needed to equalize a profile of expression levels measured from different genes in a sample with expression levels of these genes in a set of reference samples from which the reference levels were determined. Commercial software is available for performing such normalizations between different sets of expression levels.

Comparison of the measured expression level of a gene with one or more of the above reference points provides a value (i.e., numerical) or other designation (e.g., symbol or word(s)) of presence or susceptibility to a kidney transplant injury. In some methods, a binary system is used; that is a measured expression level of a gene is assigned a value or other designation indicating presence or susceptibility to a kidney transplant injury or lack thereof without regard to degree. For example, the expression level can be assigned a value of 1 to indicate presence or susceptibility to an injury and -1 to indicate absence or lack of susceptibility to the injury. Such assignment can be based on whether the measured expression level is closer to an average or mean level in kidney transplant patients having or not having a specific injury phenotype. In other methods, a ternary system is used in which an expression level is assigned a value or other designation indicating presence or susceptibility to a specific injury phenotype or lack thereof or that the expression level is uninformative. Such assignment can be based on whether the expression level is closer to the average or mean level in kidney transplant patient undergoing the specific injury, closer to an average or mean level in kidney transplant patients lacking the injury or intermediate between such levels. For example, the expression level can be assigned a value of +1, -1 or 0 depending on whether it is closer to the average or mean level in patients undergoing the injury, is closer to the average or mean level in patients not undergoing the injury or is intermediate. In other methods, a particular expression level is assigned a value on a scale, where the upper level is a measure of the highest expression level found in kidney transplant patients and the lowest level of the scale is a measure of the lowest expression level found in kidney transplant patients at a defined time point at which patients may be susceptible to a grant rejection or injury (e.g., one year post transplant). Preferably, such a scale is normalized scale (e.g., from 0-1) such that the same scale can be used for different genes. Optionally, the value of a measured expression level on such a scale is indicated as being positive or negative depending on whether the upper level of the scale associates with presence or susceptibility to the injury or lack thereof. It does not matter whether a positive or negative sign is used for an injury phenotype or lack thereof as long as the usage is consistent for different genes.

Values or other designation can also be assigned based on a change in expression level of a gene relative to a previous measurement of the expression level of gene in the same patient. Here as elsewhere expression level of a gene can be measured at the protein or nucleic acid level. Such a change can be characterized as being toward, away from or neutral with respect to average or mean expression levels of the gene in kidney transplant patients undergoing or not undergoing a grant rejection or injury. For example, a gene whose expression level changes toward an average or mean expression level in kidney transplant patients undergoing a graft injury can be assigned a value of 1, and a gene whose express level changes way from an average or mean expression level in kidney transplant patients undergoing the injury and toward an average or mean expression level in kidney transplant patients not undergoing the injury can be assigned a value -1. Of course, more sophisticated systems of assigning values are possible based on the magnitude of changes in expression of a gene in a patient.

Having determined values or other designations of expression levels of individual genes providing an indication of presence or susceptibility to a kidney graft injury or lack thereof, the values or designations are combined to provide an aggregate value for all of the genes being analyzed. If each gene is assigned a score of +1 if its expression level indicates presence or susceptibility to a graft injury and -1 if its expression level indicates absence or lack of susceptibility to the injury and optionally zero if uninformative, the different values can be combined by addition. The same approach can be used if each gene is assigned a value on the same normalized scale and assigned as being positive or negative depending whether the upper point of the scale is associate with presence or susceptibility to a specific kidney grant injury or lack thereof. In some cases, the signal intensity for each gene is obtained and used to compute a score. The score may be obtained by adding up the values for the upregulated genes to obtain an upregulated gene value and adding up the values of the downregulated genes to obtain a downregulated gene value; the downregulated gene value may be compared with the upregulated value (e.g., by calculating a ratio) to determine the score. Other methods of combining values for individual markers of disease into a composite value that can be used as a single marker are described in US20040126767 and WO/2004/059293. In some cases, the score may be used to evaluate severity of a transplant condition, such as by comparing the score with a score normally associated with kidney graft injury. In some cases, the score may be used to monitor a subject transplant recipient over time. In such case, scores at a plurality of timepoints maybe compared in order to assess the relative condition of the subject. For example, if the subject's score rises over time, that may indicate that the subject has kidney graft injury and that his or her condition is worsening over time.

## Sample Data

The data pertaining to the sample may be compared to data pertaining to one or more control samples, which may be samples from the same patient at different times. In some cases, the one or more control samples may comprise one or more samples from healthy subjects, unhealthy subjects, or a combination thereof. The one or more control samples may comprise one or more samples from healthy subjects, subjects suffering from transplant dysfunction with no rejection, subjects suffering from transplant rejection, or a combination thereof. The healthy subjects may be subjects with normal transplant function. The data pertaining to the sample may be sequentially compared to two or more classes of samples. The data pertaining to the sample may be sequentially compared to three or more classes of samples. The classes of samples may comprise control samples classified as being from subjects with normal transplant function, control samples classified as being from subjects suffering from transplant dysfunction with no rejection, control samples classified as being from subjects suffering from transplant rejection, or a combination thereof.

*Classifiers*

The methods include using a trained classifier or algorithm to analyze sample data, particularly to detect subAR. In some instances, the expression levels from sample are used to develop or train an algorithm or classifier provided herein. In some instances, gene expression levels are measured in a sample from a transplant recipient (or a healthy or transplant excellent control) and a classifier or algorithm (e.g., trained algorithm) is applied to the resulting data in order to detect, predict, monitor, or estimate the risk of a transplant condition (e.g., subAR).

Training of multi-dimensional classifiers (e.g., algorithms) may be performed using numerous samples. For example, training of the multi-dimensional classifier may be performed using at least about 10, 20, 30, 40, 50, 60, 70, 80, 90, 100, 110, 120, 130, 140, 150, 160, 170, 180, 190, 200 or more samples. In some cases, training of the multi-dimensional classifier may be performed using at least about 200, 210, 220, 230, 240, 250, 260, 270, 280, 290, 300, 350, 400, 450, 500 or more samples. In some cases, training of the multi-dimensional classifier may be performed using at least about 525, 550, 600, 650, 700, 750, 800, 850, 900, 950, 1000, 1100, 1200, 1300, 1400, 1500, 1600, 1700, 1800, 2000 or more samples.

Further disclosed herein are classifier sets and methods of producing one or more classifier sets. The classifier set may comprise one or more genes, particularly genes from Tables 7, 8, or 9. In some cases, the classifier set may comprise 1, 2, 3, 4, 5, 6, 7, 8, 9, 10, 11, 12, 13, 14, 15, 16, 17, 18, 19, 20, 50, 100, 150, 200, 300 or more genes from Tables 7, 8, 9, 10, or 11. Disclosed herein is the use of a classification system comprises one or more classifiers. In some instances, the classifier is a 2-, 3-, 4-, 5-, 6-, 7-, 8-, 9-, or 10-way classifier. In some instances, the classifier is a 15-, 20-, 25-, 30-, 35-, 40-, 45-, 50-, 55-, 60-, 65-, 70-, 75-, 80-, 85-, 90-, 95-, or 100-way classifier. In some preferred embodiments, the classifier is a three-way classifier. In some embodiments, the classifier is a four-way classifier.

A two-way classifier may classify a sample from a subject into one of two classes. In some instances, a two-way classifier may classify a sample from an organ transplant recipient into one of two classes comprising subAR and normal transplant function (TX). In some instances, a three-way classifier may classify a sample from a subject into one of three classes. A three-way classifier may classify a sample from an organ transplant recipient into one of three classes comprising AR, subAR, and TX. In some cases, the classifier may work by applying two or more classifiers sequentially. For example, the first classifier may classify AR+subAR and TX, which results in a set of samples that are classified either as (1) TX or (2) AR or subAR. In some cases, a second classifier capable of distinguishing between AR and subAR is applied to the samples classified as having AR or subAR in order to detect the subAR samples.

Classifiers and/or classifier probe sets may be used to either rule-in or rule-out a sample as healthy. For example, a classifier may be used to classify a sample as being from a healthy subject. Alternatively, a classifier may be used to classify a sample as being from an unhealthy subject. Alternatively, or additionally, classifiers may be used to either rule-in or rule-out a sample as transplant rejection. For example, a classifier may be used to classify a sample as being from a subject suffering from a transplant rejection. In another example, a classifier may be used to classify a sample as being from a subject that is not suffering from a transplant rejection. Classifiers may be used to either rule-in or rule-out a sample as transplant dysfunction with no rejection. For example, a classifier may be used to classify a sample as being from a subject with subAR. In another example, a classifier may be used to classify a sample as not being from a subject suffering from transplant dysfunction with no rejection.

The data pertaining to the sample may be compared to data pertaining to one or more control samples, which may be samples from the same patient at different times. In some cases, the one or more control samples may comprise one or more samples from healthy subjects, unhealthy subjects, or a combination thereof. The one or more control samples may comprise one or more samples from healthy subjects, subjects suffering from transplant dysfunction with no rejection, subjects suffering from transplant rejection, or a combination thereof. The healthy subjects may be subjects with normal transplant function. The data pertaining to the sample may be sequentially compared to two or more classes of samples. The data pertaining to the sample may be sequentially compared to three or more classes of samples. The classes of samples may comprise control samples classified as being from subjects with normal transplant function, control samples classified as being from subjects suffering from transplant dysfunction with no rejection, control samples classified as being from subjects suffering from transplant rejection, or a combination thereof.

The methods, kits, and systems disclosed herein may comprise one or more algorithms or uses thereof. The one or more algorithms may be used to classify one or more samples from one or more subjects. The one or more algorithms may be applied to data from one or more samples. The data may comprise gene expression data. The data may comprise sequencing data. The data may comprise array hybridization data.

The methods disclosed herein may comprise assigning a classification to one or more samples from one or more subjects. Assigning the classification to the sample may comprise applying an algorithm to the expression level. In some cases, the gene expression levels are inputted to a trained algorithm for classifying the sample as one of the conditions comprising subAR, AR, TX, subAR+AR, or other condition.

The algorithm may provide a record of its output including a classification of a sample and/or a confidence level. In some instances, the output of the algorithm can be the possibility of the subject of having a condition, such as subAR. In some instances, the output of the algorithm can be the risk of the subject of having a condition, such as subAR. In some instances, the output of the algorithm can be the possibility of the subject of developing into a condition in the future, such as subAR.

The algorithm may be a trained algorithm. The algorithm may comprise a linear classifier. The linear classifier may comprise one or more linear discriminant analysis, Fisher's linear discriminant, Naive Bayes classifier, Logistic regression, Perceptron, Support vector machine, or a combination thereof._The linear classifier may be a Support vector machine (SVM) algorithm.

The algorithm may comprise one or more linear discriminant analysis (LDA), Basic perceptron, Elastic Net, logistic regression, (Kernel) Support Vector Machines (SVM), Diagonal Linear Discriminant Analysis (DLDA), Golub Classifier, Parzen-based, (kernel) Fisher Discriminant Classifier, k-nearest neighbor, Iterative RELIEF, Classification Tree, Maximum Likelihood Classifier, Random Forest, Nearest Centroid, Prediction Analysis of Microarrays (PAM), k-medians clustering, Fuzzy C-Means Clustering, Gaussian mixture models, or a combination thereof. The algorithm may comprise a Diagonal Linear Discriminant Analysis (DLDA) algorithm. The algorithm may comprise a Nearest Centroid algorithm. The algorithm may comprise a Random Forest algorithm. The algorithm may comprise a Prediction Analysis of Microarrays (PAM) algorithm.

The methods disclosed herein may comprise use of one or more classifier equations. Classifying the sample may comprise a classifier equation. The classifier equation may be Equation 1:

$$\delta_k(x^*) = \sum_{i=1}^{p} \frac{(x_i^* - \bar{x}_{ik}')^2}{(s_i + s_0)^2} - 2\log \pi_k$$

,

wherein:

k is a number of possible classes;
$\delta_k$ may be the discriminant score for class k;
$x_i^*$ represents the expression level of gene i;
x* represents a vector of expression levels for all p genes to be used for classification drawn from the sample to be classified;
$\bar{x}_k'$ may be a shrunken centroid calculated from a training data and a shrinkage factor;
$\bar{x}_{ik}'$ may be a component of $\bar{x}_k'$ corresponding to gene i;
$s_i$ is a pooled within-class standard deviation for gene i in the training data;
$s_0$ is a specified positive constant; and
$\pi_k$ represents a prior probability of a sample belonging to class k.

Assigning the classification may comprise calculating a class probability. Calculating the class probability $\hat{p}_k(x^*)$ may be calculated by Equation 2:

$$\hat{p}_k(x^*) = \frac{e^{-\frac{1}{2}\delta_k(x^*)}}{\sum_{l=1}^{K} e^{-\frac{1}{2}\delta_l(x^*)}}$$

.

Assigning the classification may comprise a classification rule. The classification rule C(x*) may be expressed by Equation 3:

$$C(x^*) = \underset{k \in \{1, K\}}{\arg \max} \, \hat{p}_k(x^*)$$

.

VI. Diagnosis, prognosis and monitoring

The above described methods can provide a value or other designation for a patient which indicates whether the aggregate measured expression levels in a patient is more like kidney transplant patients with one of the graft injury phenotypes noted above (e.g., AR, ADNR, CAN, or TX). Such a value provides an indication that the patient either has or is at enhanced risk of developing a specific graft injury, or conversely does not have or is at reduced risk of having that specific graft injury phenotype. Risk is a relative term in which risk of one patient is compared with risk of other patients either qualitatively or quantitatively. For example, the value of one patient can be compared with a scale of values for a population of patients having undergone kidney transplant to determine whether the patient's risk relative to that of other patients. In general, diagnosis is the determination of the present condition of a patient (e.g., presence or absence of a graft injury) and prognosis is developing future course of the patient (e.g., risk of developing kidney transplant rejection or injury in the future or likelihood of improvement in response to treatment); however, the analyses contemplated by these terms may overlap or even be the same. For example, the present methods alone do not necessarily distinguish between presence and enhanced risk of a kidney transplant injury. However, these possibilities can be distinguished by additional testing.

If a patient is indicated as having or being at enhanced risk of a kidney transplant injury, the physician can subject the patient to additional testing including performing a kidney biopsy examination, or performing other analyses such as creatinine, BUN or glomerular filtration rate at increased frequency. Additionally or alternatively, the physician can change the treatment regime being administered to the patient. This includes administration of steroid boluses and the addition of other drugs to the maintenance therapy, or the administration of antilymphocyte antibodies in case of resistance to the primary line of therapy. In some embodiments, the change in treatment regime can include administering an additional or different drug, or administering a higher dosage or frequency of a drug already being administered to the patient. Many different drugs are available for treating rejection, such as immunosuppressive drugs used to treat transplant rejection calcineurin inhibitors (e.g., cyclosporine, tacrolimus), mTOR inhibitors (e.g., sirolimus and everolimus), antiproliferatives (e.g., azathioprine, mycophenolic acid), corticosteroids (e.g., prednisolone and hydrocortisone) and antibodies (e.g., basiliximab, daclizumab, Orthoclone, anti-thymocyte globulin and anti-lymphocyte globulin). In the case of HCV recurrence, the patients may be additionally administered drugs to counter the viral infection, e.g., interferons, ribavirin, and protease inhibitors. Conversely, if the value or other designation of aggregate expression levels of a patient indicates the patient does not have or is at reduced risk of graft injury, the physician need not order further diagnostic procedures, particularly not invasive ones such as biopsy. Further, the physician can continue an existing treatment regime, or even decrease the dose or frequency of an administered drug.

In some methods, expression levels are determined at intervals in a particular patient (i.e., monitoring). Such methods can provide a series of values changing over time indicating whether the aggregate expression levels in a particular patient are more like the expression levels in patients undergoing a specific kidney transplant rejection/injury or not undergoing the rejection/injury. Movement in value toward or away from the graft injury can provide an indication whether an existing immunosuppressive regime is working, whether the immunosuppressive regime should be changed or whether a biopsy or increased monitoring by other markers rate should be performed.

## VII. Drug Screening

The expression profiles associated with a kidney transplant rejection/injury or lack thereof provided by the invention are useful in screening drugs, either in clinical trials or in animal models of the injury. A clinical trial can be performed on a drug in similar fashion to the monitoring of an individual patient described above, except that drug is administered in parallel to a population of kidney transplant patients, usually in comparison with a control population administered a placebo.

The changes in expression levels of genes can be analyzed in individual patients and across a treated or control population. Analysis at the level of an individual patient provides an indication of the overall status of the patient at the end of the trial (i.e., whether gene expression profile indicates presence or enhanced susceptibility to a kidney transplant rejection/injury) and/or an indication whether that profile has changed toward or away from such indication in the course of the trial. Results for individual patients can be aggregated for a population allowing comparison between treated and control population. Mannon et al., Kidney International (1999) 55, 1935-1944. In this case, the expression levels of genes detected are the species variants or homologs of the human genes referenced above in whatever species of non-human animal on which tests are being conducted. Although the average or mean expression levels of human genes determined in human kidney transplant patients undergoing or not undergoing a specific transplant rejection/injury are not necessarily directly comparable to those of homolog genes in an animal model, the human values can nevertheless be used to provide an indication whether a change in expression level of a non-human homolog is in a direction toward or away from an injury or susceptibility thereto. The expression profile of individual animals in a trial can provide an indication of the status of the animal at the end of the trial with respect to presence or susceptibility to the injury and/or change in such status during the trial. Results from individual animals can be aggregated across a population and treated and control populations compared. Average changes in the expression levels of genes can then be compared between the two populations.

## VIII. Computer implemented methods

Expression levels can be analyzed and associated with status of a subject (e.g., presence or susceptibility to a kidney transplant injury) in a digital computer. Optionally, such a computer is directly linked to a scanner or the like receiving experimentally determined signals related to expression levels. Alternatively, expression levels can be input by other means. The computer can be programmed to convert raw signals into expression levels (absolute or relative), compare measured expression levels with one or more reference expression levels, or a scale of such values, as described above. The computer can also be programmed to assign values or other designations to expression levels based on the comparison with one or more reference expression levels, and to aggregate such values or designations for multiple genes in an expression profile. The computer can also be programmed to output a value or other designation providing an indication of presence or susceptibility to a rejection as well as any of the raw or intermediate data used in determining such a value or designation.

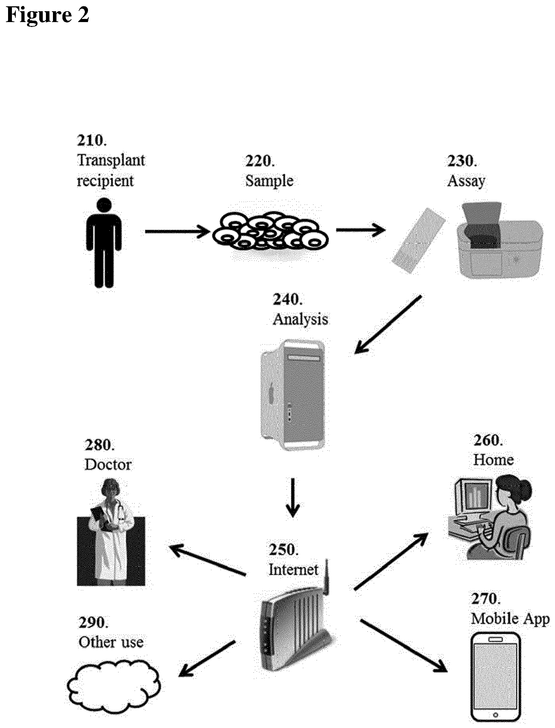

A typical computer (see US 6,785,613 Figs. 4 and 5) includes a bus which interconnects major subsystems such as a central processor, a system memory, an input/output controller, an external device such as a printer via a parallel port, a display screen via a display adapter, a serial port, a keyboard, a fixed disk drive and a floppy disk drive operative to receive a floppy disk. Many other devices can be connected such as a scanner via I/O controller, a mouse connected to serial port or a network interface. The computer contains computer readable media holding codes to allow the computer to perform a variety of functions. These functions include controlling automated apparatus, receiving input and delivering output as described above. The automated apparatus can include a robotic arm for delivering reagents for determining expression levels, as well as small vessels, e.g., microtiter wells for performing the expression analysis. The methods, systems, kits and compositions provided herein may also be capable of generating and transmitting results through a computer network. As shown in **Figure 2,** a sample (220) is first collected from a subject (e.g. transplant recipient, 210). The sample is assayed (230) and gene expression products are generated. A computer system (240) is used in analyzing the data and making classification of the sample. The result is capable of being transmitted to different types of end users via a computer network (250). In some instances, the subject (e.g. patient) may be able to access the result by using a standalone software and/or a web-based application on a local computer capable of accessing the internet (260). In some instances, the result can be accessed via a mobile application (270) provided to a mobile digital processing device (e.g. mobile phone, tablet, etc.). In some instances, the result may be accessed by physicians and help them identify and track conditions of their patients (280). In some instances, the result may be used for other purposes (290) such as education and research.

## EXAMPLES

The following examples are offered to illustrate, but not to limit the present invention.

Example 1. Differentially expressed genes associated with kidney transplant rejections

This Example describes global analysis of gene expressions in kidney transplant patients with different types of rejections or injuries.

A total of biopsy-documented 274 kidney biopsy samples from the Transplant Genomics Collaborative Group (TGCG) were processed on the Affymetrix HG-U133 PM only peg microarrays. The 274 samples that were analyzed comprised of 4 different phenotypes: Acute Rejection (AR; n=75); Acute Dysfunction No Rejection (ADNR; n=39); Chronic Allograft Nephropathy (CAN; n=61); and Transplant Excellent (TX; n=99).

Signal Filters: To eliminate low expressed signals we used a signal filter cut-off that was data driven, and expression signals < Log2 4.23 in all samples were eliminated leaving us with 48882 probe sets from a total of 54721 probe sets.

**4-Way AR/ADNR/CAN/TX classifier:** We first did a 4 way comparison of the AR, ADNR, CAN and TX samples. The samples comprised of four different classes a 4-way ANOVA analysis yielded more than 10,000 differentially expressed genes even at a stringent p value cut-off of < 0.001. Since we were trying to discover a signature that could differentiate these four classes we used only the top 200 differentially expressed probe sets to build predictive models. We ran the Nearest Centroid (NC) algorithm to build the predictive models. When we used the top 200 differentially expressed probe sets between all four phenotypes, the best predictor model was based on 199 probe sets.

Nearest Centroid (NC) classification takes the gene expression profile of a new sample, and compares it to each of the existing class centroids. The class whose centroid that it is closest to, in squared distance, is the predicted class for that new sample. It also provides the centroid distances for each sample to each of the possible phenotypes being tested. In other words, in a 2-way classifier like AR vs. TX, the tool provides the "best" classification and provides the centroid distances to the two possible outcomes: TX and AR.

We observed in multiple datasets that there are 4 classes of predictions made. First, are correctly classified as TX by both biopsy and NC. Second, are correctly classified as AR by both biopsy and NC. Third, are truly misclassified samples. In other words, the biopsy says one thing and the molecular profile another. In these cases, the centroid distances for the given classifications are dramatically different, making the molecular classification very straightforward and simply not consistent with the biopsy phenotype assigned. Whether this is because the gold standard biopsy classification is wrong or the molecular classification is wrong is impossible to know at this point.

However, there is a fourth class that we call "mixed" classifications. In these cases supposedly "misclassified" samples by molecular profile show a nearest centroid distance that is not very different when compared to that of the "correct" classification based on the biopsy. In other words, the nearest centroid distances of most of these misclassified "mixed" samples are actually very close to the correct biopsy classification. However, because NC has no rules set to deal with the mixed situation it simply calls the sample by the nominally higher centroid distance.

The fact is that most standard implementations of class prediction algorithms currently available treat all classes as dichotomous variables (yes/no diagnostically). They are not designed to deal with the reality of medicine that molecular phenotypes of clinical samples can actually represent a continuous range of molecular scores based on the expression signal intensities with complex implications for the diagnoses. Thus, "mixed" cases where the centroid distances are only slightly higher for TX than AR is still classified as a TX, even if the AR distances are only slightly less. In this case, where there is a mixture of TX and AR by expression, it is obvious that the case is actually an AR for a transplant clinician, not a TX. Perhaps just a milder form of AR and this is the reason for using thresholding.

Thus, we set a threshold for the centroid distances. The threshold is driven by the data. The threshold equals the mean difference NC provides in centroid distances for the two possible classifications (i.e. AR vs. TX) for all correctly classified samples in the data set (e.g. classes 1 and 2 of the 4 possible outcomes of classification). This means that for the "mixed" class of samples, if a biopsy-documented sample was misclassified by molecular profiling, but the misclassification was within the range of the mean calculated centroid distances of the true classifications in the rest of the data, then that sample would not be considered as a misclassified sample.

**Table 1** shows the performance of the 4 way AR, ADNR, CAN, TX NC classifier using such a data driven threshold. So, using the top 200 differentially expressed probesets from a 4-way AR, ADNR, CAN and TX ANOVA with a Nearest Centroid classifier, we are able to molecularly classify the 4 phenotypes at 97% accuracy. Smaller classifier sets did not afford any significant increase in the predictive accuracies. To validate this data we applied this classification to an externally collected data set. These were samples collected at the University of Sao Paolo in Brazil. A total of 80 biopsy-documented kidney biopsy samples were processed on the same Affymetrix HG-U133 PM only peg microarrays. These 80 samples that were analyzed comprised of the same 4 different phenotypes: AR (n=23); ADNR (n=11); CAN (n=29); and TX (n=17).

We performed the classification based on the "locked "NC predictor (meaning that none of the thresholding parameters were changed. **Table 2** shows the performance of our locked 4 way AR, ADNR, CAN, TX NC classifier in the Brazilian cohort. So, using the top 200 differentially expressed probesets from a 4-way AR, ADNR, CAN and TX ANOVA with a "locked" Nearest Centroid classifier we are able to molecularly classify the 4 phenotypes with similar accuracy in an independently and externally collected validation set. This validates our molecular classifier of the biopsy on an independent external data set. It also demonstrates that the classifier is not subject to influence based on significant racial differences represented in the Brazilian population.

**3-Way AR/ADNR/TX classifier:** Similarly, we did a 3 way comparison of the AR, ADNR and TX samples since these are the most common phenotypes encountered during the early post-transplant period with CAN usually being a late manifestation of graft injury which is progressive. The samples comprised of these 3 different classes, and a 4-way ANOVA analysis again yielded more than 10,000 differentially expressed genes, so we used only the top 200 differentially expressed probe sets to build predictive models. We ran the Nearest Centroid (NC) algorithm to build the predictive models. When we used the top 200 differentially expressed probe sets between all four phenotypes the best predictor model was based on 197 probe sets.

**Table 3** shows the performance of the 3 way AR, ADNR, TX NC classifier with which we are able to molecularly classify the 3 phenotypes at 98% accuracy in the TGCG cohort. Similarly the locked 3 way classifier performs equally well on the Brazilian cohort with 98% accuracy **(Table 4)**. Therefore, our 3 way classifier also validates on the external data set.

**2-Way CAN/TX classifier:** Finally we also did a 2 way comparison of the CAN and TX samples. The samples comprised of these 2 classes with an ANOVA analysis again yielded ~11,000 differentially expressed genes, so we used only the top 200 differentially expressed probe sets to build predictive models. We ran the Nearest Centroid (NC) algorithm to build the predictive models. When we used the top 200 differentially expressed probe sets the best predictor model was based on all 200 probe sets. **Table 5** shows the performance of the 2 way CAN, TX NC classifier with which we are able to molecularly classify the 4 phenotypes at 97% accuracy in the TGCG cohort. This locked classifier performs equally well on the Brazilian cohort with 95% accuracy **(Table 6).** Again we show that our 2 way CAN, TX classifier also validates on the external data set.

In another example of the 2-way classifier similar to the 2-way CAN/TX classifier described earlier, **Table 10** shows the top 400 probe sets of 2-Way Classifier AR vs. TX and **Table 11** shows the top 400 probe sets of 2-Way Classifier CAN/IFTA vs. TX.

**Clinical Applications**

The methods, compositions, systems and kits provided herein can be used to detect, diagnose, predict or monitor a condition of a transplant recipient. In some instances, the methods, compositions, systems and kits described herein provide information to a medical practitioner that can be useful in making a therapeutic decision. Therapeutic decisions may include decisions to: continue with a particular therapy, modify a particular therapy, alter the dosage of a particular therapy, stop or terminate a particular therapy, altering the frequency of a therapy, introduce a new therapy, introduce a new therapy to be used in combination with a current therapy, or any combination of the above

*Detecting/Diagnosing a Condition of a Transplant Recipient*

The methods, compositions, systems and kits provided herein are particularly useful for detecting or diagnosing a condition of a transplant recipient such as a condition the transplant recipient has at the time of testing. Exemplary conditions that can be detected or diagnosed with the present methods include organ transplant rejection, acute rejection (AR), chronic rejection, Acute Dysfunction with No Rejection (ADNR), normal transplant function (TX) and/or Sub-Clinical Acute Rejection (SubAR). The methods provided herein are particularly useful for transplant recipients who have received a kidney transplant. Exemplary conditions that can be detected or diagnosed in such kidney transplant recipients include: AR, chronic allograft nephropathy (CAN), ADNR, SubAR, IF/TA, and TX.

The diagnosis or detection of condition of a transplant recipient may be particularly useful in limiting the number of invasive diagnostic interventions that are administered to the patient. For example, the methods provided herein may limit or eliminate the need for a transplant recipient (e.g., kidney transplant recipient) to receive a biopsy (e.g., kidney biopsies) or to receive multiple biopsies. In some instances, the methods provided herein may also help interpreting a biopsy result, especially when the biopsy result is inconclusive.

In a further embodiment, the methods provided herein can be used alone or in combination with other standard diagnosis methods currently used to detect or diagnose a condition of a transplant recipient, such as but not limited to results of biopsy analysis for kidney allograft rejection, results of histopathology of the biopsy sample, serum creatinine level, creatinine clearance, ultrasound, radiological imaging results for the kidney, urinalysis results, elevated levels of inflammatory molecules such as neopterin, and lymphokines, elevated plasma interleukin (IL)-1 in azathioprine-treated patients, elevated IL-2 in cyclosporine-treated patients, elevated IL-6 in serum and urine, intrarenal expression of cytotoxic molecules (granzyme B and perforin) and immunoregulatory cytokines (IL-2, -4, - 10, interferon gamma and transforming growth factor-bl).

The methods provided herein are useful for distinguishing between two or more conditions or disorders (e.g., AR vs ADNR, SubAR vs ADNR, etc.). In some instances, the methods are used to determine whether a transplant recipient has AR, ADNR or TX. In some instances, the methods are used to determine whether a transplant recipient has AR, ADNR, SubAR and/ or TX, or any subset or combination thereof. In some instances, the methods are used to determine whether a transplant recipient has AR, ADNR, SubAR, TX, HCV, or any subset or combination thereof. As previously described, elevated serum creatinine levels from baseline levels in kidney transplant recipients may be indicative of AR or ADNR. In preferred embodiments, the methods provided herein are used to distinguish AR from ADNR in a kidney transplant recipient. In some preferred embodiments, the methods provided herein are used to distinguish AR from ADNR in a liver transplant recipient. In some instances, the methods are used to determine whether a transplant recipient has AR, ADNR, SubAR , TX, acute transplant dysfunction, transplant dysfunction, transplant dysfunction with no rejection, or any subset or combination thereof. In some instances, the methods provided herein are used to distinguish AR from ADNR from CAN a kidney transplant recipient.

*Predicting a Condition of a Transplant Recipient*

In some embodiments, the methods provided herein can predict AR prior to actual onset of the conditions. In some instances, the methods provided herein can predict AR, IFTA, CAN, ADNR, SubAR or other disorders in a transplant recipient at least 1 day, 5 days, 10 days, 30 days, 50 days or 100 days prior to onset. In other instances, the methods provided herein can predict AR, IFTA, CAN, ADNR, SubAR or other disorders in a transplant recipient at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or 31 days prior to onset. In other instances, the methods provided herein can predict AR, IFTA, CAN, ADNR, SubAR or other disorders in a transplant recipient at least 1, 2, 3, 4, 5, 6, 7, 8, 9, 10, 11, 12 months prior to onset.

*Monitoring a Condition of a Transplant Recipient*

Provided herein are methods, systems, kits and compositions for monitoring a condition of a transplant recipient. Often, the monitoring is conducted by serial testing, such as serial non-invasive tests, serial minimally-invasive tests (e.g., blood draws), serial invasive tests (biopsies), or some combination thereof. Preferably, the monitoring is conducted by administering serial non-invasive tests or serial minimally-invasive tests (e.g., blood draws).

In some instances, the transplant recipient is monitored as needed using the methods described herein. Alternatively the transplant recipient may be monitored hourly, daily, weekly, monthly, yearly or at any pre-specified intervals. In some instances, the transplant recipient is monitored at least once every 1, 2, 3, 4, 5, 6, 7, 8, 9, 10, 11, 12, 13, 14, 15, 16, 17, 18, 19, 20, 21, 22, 23 or 24 hours. In some instances the transplant recipient is monitored at least once every 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or 31 days. In some instances, the transplant recipient is monitored at least once every 1, 2, 3, 4, 5, 6, 7, 8, 9, 10, 11, 12 months. In some instances, the transplant recipient is monitored at least once every 1, 2, 3, 4, 5, 6, 7, 8, 9, 10 years or longer, for the lifetime of the patient and the graft.

In some instances, gene expression levels in the patients can be measured, for example, within, one month, three months, six months, one year, two years, five years or ten years after a transplant. In some methods, gene expression levels are determined at regular intervals, e.g., every 3 months, 6 months or every year post-transplant, either indefinitely, or until evidence of a condition is observed, in which case the frequency of monitoring is sometimes increased. In some methods, baseline values of expression levels are determined in a subject before a transplant in combination with determining expression levels at one or more time points thereafter.

The results of diagnosing, predicting, or monitoring a condition of a transplant recipient may be useful for informing a therapeutic decision such as determining or monitoring a therapeutic regimen. In some instances, determining a therapeutic regimen may comprise administering a therapeutic drug. In some instances, determining a therapeutic regimen comprises modifying, continuing, initiating or stopping a therapeutic regimen. In some instances, determining a therapeutic regimen comprises treating the disease or condition. In some instances, the therapy is an immunosuppressive therapy. In some instances, the therapy is an antimicrobial therapy. In other instances, diagnosing, predicting, or monitoring a disease or condition comprises determining the efficacy of a therapeutic regimen or determining drug resistance to the therapeutic regimen.

Modifying the therapeutic regimen may comprise terminating a therapy. Modifying the therapeutic regimen may comprise altering a dosage of a therapy. Modifying the therapeutic regimen may comprise altering a frequency of a therapy. Modifying the therapeutic regimen may comprise administering a different therapy. In some instances, the results of diagnosing, predicting, or monitoring a condition of a transplant recipient may be useful for informing a therapeutic decision such as removal of the transplant. In some instances, the removal of the transplant can be an immediate removal. In other instances, the therapeutic decision can be a retransplant. Other examples of therapeutic regimen can include a blood transfusion in instances where the transplant recipient is refractory to immunosuppressive or antibody therapy.

Examples of therapeutic regimen can include administering compounds or agents that are e.g., compounds or agents having immunosuppressive properties (e.g., a calcineurin inhibitor, cyclosporine A or FK 506); a mTOR inhibitor (e.g., rapamycin, 40-O-(2-hydroxyethyl)-rapamycin, CCI779, ABT578, AP23573, biolimus-7 or biolimus-9); an ascomycin having immuno-suppressive properties (e.g., ABT-281, ASM981, etc.); corticosteroids; cyclophosphamide; azathioprene; methotrexate; leflunomide; mizoribine; mycophenolic acid or salt; mycophenolate mofetil; 15-deoxyspergualine or an immunosuppressive homologue, analogue or derivative thereof; a PKC inhibitor (e.g., as disclosed in WO 02/38561 or WO 03/82859); a JAK3 kinase inhibitor (e.g., N-benzyl-3,4-dihydroxy-benzylidene-cyanoacetamide a-cyano-(3,4-dihydroxy)-]N-benzylcinnamamide (Tyrphostin AG 490), prodigiosin 25-C(PNU156804), [4-(4'-hydroxyphenyl)-amino-6,7-dimethoxyquinazoline] (WHI-P131), [4-(3'-bromo-4'-hydroxylphenyl)-amino-6,7-dimethoxyquinazoline] (WHI-P154), [4-(3',5'-dibromo-4'-hydroxylphenyl)-amino-6,7-dimethoxyquinazoline] WHI-P97, KRX-211, 3-{(3R,4R)-4-methyl-3-[methyl-(7H-pyrrolo[2,3-d]pyrimidin-4-yl)-amino]-pi- peridin-1-yl}-3-oxo-propionitrile, in free form or in a pharmaceutically acceptable salt form, e.g., mono-citrate (also called CP-690,550), or a compound as disclosed in WO 04/052359 or WO 05/066156); a SIP receptor agonist or modulator (e.g., FTY720 optionally phosphorylated or an analog thereof, e.g., 2-amino-2-[4-(3-benzyloxyphenylthio)-2-chlorophenyl]ethyl-1,3-propanediol optionally phosphorylated or 1-{4-[1-(4-cyclohexyl-3-trifluoromethyl-benzyloxyimino)-ethyl]-2-ethyl-be-nzyl}-azetidine-3-carboxylic acid or its pharmaceutically acceptable salts); immunosuppressive monoclonal antibodies (e.g., monoclonal antibodies to leukocyte receptors, e.g., MHC, CD2, CD3, CD4, CD7, CD8, CD25, CD28, CD40, CD45, CD52, CD58, CD80, CD86 or their ligands); other immunomodulatory compounds (e.g., a recombinant binding molecule having at least a portion of the extracellular domain of CTLA4 or a mutant thereof, e.g., an at least extracellular portion of CTLA4 or a mutant thereof joined to a non-CTLA4 protein sequence, e.g., CTLA4Ig (for ex. designated ATCC 68629) or a mutant thereof, e.g., LEA29Y); adhesion molecule inhibitors (e.g., LFA-1 antagonists, ICAM-1 or -3 antagonists, VCAM-4 antagonists or VLA-4 antagonists). These compounds or agents may also be used alone or in combination. Immunosuppressive protocols can differ in different clinical settings. In some instances, in AR, the first-line treatment is pulse methylprednisolone, 500 to 1000 mg, given intravenously daily for 3 to 5 days. In some instances, if this treatment fails, than OKT3 or polyclonal anti-T cell antibodies will be considered. In other instances, if the transplant recipient is still experiencing AR, antithymocyte globulin (ATG) may be used.

### Kidney Transplants

The methods, compositions, systems and kits provided herein are particularly useful for detecting or diagnosing a condition of a kidney transplant. Kidney transplantation may be needed when a subject is suffering from kidney failure, wherein the kidney failure may be caused by hypertension, diabetes melitus, kidney stone, inherited kidney disease, inflammatory disease of the nephrons and glomeruli, side effects of drug therapy for other diseases, etc. Kidney transplantation may also be needed by a subject suffering from dysfunction or rejection of a transplanted kidney.

Kidney function may be assessed by one or more clinical and/or laboratory tests such as complete blood count (CBC), serum electrolytes tests (including sodium, potassium, chloride, bicarbonate, calcium, and phosphorus), blood urea test, blood nitrogen test, serum creatinine test, urine electrolytes tests, urine creatinine test, urine protein test, urine fractional excretion of sodium (FENA) test, glomerular filtration rate (GFR) test. Kidney function may also be assessed by a renal biopsy. Kidney function may also be assessed by one or more gene expression tests. The methods, compositions, systems and kits provided herein may be used in combination with one or more of the kidney tests mentioned herein. The methods, compositions, systems and kits provided herein may be used before or after a kidney transplant. In some instances, the method may be used in combination with complete blood count. In some instances, the method may be used in combination with serum electrolytes (including sodium, potassium, chloride, bicarbonate, calcium, and phosphorus). In some instances, the method may be used in combination with blood urea test. In some instances, the method may be used in combination with blood nitrogen test. In some instances, the method may be used in combination with a serum creatinine test. In some instances, the method may be used in combination with urine electrolytes tests. In some instances, the method may be used in combination with urine creatinine test. In some instances, the method may be used in combination with urine protein test. In some instances, the method may be used in combination with urine fractional excretion of sodium (FENA) test. In some instances, the method may be used in combination with glomerular filtration rate (GFR) test. In some instances, the method may be used in combination with a renal biopsy. In some instances, the method may be used in combination with one or more other gene expression tests. In some instances, the method may be used when the result of the serum creatinine test indicates kidney dysfunction and/or transplant rejection. In some instances, the method may be used when the result of the glomerular filtration rate (GFR) test indicates kidney dysfunction and/or transplant rejection. In some instances, the method may be used when the result of the renal biopsy indicates kidney dysfunction and/or transplant rejection. In some instances, the method may be used when the result of one or more other gene expression tests indicates kidney dysfunction and/or transplant rejection.

**Computer program**

The methods, kits, and systems disclosed herein may include at least one computer program, or use of the same. A computer program may include a sequence of instructions, executable in the digital processing device's CPU, written to perform a specified task. Computer readable instructions may be implemented as program modules, such as functions, objects, Application Programming Interfaces (APIs), data structures, and the like, that perform particular tasks or implement particular abstract data types. In light of the disclosure provided herein, those of skill in the art will recognize that a computer program may be written in various versions of various languages.

The functionality of the computer readable instructions may be combined or distributed as desired in various environments. The computer program will normally provide a sequence of instructions from one location or a plurality of locations. In various embodiments, a computer program includes, in part or in whole, one or more web applications, one or more mobile applications, one or more standalone applications, one or more web browser plug-ins, extensions, add-ins, or add-ons, or combinations thereof.

Further disclosed herein are systems for classifying one or more samples and uses thereof. The system may comprise (a) a digital processing device comprising an operating system configured to perform executable instructions and a memory device; (b) a computer program including instructions executable by the digital processing device to classify a sample from a subject comprising: (i) a first software module configured to receive a gene expression profile of one or more genes from the sample from the subject; (ii) a second software module configured to analyze the gene expression profile from the subject; and (iii) a third software module configured to classify the sample from the subject based on a classification system comprising three or more classes. At least one of the classes may be selected from transplant rejection, transplant dysfunction with no rejection and normal transplant function. At least two of the classes may be selected from transplant rejection, transplant dysfunction with no rejection and normal transplant function. All three of the classes may be selected from transplant rejection, transplant dysfunction with no rejection and normal transplant function. Analyzing the gene expression profile from the subject may comprise applying an algorithm. Analyzing the gene expression profile may comprise normalizing the gene expression profile from the subject. In some instances, normalizing the gene expression profile does not comprise quantile normalization.

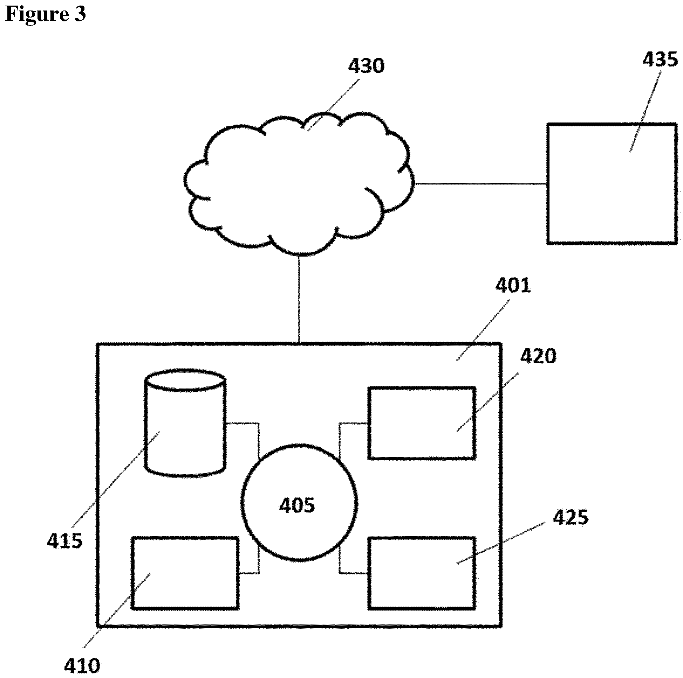

**Figure 3** shows a computer system (also "system" herein) **401** programmed or otherwise configured for implementing the methods of the disclosure, such as producing a selector set and/or for data analysis. The system **401** includes a central processing unit (CPU, also "processor" and "computer processor" herein) **405,** which can be a single core or multi core processor, or a plurality of processors for parallel processing. The system **401** also includes memory **410** (e.g., random-access memory, read-only memory, flash memory), electronic storage unit **415** (e.g., hard disk), communications interface **420** (e.g., network adapter) for communicating with one or more other systems, and peripheral devices **425,** such as cache, other memory, data storage and/or electronic display adapters. The memory **410,** storage unit **415,** interface **420** and peripheral devices **425** are in communication with the CPU **405** through a communications bus (solid lines), such as a motherboard. The storage unit **415** can be a data storage unit (or data repository) for storing data. The system **401** is operatively coupled to a computer network ("network") **430** with the aid of the communications interface **420.** The network **430** can be the Internet, an internet and/or extranet, or an intranet and/or extranet that is in communication with the Internet. The network **430** in some instances is a telecommunication and/or data network. The network **430** can include one or more computer servers, which can enable distributed computing, such as cloud computing.

The network **430** in some instances, with the aid of the system **401,** can implement a peer-to-peer network, which may enable devices coupled to the system **401** to behave as a client or a server.

The system **401** is in communication with a processing system **435**. The processing system **435** can be configured to implement the methods disclosed herein. In some examples, the processing system **435** is a nucleic acid sequencing system, such as, for example, a next generation sequencing system (e.g., Illumina sequencer, Ion Torrent sequencer, Pacific Biosciences sequencer). The processing system **435** can be in communication with the system **401** through the network **430,** or by direct (e.g., wired, wireless) connection. The processing system **435** can be configured for analysis, such as nucleic acid sequence analysis.

Methods as described herein can be implemented by way of machine (or computer processor) executable code (or software) stored on an electronic storage location of the system **401,** such as, for example, on the memory **410** or electronic storage unit **415**. During use, the code can be executed by the processor **405**. In some examples, the code can be retrieved from the storage unit **415** and stored on the memory **410** for ready access by the processor **405**. In some situations, the electronic storage unit **415** can be precluded, and machine-executable instructions are stored on memory **410**.

### Digital processing device

The methods, kits, and systems disclosed herein may include a digital processing device, or use of the same. In further embodiments, the digital processing device includes one or more hardware central processing units (CPU) that carry out the device's functions. In still further embodiments, the digital processing device further comprises an operating system configured to perform executable instructions. In some embodiments, the digital processing device is optionally connected a computer network. In further embodiments, the digital processing device is optionally connected to the Internet such that it accesses the World Wide Web. In still further embodiments, the digital processing device is optionally connected to a cloud computing infrastructure. In other embodiments, the digital processing device is optionally connected to an intranet. In other embodiments, the digital processing device is optionally connected to a data storage device.

In accordance with the description herein, suitable digital processing devices include, by way of non-limiting examples, server computers, desktop computers, laptop computers, notebook computers, sub-notebook computers, netbook computers, netpad computers, set-top computers, handheld computers, Internet appliances, mobile smartphones, tablet computers, personal digital assistants, video game consoles, and vehicles. Those of skill in the art will recognize that many smartphones are suitable for use in the system described herein. Those of skill in the art will also recognize that select televisions, video players, and digital music players with optional computer network connectivity are suitable for use in the system described herein. Suitable tablet computers include those with booklet, slate, and convertible configurations, known to those of skill in the art.

The digital processing device will normally include an operating system configured to perform executable instructions. The operating system is, for example, software, including programs and data, which manages the device's hardware and provides services for execution of applications. Those of skill in the art will recognize that suitable server operating systems include, by way of non-limiting examples, FreeBSD, OpenBSD, NetBSD®, Linux, Apple® Mac OS X Server®, Oracle® Solaris®, Windows Server®, and Novell® NetWare®. Those of skill in the art will recognize that suitable personal computer operating systems include, by way of non-limiting examples, Microsoft® Windows®, Apple® Mac OS X®, UNIX®, and UNIX-like operating systems such as GNU/Linux®. In some embodiments, the operating system is provided by cloud computing. Those of skill in the art will also recognize that suitable mobile smart phone operating systems include, by way of non-limiting examples, Nokia® Symbian® OS, Apple® iOS®, Research In Motion® BlackBerry OS®, Google® Android®, Microsoft® Windows Phone® OS, Microsoft® Windows Mobile® OS, Linux®, and Palm® WebOS®.

The device generally includes a storage and/or memory device. The storage and/or memory device is one or more physical apparatuses used to store data or programs on a temporary or permanent basis. In some embodiments, the device is volatile memory and requires power to maintain stored information. In some embodiments, the device is non-volatile memory and retains stored information when the digital processing device is not powered. In further embodiments, the non-volatile memory comprises flash memory. In some embodiments, the non-volatile memory comprises dynamic random-access memory (DRAM). In some embodiments, the non-volatile memory comprises ferroelectric random access memory (FRAM). In some embodiments, the non-volatile memory comprises phase-change random access memory (PRAM). In other embodiments, the device is a storage device including, by way of non-limiting examples, CD-ROMs, DVDs, flash memory devices, magnetic disk drives, magnetic tapes drives, optical disk drives, and cloud computing based storage. In further embodiments, the storage and/or memory device is a combination of devices such as those disclosed herein.

A display to send visual information to a user will normally be initialized. Examples of displays include a cathode ray tube (CRT, a liquid crystal display (LCD), a thin film transistor liquid crystal display (TFT-LCD, an organic light emitting diode (OLED) display. In various further embodiments, on OLED display is a passive-matrix OLED (PMOLED) or active-matrix OLED (AMOLED) display. In some embodiments, the display may be a plasma display , a video projector or a combination of devices such as those disclosed herein.

The digital processing device would normally include an input device to receive information from a user. The input device may be, for example, a keyboard, a pointing device including, by way of non-limiting examples, a mouse, trackball, track pad, joystick, game controller, or stylus;a touch screen, or a multi-touch screen, a microphone to capture voice or other sound input, a video camera to capture motion or visual input or a combination of devices such as those disclosed herein.

## Non-transitory computer readable storage medium

The methods, kits, and systems disclosed herein may include one or more non-transitory computer readable storage media encoded with a program including instructions executable by the operating system to perform and analyze the test described herein; preferably connected to a networked digital processing device. The computer readable storage medium is a tangible component of a digital that is optionally removable from the digital processing device. The computer readable storage medium includes, by way of non-limiting examples, CD-ROMs, DVDs, flash memory devices, solid state memory, magnetic disk drives, magnetic tape drives, optical disk drives, cloud computing systems and services, and the like. In some instances, the program and instructions are permanently, substantially permanently, semi-permanently, or non-transitorily encoded on the media.

A non-transitory computer-readable storage media may be encoded with a computer program including instructions executable by a processor to create or use a classification system. The storage media may comprise (a) a database, in a computer memory, of one or more clinical features of two or more control samples, wherein (i) the two or more control samples may be from two or more subjects; and (ii) the two or more control samples may be differentially classified based on a classification system comprising three or more classes; (b) a first software module configured to compare the one or more clinical features of the two or more control samples; and (c) a second software module configured to produce a classifier set based on the comparison of the one or more clinical features.

At least two of the classes may be selected from transplant rejection, transplant dysfunction with no rejection and normal transplant function. All three classes may be selected from transplant rejection, transplant dysfunction with no rejection and normal transplant function. The storage media may further comprise one or more additional software modules configured to classify a sample from a subject. Classifying the sample from the subject may comprise a classification system comprising three or more classes. At least two of the classes may be selected from transplant rejection, transplant dysfunction with no rejection and normal transplant function. All three classes may be selected from transplant rejection, transplant dysfunction with no rejection and normal transplant function.

## Web application

In some embodiments, a computer program includes a web application. In light of the disclosure provided herein, those of skill in the art will recognize that a web application, in various embodiments, utilizes one or more software frameworks and one or more database systems. In some embodiments, a web application is created upon a software framework such as Microsoft® .NET or Ruby on Rails (RoR). In some embodiments, a web application utilizes one or more database systems including, by way of non-limiting examples, relational, non-relational, object oriented, associative, and XML database systems. In further embodiments, suitable relational database systems include, by way of non-limiting examples, Microsoft® SQL Server, mySQL™, and Oracle®. Those of skill in the art will also recognize that a web application, in various embodiments, is written in one or more versions of one or more languages. A web application may be written in one or more markup languages, presentation definition languages, client-side scripting languages, server-side coding languages, database query languages, or combinations thereof. In some embodiments, a web application is written to some extent in a markup language such as Hypertext Markup Language (HTML), Extensible Hypertext Markup Language (XHTML), or eXtensible Markup Language (XML). In some embodiments, a web application is written to some extent in a presentation definition language such as Cascading Style Sheets (CSS). In some embodiments, a web application is written to some extent in a client-side scripting language such as Asynchronous Javascript and XML (AJAX), Flash® Actionscript, Javascript, or Silverlight®. In some embodiments, a web application is written to some extent in a server-side coding language such as Active Server Pages (ASP), ColdFusion®, Perl, Java™, JavaServer Pages (JSP), Hypertext Preprocessor (PHP), Python™, Ruby, Tcl, Smalltalk, WebDNA®, or Groovy. In some embodiments, a web application is written to some extent in a database query language such as Structured Query Language (SQL). In some embodiments, a web application integrates enterprise server products such as IBM® Lotus Domino®. In some embodiments, a web application includes a media player element. In various further embodiments, a media player element utilizes one or more of many suitable multimedia technologies including, by way of non-limiting examples, Adobe® Flash®, HTML 5, Apple® QuickTime®, Microsoft® Silverlight®, Java™, and Unity®.

**Mobile application**

In some embodiments, a computer program includes a mobile application provided to a mobile digital processing device. In some embodiments, the mobile application is provided to a mobile digital processing device at the time it is manufactured. In other embodiments, the mobile application is provided to a mobile digital processing device via the computer network described herein.

In view of the disclosure provided herein, a mobile application is created by techniques known to those of skill in the art using hardware, languages, and development environments known to the art. Those of skill in the art will recognize that mobile applications are written in several languages. Suitable programming languages include, by way of non-limiting examples, C, C++, C#, Objective-C, Java™, Javascript, Pascal, Object Pascal, Python™, Ruby, VB.NET, WML, and XHTML/HTML with or without CSS, or combinations thereof.

Suitable mobile application development environments are available from several sources. Commercially available development environments include, by way of non-limiting examples, AirplaySDK, alcheMo, Appcelerator®, Celsius, Bedrock, Flash Lite, .NET Compact Framework, Rhomobile, and WorkLight Mobile Platform. Other development environments are available without cost including, by way of non-limiting examples, Lazarus, MobiFlex, MoSync, and Phonegap. Also, mobile device manufacturers distribute software developer kits including, by way of non-limiting examples, iPhone and iPad (iOS) SDK, Android™ SDK, BlackBerry® SDK, BREW SDK, Palm® OS SDK, Symbian SDK, webOS SDK, and Windows® Mobile SDK.

Those of skill in the art will recognize that several commercial forums are available for distribution of mobile applications including, by way of non-limiting examples, Apple® App Store, Android™ Market, BlackBerry® App World, App Store for Palm devices, App Catalog for webOS, Windows® Marketplace for Mobile, Ovi Store for Nokia® devices, Samsung® Apps, and Nintendo® DSi Shop.

**Standalone application**

In some embodiments, a computer program includes a standalone application, which is a program that is run as an independent computer process, not an add-on to an existing process, e.g., not a plug-in. Those of skill in the art will recognize that standalone applications are often compiled. A compiler is a computer program(s) that transforms source code written in a programming language into binary object code such as assembly language or machine code. Suitable compiled programming languages include, by way of non-limiting examples, C, C++, Objective-C, COBOL, Delphi, Eiffel, Java™, Lisp, Python™, Visual Basic, and VB .NET, or combinations thereof. Compilation is often performed, at least in part, to create an executable program. In some embodiments, a computer program includes one or more executable complied applications.

**Web browser plug-in**

In some embodiments, the computer program includes a web browser plug-in. In computing, a plug-in is one or more software components that add specific functionality to a larger software application. Makers of software applications support plug-ins to enable third-party developers to create abilities which extend an application, to support easily adding new features, and to reduce the size of an application. When supported, plug-ins enable customizing the functionality of a software application. For example, plug-ins are commonly used in web browsers to play video, generate interactivity, scan for viruses, and display particular file types. Those of skill in the art will be familiar with several web browser plug-ins including, Adobe® Flash® Player, Microsoft® Silverlight®, and Apple® QuickTime®. In some embodiments, the toolbar comprises one or more web browser extensions, add-ins, or add-ons. In some embodiments, the toolbar comprises one or more explorer bars, tool bands, or desk bands.

In view of the disclosure provided herein, those of skill in the art will recognize that several plug-in frameworks are available that enable development of plug-ins in various programming languages, including, by way of non-limiting examples, C++, Delphi, Java™, PHP, Python™, and VB .NET, or combinations thereof.

Web browsers (also called Internet browsers) are software applications, designed for use with network-connected digital processing devices, for retrieving, presenting, and traversing information resources on the World Wide Web. Suitable web browsers include, by way of non-limiting examples, Microsoft® Internet Explorer®, Mozilla® Firefox®, Google® Chrome, Apple® Safari®, Opera Software® Opera®, and KDE Konqueror. In some embodiments, the web browser is a mobile web browser. Mobile web browsers (also called mircrobrowsers, mini-browsers, and wireless browsers) are designed for use on mobile digital processing devices including, by way of non-limiting examples, handheld computers, tablet computers, netbook computers, subnotebook computers, smartphones, music players, personal digital assistants (PDAs), and handheld video game systems. Suitable mobile web browsers include, by way of non-limiting examples, Google® Android® browser, RIM BlackBerry® Browser, Apple® Safari®, Palm® Blazer, Palm® WebOS® Browser, Mozilla® Firefox® for mobile, Microsoft® Internet Explorer® Mobile, Amazon® Kindle® Basic Web, Nokia® Browser, Opera Software® Opera® Mobile, and Sony® PSP™ browser.

**Software modules**

The methods, kits, and systems disclosed herein may include software, server, and/or database modules, or use of the same. In view of the disclosure provided herein, software modules are created by techniques known to those of skill in the art using machines, software, and languages known to the art. The software modules disclosed herein are implemented in a multitude of ways. In various embodiments, a software module comprises a file, a section of code, a programming object, a programming structure, or combinations thereof. In further various embodiments, a software module comprises a plurality of files, a plurality of sections of code, a plurality of programming objects, a plurality of programming structures, or combinations thereof. In various embodiments, the one or more software modules comprise, by way of non-limiting examples, a web application, a mobile application, and a standalone application. In some embodiments, software modules are in one computer program or application. In other embodiments, software modules are in more than one computer program or application. In some embodiments, software modules are hosted on one machine. In other embodiments, software modules are hosted on more than one machine. In further embodiments, software modules are hosted on cloud computing platforms. In some embodiments, software modules are hosted on one or more machines in one location. In other embodiments, software modules are hosted on one or more machines in more than one location.

**Databases**

The methods, kits, and systems disclosed herein may comprise one or more databases, or use of the same. In view of the disclosure provided herein, those of skill in the art will recognize that many databases are suitable for storage and retrieval of information pertaining to gene expression profiles, sequencing data, classifiers, classification systems, therapeutic regimens, or a combination thereof. In various embodiments, suitable databases include, by way of non-limiting examples, relational databases, non-relational databases, object oriented databases, object databases, entity-relationship model databases, associative databases, and XML databases. In some embodiments, a database is internet-based. In further embodiments, a database is web-based. In still further embodiments, a database is cloud computing-based. In other embodiments, a database is based on one or more local computer storage devices.

**Data transmission**

The methods, kits, and systems disclosed herein may be used to transmit one or more reports. The one or more reports may comprise information pertaining to the classification and/or identification of one or more samples from one or more subjects. The one or more reports may comprise information pertaining to a status or outcome of a transplant in a subject. The one or more reports may comprise information pertaining to therapeutic regimens for use in treating transplant rejection in a subject in need thereof. The one or more reports may comprise information pertaining to therapeutic regimens for use in treating transplant dysfunction in a subject in need thereof. The one or more reports may comprise information pertaining to therapeutic regimens for use in suppressing an immune response in a subject in need thereof.

The one or more reports may be transmitted to a subject or a medical representative of the subject. The medical representative of the subject may be a physician, physician's assistant, nurse, or other medical personnel. The medical representative of the subject may be a family member of the subject. A family member of the subject may be a parent, guardian, child, sibling, aunt, uncle, cousin, or spouse. The medical representative of the subject may be a legal representative of the subject.

It is understood that the examples and embodiments described herein are for illustrative purposes only and that various modifications or changes in light thereof will be suggested to persons skilled in the art and are to be included within the spirit and purview of this application and scope of the appended claims. Although any methods and materials similar or equivalent to those described herein can be used in the practice or testing of the present invention, the preferred methods and materials are described.

All publications, GenBank sequences, ATCC deposits, patents and patent applications cited herein are hereby expressly incorporated by reference in their entirety and for all purposes as if each is individually so denoted.

**Table 1: Biopsy Expression Profiling of Kidney Transplants: 4-Way Classifier AR vs. ADNR vs. CAN vs. TX (TGCG Samples)**

| Algorithm | Predictors | Comparison | Validation Cohort | | | | | |
|---|---|---|---|---|---|---|---|---|
| | | | AUC | Predictive Accuracy (%) | Sensitivity (%) | Specificity (%) | Postive Predictive Value (%) | Negative Predictive Value (%) |
| Nearest Centroid | 199 | AR vs. TX | 0.957 | 95 | 96 | 96 | 94 | 97 |
| Nearest Centroid | 199 | ADNR vs. TX | 0.977 | 97 | 94 | 100 | 100 | 97 |
| Nearest Centroid | 199 | CAN vs. TX | 0.992 | 99 | 98 | 100 | 100 | 99 |

**Table 2: Biopsy Expression Profiling of Kidney Transplants: 4-Way Classifier AR vs. ADNR vs. CAN vs. TX (Brazilian Samples)**

| Algorithm | Predictors | Comparison | Validation Cohort | | | | | |
|---|---|---|---|---|---|---|---|---|
| | | | AUC | Predictive Accuracy (%) | Sensitivity (%) | Specificity (%) | Postive Predictive Value (%) | Negative Predictive Value (%) |
| Nearest Centroid | 199 | AR vs. TX | 0.976 | 98 | 100 | 95 | 95 | 100 |
| Nearest Centroid | 199 | ADNR vs. TX | 1.000 | 100 | 100 | 100 | 100 | 100 |
| Nearest Centroid | 199 | CAN vs. TX | 1.000 | 100 | 100 | 100 | 100 | 100 |

EP 3 825 417 A2

**Table 3: Biopsy Expression Profiling of Kidney Transplants: 3-Way Classifier AR vs. ADNR vs. TX (TGCG Samples)**

| Algorithm | Predictors | Comparison | Validation Cohort | | | | | |
|---|---|---|---|---|---|---|---|---|
| | | | AUC | Predictive Accuracy (%) | Sensitivity (%) | Specificity (%) | Postive Pred ictive Value (%) | Negative Pred ictive Value (%) |
| Nearest Centroid | 197 | AR vs. TX | 0.979 | 98 | 96 | 100 | 100 | 96 |
| Nearest Centroid | 197 | ADNR vs. TX | 0.987 | 99 | 97 | 100 | 100 | 98 |
| Nearest Centroid | 197 | AR vs. ADNR | 0.968 | 97 | 100 | 93 | 95 | 100 |

**Table 4: Biopsy Expression Profiling of Kidney Transplants: 3-Wa Classifier AR vs. ADNR vs. TX (Brazilian Samples)**

| Algorithm | Predictors | Comparison | Validation Cohort | | | | | |
|---|---|---|---|---|---|---|---|---|
| | | | AUC | Predictive Accuracy (%) | Sensitivity (%) | Specificity (%) | Postive Pred ictive Value (%) | Negative Pred ictive Value (%) |
| Nearest Centroid | 197 | AR vs. TX | 0.976 | 98 | 100 | 95 | 95 | 100 |
| Nearest Centroid | 197 | ADNR vs. TX | 1.000 | 100 | 100 | 100 | 100 | 100 |
| Nearest Centroid | 197 | AR vs. ADNR | 0.962 | 97 | 100 | 91 | 95 | 100 |

**Table 5: Biopsy Expression Profiling of Kidney Transplants: 2-Wa Classifier CAN vs. TX (TGCG Samples)**

| Algorithm | Predictors | Comparison | Validation Cohort | | | | | |
|---|---|---|---|---|---|---|---|---|
| | | | AUC | Predictive Accuracy (%) | Sensitivity (%) | Specificity (%) | Postive Predictive Value (%) | Negative Predictive Value (%) |
| Nearest Centroid | 200 | AR vs. TX | 0.965 | 97 | 96 | 97 | 96 | 97 |

**Table 6: Biopsy Expression Profiling of Kidney Transplants: 2-Way Classifier CAN vs. TX (Brazilian Samples)**

| Algorithm | Predictors | Comparison | AUC | Predictive Accuracy (%) | Validation Cohort | | | |
|---|---|---|---|---|---|---|---|---|
| | | | | | Sensitivity (%) | Specificity (%) | Postive Predictive Value (%) | Negative Predictive Value (%) |
| Nearest Centroid | 200 | AR vs. TX | 0.954 | 95 | 95 | 96 | 95 | 96 |

Table 7. Biopsy Expression Profiling of Kidney Transplants: 4-Way Classifier AR vs. ADNR vs. CAN vs. TX (TGCG Samples)

| # | Probeset ID | Entrez Gene | Gene Symbol | Gene Title | p-value (Final Phenotype) | ADNR - Mean | AR - Mean | CAN - Mean | TX - Mean |
|---|---|---|---|---|---|---|---|---|---|
| 1 | 204446_PM_s_at | 240 | ALOX5 | arachidonate 5-lipoxygenase | 2.82E-34 | 91.9 | 323.9 | 216.7 | 54.7 |
| 2 | 202207_PM_at | 10123 | ARL4C | ADP-ribosylation factor-like 4C | 1.31E-32 | 106.9 | 258.6 | 190.4 | 57.2 |
| 3 | 204698_PM_at | 3669 | ISG20 | interferon stimulated exonuclease gene 20kDa | 1.50E-31 | 41.5 | 165.1 | 96.1 | 27.6 |
| 4 | 225701_PM_at | 80709 | AKNA | AT-hook transcription factor | 1.75E-31 | 37.7 | 102.8 | 73.2 | 29.0 |
| 5 | 207651_PM_at | 29909 | GPR171 | G protein-coupled receptor 171 | 6.30E-31 | 25.8 | 89.9 | 57.0 | 20.9 |
| 6 | 204205_PM_at | 60489 | APOBEC3G | apolipoprotein B mRNA editing enzyme, catalytic polypeptide-like 3G | 1.27E-30 | 95.4 | 289.4 | 192.0 | 78.7 |
| 7 | 208948_PM_s_at | 6780 | STAU1 | staufen, RNA binding protein, homolog 1 (Drosophila) | 1.37E-30 | 1807.9 | 1531.8 | 1766.0 | 2467.4 |
| 8 | 217733_PM_s_at | 9168 | TMSB10 | thymosin beta 10 | 2.38E-30 | 4414.7 | 6331.3 | 5555.2 | 3529.0 |
| 9 | 205831_PM_at | 914 | CD2 | CD2 molecule | 2.73E-30 | 40.4 | 162.5 | 100.9 | 33.9 |
| 10 | 209083_PM_at | 11151 | CORO1A | coronin, actin binding protein, 1A | 5.57E-30 | 46.9 | 163.8 | 107.1 | 34.3 |
| 11 | 210915_PM_x_at | 28638 | TRBC2 | T cell receptor beta constant 2 | 5.60E-30 | 39.7 | 230.7 | 129.7 | 37.5 |
| 12 | 211368_PM_s_at | 834 | CASP1 | caspase 1, apoptosis-related cysteine peptidase (interleukin 1, beta, convertase) | 6.21E-30 | 102.6 | 274.3 | 191.4 | 81.8 |
| 13 | 201042_PM_at | 7052 | TGM2 | transglutaminase 2 (C polypeptide, protein-glutamine-gamma-glutamyltransferase) | 6.28E-30 | 131.8 | 236.5 | 172.6 | 80.1 |
| 14 | 227353_PM_at | 147138 | TMC8 | transmembrane channel-like 8 | 7.76E-30 | 19.8 | 64.2 | 42.7 | 16.6 |
| 15 | 1555852_PM_at | 100507463 | LOC100507463 | hypothetical LOC100507463 | 8.29E-30 | 78.9 | 202.6 | 154.2 | 70.9 |
| 16 | 226878-PM-at | 3111 | HLA-DOA | major histocompatibility complex, class II, DO alpha | 1.63E-29 | 102.0 | 288.9 | 201.4 | 94.3 |
| 17 | 238327_PM_at | 440836 | ODF3B | outer dense fiber of sperm tails 3B | 1.74E-29 | 32.8 | 81.4 | 58.5 | 26.1 |
| 18 | 229437_PM_at | 114614 | MIR155HG | MIR155 host gene (non-protein coding) | 1.78E-29 | 15.4 | 50.4 | 28.5 | 12.9 |
| 19 | 33304_PM_at | 3669 | ISG20 | interferon stimulated exonuclease gene 20kDa | 2.40E-29 | 33.2 | 101.3 | 63.4 | 22.1 |

(continued)

| # | Probeset ID | Entrez Gene | Gene Symbol | Gene Title | p-value (Final Phenotype) | ADNR - Mean | AR - Mean | CAN - Mean | TX - Mean |
|---|---|---|---|---|---|---|---|---|---|
| 20 | 226621_PM_at | 9180 | OSMR | oncostatin M receptor | 2.42E-29 | 545.6 | 804.5 | 682.9 | 312.1 |
| 21 | 1553906_PM_s_at | 221472 | FGD2 | FYVE, RhoGEF and PH domain containing 2 | 2.43E-29 | 104.6 | 321.0 | 219.3 | 71.9 |
| 22 | 1405_PM_i_at | 6352 | CCL5 | chemokine (C-C motif) ligand 5 | 2.54E-29 | 68.0 | 295.7 | 195.6 | 54.6 |
| 23 | 226219_PM_at | 257106 | ARHGAP30 | Rho GTPase activating protein 30 | 2.92E-29 | 46.4 | 127.9 | 91.8 | 37.5 |
| 24 | 204891_PM_s_at | 3932 | LCK | lymphocyte-specific protein tyrosine kinase | 3.79E-29 | 19.3 | 74.2 | 43.4 | 17.8 |
| 25 | 210538_PM_s_at | 330 | BIRC3 | baculoviral IAP repeat-containing 3 | 5.06E-29 | 106.7 | 276.7 | 199.1 | 84.6 |
| 26 | 202644_PM_s_at | 7128 | TNFAIP3 | tumor necrosis factor, alpha-induced protein 3 | 5.47E-29 | 169.8 | 380.4 | 278.2 | 136.6 |
| 27 | 227346_PM_at | 10320 | IKZF1 | IKAROS family zinc finger 1 (Ikaros) | 7.07E-29 | 24.9 | 79.7 | 53.3 | 19.8 |
| 28 | 202957_PM_at | 3059 | HCLS1 | hematopoietic cell-specific Lyn substrate 1 | 8.26E-29 | 119.2 | 299.5 | 229.9 | 82.2 |
| 29 | 202307_PM_s_at | 6890 | TAP1 | transporter 1, ATP-binding cassette, sub-family B (MDR/TAP) | 1.01E-28 | 172.4 | 420.6 | 280.0 | 141.0 |
| 30 | 202748_PM_at | 2634 | GBP2 | guanylate binding protein 2, interferon-inducible | 1.10E-28 | 196.7 | 473.0 | 306.7 | 141.0 |
| 31 | 211796_PM_s_at | 28638 /// 28639 | TRBC1 /// TRBC2 | T cell receptor beta constant 1 /// T cell receptor beta constant 2 | 1.31E-28 | 69.2 | 431.5 | 250.2 | 63.6 |
| 32 | 213160_PM_at | 1794 | DOCK2 | dedicator of cytokinesis 2 | 1.36E-28 | 33.7 | 92.6 | 66.0 | 27.8 |
| 33 | 211656_PM_x_at | 100133583 /// 3119 | HLA-DQB1 /// LOC100133583 | major histocompatibility complex, class II, DQ beta 1 /// HLA class II histocompatibili | 1.63E-28 | 211.3 | 630.2 | 459.8 | 208.2 |
| 34 | 223322_PM_at | 83593 | RASSF5 | Ras association (RalGDS/AF-6) domain family member 5 | 1.68E-28 | 41.5 | 114.4 | 79.3 | 39.2 |
| 35 | 205488_PM_at | 3001 | GZMA | granzyme A (granzyme 1, cytotoxic T-lymphocyte-associated serine esterase 3) | 1.72E-28 | 37.3 | 164.8 | 102.3 | 33.4 |
| 36 | 213603_PM_s_at | 5880 | RAC2 | ras-related C3 botulinum toxin substrate 2 (rho family, small GTP binding protein Rac2) | 1.87E-28 | 113.9 | 366.5 | 250.3 | 86.5 |

| # | Probeset ID | Entrez Gene | Gene Symbol | Gene Title | p-value (Final Phenotype) | ADNR - Mean | AR - Mean | CAN - Mean | TX - Mean |
|---|---|---|---|---|---|---|---|---|---|
| 37 | 229390_PM_at | 441168 | FAM26F | family with sequence similarity 26, member F | 1.94E-28 | 103.8 | 520.0 | 272.4 | 75.9 |
| 38 | 206804_PM_at | 917 | CD3G | CD3g molecule, gamma (CD3-TCR complex) | 1.99E-28 | 19.7 | 60.6 | 36.4 | 17.3 |
| 39 | 209795_PM_at | 969 | CD69 | CD69 molecule | 2.06E-28 | 17.6 | 57.6 | 40.6 | 15.2 |
| 40 | 219574_PM_at | 55016 | 1-Mar | membrane-associated ring finger (C3HC4) 1 | 2.07E-28 | 51.5 | 126.0 | 87.5 | 36.2 |
| 41 | 207320_PM_x_at | 6780 | STAU1 | staufen, RNA binding protein, homolog 1 (Drosophila) | 2.21E-28 | 1425.1 | 1194.6 | 1383.2 | 1945.3 |
| 42 | 218983_PM_at | 51279 | C1RL | complement component 1, r subcomponent-like | 2.97E-28 | 167.1 | 244.5 | 206.9 | 99.4 |
| 43 | 206011_PM_at | 834 | CASP1 | caspase 1, apoptosis-related cysteine peptidase (interleukin 1, beta, convertase) | 3.23E-28 | 74.5 | 198.0 | 146.2 | 60.7 |
| 44 | 213539_PM_at | 915 | CD3D | CD3d molecule, delta (CD3-TCR complex) | 5.42E-28 | 70.8 | 335.1 | 168.1 | 60.0 |
| 45 | 213193_PM_x_at | 28639 | TRBC1 | T cell receptor beta constant 1 | 5.69E-28 | 95.3 | 490.9 | 286.5 | 92.1 |
| 46 | 232543_PM_x_at | 64333 | ARHGAP9 | Rho GTPase activating protein 9 | 6.79E-28 | 31.7 | 99.1 | 62.3 | 25.8 |
| 47 | 200986_PM_at | 710 | SERPING1 | serpin peptidase inhibitor, clade G (C1 inhibitor), member 1 | 7.42E-28 | 442.7 | 731.5 | 590.2 | 305.3 |
| 48 | 213037_PM_x_at | 6780 | STAU1 | staufen, RNA binding protein, homolog 1 (Drosophila) | 9.35E-28 | 1699.0 | 1466.8 | 1670.1 | 2264.9 |
| 49 | 204670_PM_x_at | 3123 /// 3126 | HLA-DRB1 /// HLA-DRB4 | major histocompatibility complex, class II, DR beta 1 /// major histocompatibility comp | 1.03E-27 | 2461.9 | 4344.6 | 3694.9 | 2262.0 |
| 50 | 217028_PM_at | 7852 | CXCR4 | chemokine (C-X-C motif) receptor 4 | 1.52E-27 | 100.8 | 304.4 | 208.4 | 75.3 |
| 51 | 203761_PM_at | 6503 | SLA | Src-like-adaptor | 1.61E-27 | 69.6 | 179.2 | 138.4 | 51.4 |
| 52 | 201137_PM_s_at | 3115 | HLA-DPB1 | major histocompatibility complex, class II, DP beta 1 | 1.95E-27 | 1579.1 | 3863.7 | 3151.7 | 1475.9 |

(continued)

| # | Probeset ID | Entrez Gene | Gene Symbol | Gene Title | p-value (Final Phenotype) | ADNR-Mean | AR-Mean | CAN-Mean | TX-Mean |
|---|---|---|---|---|---|---|---|---|---|
| 53 | 205269_PM_at | 3937 | LCP2 | lymphocyte cytosolic protein 2 (SH2 domain containing leukocyte protein of 76kDa) | 2.16E-27 | 30.2 | 92.2 | 58.9 | 22.9 |
| 54 | +205821_PM_at | 22914 | KLRK1 | killer cell lectin-like receptor subfamily K, member 1 | 2.56E-27 | 30.3 | 111.5 | 72.5 | 31.3 |
| 55 | 204655_PM_at | 6352 | CCL5 | chemokine (C-C motif) ligand 5 | 3.28E-27 | 77.5 | 339.4 | 223.4 | 66.8 |
| 56 | 226474_PM_at | 84166 | NLRC5 | NLR family, CARD domain containing 5 | 3.54E-27 | 64.4 | 173.5 | 129.8 | 55.1 |
| 57 | 212503_PM_s_at | 22982 | DIP2C | DIP2 disco-interacting protein 2 homolog C (Drosophila) | 3.69E-27 | 559.7 | 389.2 | 502.0 | 755.0 |
| 58 | 213857_PM_s_at | 961 | CD47 | CD47 molecule | 4.33E-27 | 589.6 | 858.0 | 703.2 | 481.2 |
| 59 | 206118_PM_at | 6775 | STAT4 | signal transducer and activator of transcription 4 | 4.58E-27 | 21.0 | 49.5 | 37.7 | 18.1 |
| 60 | 227344_PM_at | 10320 | IKZF1 | IKAROS family zinc finger 1 (Ikaros) | 5.87E-27 | 17.8 | 40.0 | 28.5 | 14.9 |
| 61 | 230550_PM_at | 64231 | MS4A6A | membrane-spanning 4-domains, subfamily A, member 6A | 5.98E-27 | 44.8 | 124.3 | 88.0 | 30.9 |
| 62 | 235529_PM_x_at | 25939 | SAMHD1 | SAM domain and HD domain 1 | 6.56E-27 | 189.3 | 379.9 | 289.1 | 128.0 |
| 63 | 205758_PM_at | 925 | CD8A | CD8a molecule | 7.28E-27 | 24.2 | 105.8 | 60.3 | 22.2 |
| 64 | 211366_PM_x_at | 834 | CASP1 | caspase 1, apoptosis-related cysteine peptidase (interleukin 1, beta, convertase) | 7.37E-27 | 115.3 | 261.0 | 186.0 | 87.1 |
| 65 | 209606_PM_at | 9595 | CYTIP | cytohesin 1 interacting protein | 7.48E-27 | 41.4 | 114.3 | 79.0 | 32.9 |
| 66 | 201721_PM_s_at | 7805 | LAPTM5 | lysosomal protein transmembrane 5 | 8.04E-27 | 396.5 | 934.6 | 661.3 | 249.4 |
| 67 | 204774_PM_at | 2123 | EVI2A | ecotropic viral integration site 2A | 8.14E-27 | 63.6 | 168.5 | 114.7 | 44.9 |
| 68 | 215005_PM_at | 54550 | NECAB2 | N-terminal EF-hand calcium binding protein 2 | 8.32E-27 | 36.7 | 23.4 | 30.9 | 65.7 |
| 69 | 229937_PM_x_at | 10859 | LILRB1 | Leukocyte immunoglobulin-like receptor, subfamily B (with TM and ITIM domains), member | 8.33E-27 | 23.5 | 79.9 | 50.0 | 18.5 |

| # | Probeset ID | Entrez Gene | Gene Symbol | Gene Title | p-value (Final Phenotype) | ADNR - Mean | AR - Mean | CAN - Mean | TX - Mean |
|---|---|---|---|---|---|---|---|---|---|
| 70 | 209515_PM_s_at | 5873 | RAB27A | RAB27A, member RAS oncogene family | 8.93E-27 | 127.3 | 192.2 | 160.5 | 85.2 |
| 71 | 242916_PM_at | 11064 | CEP110 | centrosomal protein 110kDa | 8.98E-27 | 30.8 | 68.1 | 51.2 | 26.2 |
| 72 | 205270_PM_s_at | 3937 | LCP2 | lymphocyte cytosolic protein 2 (SH2 domain containing leukocyte protein of 76kDa) | 9.04E-27 | 56.8 | 162.6 | 104.4 | 44.6 |
| 73 | 214022_PM_s_at | 8519 | IFITM1 | interferon induced transmembrane protein 1 (9-27) | 9.31E-27 | 799.1 | 1514.7 | 1236.6 | 683.3 |
| 74 | 1552703_PM_s_at | 114769 /// 834 | CARD16 /// CASP1 | caspase recruitment domain family, member 16 /// caspase 1, apoptosis-related cysteine | 1.01E-26 | 64.6 | 167.8 | 120.6 | 54.9 |
| 75 | 202720_PM_at | 26136 | TES | testis derived transcript (3 LIM domains) | 1.05E-26 | 285.4 | 379.0 | 357.9 | 204.2 |
| 76 | 202659_PM_at | 5699 | PSMB10 | proteasome (prosome, macropain) subunit, beta type, 10 | 1.10E-26 | 180.7 | 355.6 | 250.3 | 151.5 |
| 77 | 236295_PM_s_at | 197358 | NLRC3 | NLR family, CARD domain containing 3 | 1.19E-26 | 19.0 | 52.5 | 37.0 | 18.6 |
| 78 | 22904l_PM_s_at | --- | --- | --- | 1.31E-26 | 36.5 | 132.1 | 84.7 | 32.4 |
| 79 | 205798_PM_at | 3575 | IL7R | interleukin 7 receptor | 1.32E-26 | 44.1 | 136.8 | 106.3 | 33.4 |
| 80 | 209970_PM_x_at | 834 | CASP1 | caspase 1, apoptosis-related cysteine peptidase (interleukin 1, beta, convertase) | 1.36E-26 | 116.0 | 266.6 | 181.6 | 88.7 |
| 81 | 204336_PM_s_at | 10287 | RGS19 | regulator of G-protein signaling 19 | 1.54E-26 | 95.2 | 187.7 | 135.7 | 67.0 |
| 82 | 204912_PM_at | 3587 | IL10RA | interleukin 10 receptor, alpha | 1.61E-26 | 57.0 | 178.7 | 117.2 | 46.1 |
| 83 | 227184_PM_at | 5724 | PTAFR | platelet-activating factor receptor | 1.70E-26 | 89.8 | 191.4 | 134.4 | 62.7 |
| 84 | 209969_PM_s_at | 6772 | STAT1 | signal transducer and activator of transcription 1, 91kDa | 1.82E-26 | 395.8 | 1114.5 | 664.6 | 320.8 |
| 85 | 232617_PM_at | 1520 | CTSS | cathepsin S | 1.88E-26 | 209.6 | 537.9 | 392.2 | 154.8 |
| 86 | 224451_PM_x_at | 64333 | ARHGAP9 | Rho GTPase activating protein 9 | 1.94E-26 | 34.2 | 103.4 | 71.8 | 29.4 |
| 87 | 209670_PM_at | 28755 | TRAC | T cell receptor alpha constant | 2.06E-26 | 37.9 | 149.9 | 96.2 | 38.5 |

| # | Probeset ID | Entrez Gene | Gene Symbol | Gene Title | p-value (Final Phenotype) | ADNR - Mean | AR - Mean | CAN - Mean | TX - Mean |
|---|---|---|---|---|---|---|---|---|---|
| 88 | 1559584_PM_a_at | 283897 | C16orf54 | chromosome 16 open reading frame 54 | 2.22E-26 | 31.3 | 95.8 | 71.5 | 26.1 |
| 89 | 208306_PM_x_at | 3123 | HLA-DRB1 | Major histocompatibility complex, class II, DR beta 1 | 2.29E-26 | 2417.5 | 4278.5 | 3695.8 | 2255.0 |
| 90 | 229383_PM_at | 55016 | 1-Mar | membrane-associated ring finger (C3HC4) 1 | 2.36E-26 | 33.8 | 88.0 | 52.1 | 22.9 |
| 91 | 235735_PM_at | --- | --- | --- | 2.46E-26 | 13.0 | 34.9 | 24.5 | 11.2 |
| 92 | 203416_PM_at | 963 | CD53 | CD53 molecule | 2.56E-26 | 215.9 | 603.0 | 422.7 | 157.8 |
| 93 | 212504_PM_at | 22982 | DIP2C | DIP2 disco-interacting protein 2 homolog C (Drosophila) | 3.21E-26 | 334.5 | 227.2 | 289.4 | 452.5 |
| 94 | 204279_PM_at | 5698 | PSMB9 | proteasome (prosome, macropain) subunit, beta type, 9 (large multifunctional peptidase | 3.45E-26 | 241.6 | 637.4 | 419.4 | 211.3 |
| 95 | 235964_PM_x_at | 25939 | SAMHD1 | SAM domain and HD domain 1 | 3.60E-26 | 172.9 | 345.9 | 270.1 | 117.5 |
| 96 | 213566_PM_at | 6039 | RNASE6 | ribonuclease, RNase A family, k6 | 3.84E-26 | 180.9 | 482.0 | 341.1 | 134.3 |
| 97 | 221698_PM_s_at | 64581 | CLEC7A | C-type lectin domain family 7, member A | 4.00E-26 | 61.5 | 164.6 | 112.8 | 49.7 |
| 98 | 227125_PM_at | 3455 | IFNAR2 | interferon (alpha, beta and omega) receptor 2 | 4.03E-26 | 70.0 | 126.2 | 96.7 | 55.8 |
| 99 | 226525_PM_at | 9262 | STK17B | serine/threonine kinase 17b | 4.14E-26 | 146.8 | 338.7 | 259.1 | 107.6 |
| 100 | 221666_PM_s_at | 29108 | PYCARD | PYD and CARD domain containing | 4.95E-26 | 60.7 | 132.8 | 95.5 | 44.7 |
| 101 | 209774_PM_x_at | 2920 | CXCL2 | chemokine (C-X-C motif) ligand 2 | 5.73E-26 | 24.9 | 52.9 | 38.2 | 15.5 |
| 102 | 206082_PM_at | 10866 | HCP5 | HLA complex P5 | 5.98E-26 | 76.2 | 185.1 | 129.0 | 66.6 |
| 103 | 229391_PM_s_at | 441168 | FAM26F | family with sequence similarity 26, member F | 6.03E-26 | 98.6 | 379.7 | 212.1 | 73.7 |
| 104 | 229295_PM_at | 150166 /// 23765 | IL17RA/// LOC150166 | interleukin 17 receptor A /// hypothetical protein LOC150166 | 6.13E-26 | 76.4 | 131.8 | 98.4 | 50.0 |
| 105 | 202901_PM_x_at | 1520 | CTSS | cathepsin S | 6.32E-26 | 67.8 | 180.5 | 130.9 | 45.1 |

| # | Probeset ID | Entrez Gene | Gene Symbol | Gene Title | p-value (Final Phenotype) | ADNR - Mean | AR - Mean | CAN - Mean | TX - Mean |
|---|---|---|---|---|---|---|---|---|---|
| 106 | 226991_PM_at | 4773 | NFATC2 | nuclear factor of activated T-cells, cytoplasmic, calcineurin-dependent 2 | 6.49E-26 | 37.9 | 87.0 | 66.7 | 30.3 |
| 107 | 223280_PM_x_at | 64231 | MS4A6A | membrane-spanning 4-domains, subfamily A, member 6A | 6.72E-26 | 269.0 | 711.8 | 451.2 | 199.5 |
| 108 | 201601_PM_x_at | 8519 | IFITM1 | interferon induced transmembrane protein 1 (9-27) | 7.27E-26 | 1471.3 | 2543.5 | 2202.5 | 1251.1 |
| 109 | 1552701_PM_a_at | 114769 | CARD16 | caspase recruitment domain family, member 16 | 7.33E-26 | 143.8 | 413.6 | 273.3 | 119.8 |
| 110 | 229625_PM_at | 115362 | GBP5 | guanylate binding protein 5 | 7.80E-26 | 29.3 | 133.3 | 68.6 | 24.0 |
| 111 | 38149_PM_at | 9938 | ARHGAP25 | Rho GTPase activating protein 25 | 9.83E-26 | 51.3 | 108.9 | 83.2 | 43.2 |
| 112 | 203932_PM_at | 3109 | HLA-DMB | major histocompatibility complex, class II, DM beta | 1.03E-25 | 422.4 | 853.9 | 633.5 | 376.0 |
| 113 | 228964_PM_at | 639 | PRDM1 | PR domain containing 1, with ZNF domain | 1.15E-25 | 21.2 | 52.1 | 41.5 | 17.0 |
| 114 | 225799_PM_at | 112597 /// 541471 | LOC541471 /// NCRNA00152 | hypothetical LOC541471 /// non-protein coding RNA 152 | 1.23E-25 | 230.5 | 444.3 | 339.2 | 172.0 |
| 115 | 204118_PM_at | 962 | CD48 | CD48 molecule | 1.34E-25 | 82.9 | 341.5 | 212.4 | 65.2 |
| 116 | 211742_PM_s_at | 2124 | EVI2B | ecotropic viral integration site 2B | 1.36E-25 | 73.9 | 236.5 | 166.2 | 53.2 |
| 117 | 213416_PM_at | 3676 | ITGA4 | integrin, alpha 4 (antigen CD49D, alpha 4 subunit of VLA-4 receptor) | 1.47E-25 | 26.3 | 78.1 | 50.8 | 22.8 |
| 118 | 211991_PM_s_at | 3113 | HLA-DPA1 | major histocompatibility complex, class II, DP alpha 1 | 1.50E-25 | 1455.0 | 3605.4 | 2837.2 | 1462.9 |
| 119 | 232024_PM_at | 26157 | GIMAP2 | GTPase, IMAP family member 2 | 1.57E-25 | 90.2 | 197.7 | 146.7 | 72.5 |
| 120 | 205159_PM_at | 1439 | CSF2RB | colony stimulating factor 2 receptor, beta, low-affinity (granulocyte-macrophage) | 1.73E-25 | 33.7 | 107.5 | 70.4 | 26.3 |
| 121 | 228471_PM_at | 91526 | ANKRD44 | ankyrin repeat domain 44 | 1.79E-25 | 106.1 | 230.3 | 184.6 | 86.5 |
| 122 | 203332_PM_s_at | 3635 | INPP5D | inositol polyphosphate-5-phosphatase, 145kDa | 1.88E-25 | 27.9 | 60.5 | 42.6 | 24.0 |

(continued)

| # | Probeset ID | Entrez Gene | Gene Symbol | Gene Title | p-value (Final Phenotype) | ADNR-Mean | AR-Mean | CAN-Mean | TX-Mean |
|---|---|---|---|---|---|---|---|---|---|
| 123 | 223502_PM_s_at | 10673 | TNFSF13B | tumor necrosis factor (ligand) superfamily, member 13b | 2.02E-25 | 73.0 | 244.3 | 145.5 | 60.0 |
| 124 | 229723_PM_at | 117289 | TAGAP | T-cell activation RhoGTPase activating protein | 2.07E-25 | 29.2 | 82.9 | 55.9 | 26.2 |
| 125 | 206978_PM_at | 729230 | CCR2 | chemokine (C-C motif) receptor 2 | 2.17E-25 | 32.1 | 100.7 | 68.6 | 27.3 |
| 126 | 1555832_PM_s_at | 1316 | KLF6 | Kruppel-like factor 6 | 2.31E-25 | 899.4 | 1076.8 | 1003.3 | 575.1 |
| 127 | 211990_PM_at | 3113 | HLA-DPA1 | major histocompatibility complex, class II, DP alpha 1 | 2.53E-25 | 2990.7 | 5949.1 | 5139.9 | 3176.3 |
| 128 | 202018_PM_s_at | 4057 | LTF | lactotransferrin | 2.90E-25 | 392.3 | 1332.4 | 624.5 | 117.7 |
| 129 | 210644_PM_s_at | 3903 | LAIR1 | leukocyte-associated immunoglobulin-like receptor 1 | 2.90E-25 | 29.7 | 74.6 | 45.3 | 21.2 |
| 130 | 222294_PM_s_at | 5873 | RAB27A | RAB27A, member RAS oncogene family | 3.13E-25 | 198.7 | 309.1 | 263.0 | 146.4 |
| 131 | 238668_PM_at | --- | --- | --- | 3.29E-25 | 18.2 | 49.2 | 33.6 | 14.5 |
| 132 | 213975_PM_s_at | 4069 | LYZ | lysozyme | 3.31E-25 | 458.4 | 1626.0 | 1089.7 | 338.1 |
| 133 | 204220_PM_at | 9535 | GMFG | glia maturation factor, gamma | 3.46E-25 | 147.0 | 339.3 | 241.4 | 128.9 |
| 134 | 243366_PM_s_at | --- | --- | --- | 3.46E-25 | 24.7 | 72.1 | 52.5 | 22.0 |
| 135 | 221932_PM_s_at | 51218 | GLRX5 | glutaredoxin 5 | 3.64E-25 | 1351.5 | 1145.8 | 1218.1 | 1599.3 |
| 136 | 225415_PM_at | 151636 | DTX3L | deltex 3-like (Drosophila) | 3.77E-25 | 230.2 | 376.4 | 290.8 | 166.9 |
| 137 | 205466_PM_s_at | 9957 | HS3ST1 | heparan sulfate (glucosamine) 3-O-sulfotransferase 1 | 4.15E-25 | 73.6 | 123.8 | 96.0 | 42.1 |
| 138 | 200904_PM_at | 3133 | HLA-E | major histocompatibility complex, class I,E | 4.20E-25 | 1142.5 | 1795.2 | 1607.7 | 994.7 |
| 139 | 228442_PM_at | 4773 | NFATC2 | nuclear factor of activated T-cells, cytoplasmic, calcineurin-dependent 2 | 4.48E-25 | 39.0 | 84.8 | 62.8 | 32.0 |
| 140 | 204923_PM_at | 54440 | SASH3 | SAM and SH3 domain containing 3 | 4.49E-25 | 25.4 | 68.2 | 47.6 | 21.7 |
| 141 | 223640_PM_at | 10870 | HCST | hematopoietic cell signal transducer | 4.52E-25 | 91.0 | 234.0 | 158.3 | 72.7 |

(continued)

| # | Probeset ID | Entrez Gene | Gene Symbol | Gene Title | p-value (Final Phenotype) | ADNR - Mean | AR - Mean | CAN - Mean | TX - Mean |
|---|---|---|---|---|---|---|---|---|---|
| 142 | 211582 PM_x_at | 7940 | LST1 | leukocyte specific transcript 1 | 4.53E-25 | 57.5 | 183.8 | 121.2 | 49.4 |
| 143 | 219014 PM_at | 51316 | PLAC8 | placenta-specific 8 | 5.94E-25 | 38.8 | 164.1 | 88.6 | 30.7 |
| 144 | 210895_PM_s_at | 942 | CD86 | CD86 molecule | 6.21E-25 | 32.3 | 85.0 | 52.6 | 21.6 |
| 145 | AFFX-HUMISGF3A/M97935_3_at | 6772 | STAT1 | signal transducer and activator of transcription 1, 91kDa | 6.81E-25 | 642.1 | 1295.1 | 907.8 | 539.6 |
| 146 | 201315_PM_x_at | 10581 | IFITM2 | interferon induced transmembrane protein 2 (1-8D) | 6.87E-25 | 2690.9 | 3712.1 | 3303.7 | 2175.3 |
| 147 | 228532_PM_at | 128346 | C1orf162 | chromosome 1 open reading frame 162 | 7.07E-25 | 82.6 | 217.7 | 140.2 | 60.0 |
| 148 | 202376_PM_at | 12 | SERPINA3 | serpin peptidase inhibitor, clade A (alpha-1 antiproteinase, antitrypsin), member 3 | 7.13E-25 | 186.2 | 387.1 | 210.2 | 51.7 |
| 149 | 212587_PM_s_at | PM 5788 | PTPRC | protein tyrosine phosphatase, receptor type, C | 7.18E-25 | 114.8 | 398.6 | 265.7 | 90.3 |
| 150 | 223218_PM_s_at | 64332 | NFKBIZ | nuclear factor of kappa light polypeptide gene enhancer in B-cells inhibitor, zeta | 7.26E-25 | 222.6 | 497.9 | 399.9 | 159.1 |
| 151 | 224356_PM_x_at | 64231 | MS4A6A | membrane-spanning 4-domains, subfamily A, member 6A | 7.33E-25 | 150.6 | 399.6 | 249.6 | 111.2 |
| 152 | 206420_PM_at | 10261 | IGSF6 | immunoglobulin superfamily, member 6 | 7.58E-25 | 45.1 | 131.5 | 74.3 | 32.7 |
| 153 | 225764_PM_at | 2120 | ETV6 | ets variant 6 | 7.66E-25 | 92.6 | 133.0 | 112.8 | 77.0 |
| 154 | 1555756_PM_a_at | 64581 | CLEC7A | C-type lectin domain family 7, member A | 7.74E-25 | 16.6 | 45.4 | 28.9 | 13.2 |
| 155 | 226218_PM_at | 3575 | IL7R | interleukin 7 receptor | 8.14E-25 | 55.6 | 197.0 | 147.1 | 41.4 |
| 156 | 209198_PM_s_at | 23208 | SYT11 | synaptotagmin XI | 8.28E-25 | 30.0 | 45.3 | 41.8 | 22.8 |
| 157 | 202803_PM_s_at | 3689 | ITGB2 | integrin, beta 2 (complement component 3 receptor 3 and 4 subunit) | 9.57E-25 | 100.5 | 253.0 | 182.6 | 65.2 |
| 158 | 215049_PM_x_at | 9332 | CD163 | CD163 molecule | 9.85E-25 | 232.8 | 481.3 | 344.5 | 112.9 |

(continued)

| # | Probeset ID | Entrez Gene | Gene Symbol | Gene Title | p-value (Final Phenotype) | ADNR-Mean | AR-Mean | CAN-Mean | TX-Mean |
|---|---|---|---|---|---|---|---|---|---|
| 159 | 202953_PM_at | 713 | C1QB | complement component 1, q subcomponent, B chain | 9.99E-25 | 215.8 | 638.4 | 401.1 | 142.5 |
| 160 | 208091_PM_s_at | 81552 | VOPP1 | vesicular, overexpressed in cancer, prosurvival protein 1 | 1.02E-24 | 495.5 | 713.9 | 578.1 | 409.7 |
| 161 | 201288_PM_at | 397 | ARHGDIB | Rho GDP dissociation inhibitor (GDI) beta | 1.13E-24 | 354.9 | 686.8 | 542.2 | 308.1 |
| 162 | 213733_PM_at | 4542 | MYO1F | myosin IF | 1.27E-24 | 26.8 | 52.7 | 39.4 | 20.9 |
| 163 | 212588_PM_at | 5788 | PTPRC | protein tyrosine phosphatase, receptor type, C | 1.41E-24 | 94.4 | 321.0 | 217.7 | 76.4 |
| 164 | 242907_PM_at | --- | --- | --- | 1.49E-24 | 59.3 | 165.1 | 99.7 | 39.8 |
| 165 | 209619_PM_at | 972 | CD74 | CD74 molecule, major histocompatibility complex, class II invariant chain | 1.55E-24 | 989.0 | 1864.7 | 1502.3 | 864.9 |
| 166 | 239237_PM_at | - | - | - | 1.75E-24 | 15.9 | 34.9 | 25.3 | 14.5 |
| 167 | 217022_PM_s_at | 100126583 ///3493/// 3494 | IGHA1/// IGHA2 /// LOC100126583 | immunoglobulin heavy constant alpha 1/// immunoglobulin heavy constant alpha 2 (A2m ma | 1.80E-24 | 77.7 | 592.5 | 494.6 | 49.4 |
| 168 | 201859_PM_at | 5552 | SRGN | serglycin | 1.82E-24 | 1237.9 | 2171.9 | 1747.0 | 981.8 |
| 169 | 243418_PM_at | - | - | - | 1.88E-24 | 56.3 | 31.1 | 49.8 | 104.8 |
| 170 | 202531_PM_at | 3659 | IRF1 | interferon regulatory factor 1 | 1.93E-24 | 92.9 | 226.0 | 154.5 | 77.0 |
| 171 | 208966_PM_x_at | 3428 | IFI16 | interferon, gamma-inducible protein 16 | 1.98E-24 | 406.7 | 760.4 | 644.9 | 312.6 |
| 172 | 1555759_PM_a_at | 6352 | CCL5 | chemokine (C-C motif) ligand 5 | 2.02E-24 | 81.4 | 350.8 | 233.2 | 68.3 |
| 173 | 202643_PM_s_at | 7128 | TNFAIP3 | tumor necrosis factor, alpha-induced protein 3 | 2.11E-24 | 43.7 | 92.8 | 68.1 | 34.8 |
| 174 | 223922_PM_x_at | 64231 | MS4A6A | membrane-spanning 4-domains, subfamily A, member 6A | 2.22E-24 | 289.2 | 656.8 | 424.1 | 214.5 |
| 175 | 209374_PM_s_at | 3507 | IGHM | immunoglobulin heavy constant mu | 2.26E-24 | 61.8 | 437.0 | 301.0 | 45.0 |
| 176 | 227677_PM_at | 3718 | JAK3 | Janus kinase 3 | 2.29E-24 | 18.6 | 51.7 | 32.0 | 15.5 |

(continued)

| # | Probeset ID | Entrez Gene | Gene Symbol | Gene Title | p-value (Final Phenotype) | ADNR-Mean | AR-Mean | CAN-Mean | TX-Mean |
|---|---|---|---|---|---|---|---|---|---|
| 177 | 221840_PM_at | 5791 | PTPRE | protein tyrosine phosphatase, receptor type, E | 2.38E-24 | 71.0 | 133.2 | 102.5 | 51.8 |
| 178 | 200887_PM_s_at | 6772 | STAT1 | signal transducer and activator of transcription 1, 91kDa | 2.47E-24 | 1141.7 | 2278.6 | 1602.9 | 972.9 |
| 179 | 221875_PM_x_at | 3134 | HLA-F | major histocompatibility complex, class I, F | 2.72E-24 | 1365.3 | 2400.6 | 1971.8 | 1213.0 |
| 180 | 206513_PM_at | 9447 | AIM2 | absent in melanoma 2 | 2.87E-24 | 17.2 | 50.7 | 30.5 | 13.9 |
| 181 | 214574_PM_x_at | 7940 | LST1 | leukocyte specific transcript 1 | 2.95E-24 | 74.1 | 222.8 | 142.0 | 61.7 |
| 182 | 231776_PM_at | 8320 | EOMES | eomesodermin | 3.07E-24 | 24.0 | 63.9 | 43.5 | 22.4 |
| 183 | 205639_PM_at | 313 | AOAH | acyloxyacyl hydrolase (neutrophil) | 4.03E-24 | 30.3 | 72.6 | 45.3 | 25.2 |
| 184 | 201762_PM_s_at | 5721 | PSME2 | proteasome (prosome, macropain) activator subunit 2 (PA28 beta) | 4.45E-24 | 1251.6 | 1825.1 | 1423.4 | 1091.0 |
| 185 | 217986_PM_s_at | 11177 | BAZ1A | bromodomain adjacent to zinc finger domain, 1A | 4.79E-24 | 87.5 | 145.2 | 116.4 | 62.5 |
| 186 | 235229_PM_at | --- | --- | --- | 4.84E-24 | 50.9 | 210.6 | 135.0 | 41.9 |
| 187 | 204924_PM_at | 7097 | TLR2 | toll-like receptor 2 | 4.84E-24 | 96.8 | 162.0 | 116.6 | 66.6 |
| 188 | 202208_PM_s_at | 10123 | ARL4C | ADP-ribosylation factor-like 4C | 4.89E-24 | 54.0 | 99.6 | 77.0 | 42.2 |
| 189 | 227072_PM_at | 25914 | RTTN | rotatin | 5.01E-24 | 101.1 | 74.5 | 83.6 | 132.9 |
| 190 | 202206_PM_at | 10123 | ARL4C | ADP-ribosylation factor-like 4C | 5.08E-24 | 60.8 | 128.5 | 96.1 | 36.0 |
| 191 | 204563_PM_at | 6402 | SELL | selectin L | 5.11E-24 | 40.7 | 134.7 | 76.1 | 31.7 |
| 192 | 219386_PM_s_at | 56833 | SLAMF8 | SLAM family member 8 | 5.17E-24 | 28.2 | 92.1 | 52.2 | 19.4 |
| 193 | 218232_PM_at | 712 | C1QA | complement component 1, q subcomponent, A chain | 5.88E-24 | 128.8 | 287.1 | 197.0 | 85.8 |
| 194 | 232311_PM_at | 567 | B2M | Beta-2-microglobulin | 6.06E-24 | 42.3 | 118.6 | 83.7 | 35.2 |
| 195 | 219684_PM_at | 64108 | RTP4 | receptor (chemosensory) transporter protein 4 | 6.09E-24 | 63.1 | 129.3 | 93.7 | 50.4 |

(continued)

| # | Probeset ID | Entrez Gene | Gene Symbol | Gene Title | p-value (Final Phenotype) | ADNR-Mean | AR - Mean | CAN - Mean | TX - Mean |
|---|---|---|---|---|---|---|---|---|---|
| 196 | 204057_PM_at | 3394 | IRF8 | interferon regulatory factor 8 | 6.59E-24 | 89.8 | 184.8 | 134.9 | 71.4 |
| 197 | 208296_PM_x_at | 25816 | TNFAIP8 | tumor necrosis factor, alpha-induced protein 8 | 6.65E-24 | 136.9 | 242.5 | 195.1 | 109.6 |
| 198 | 204122_PM_at | 7305 | TYROBP | TYRO protein tyrosine kinase binding protein | 6.73E-24 | 190.5 | 473.4 | 332.8 | 143.3 |
| 199 | 224927_PM_at | 170954 | KIAA1949 | KIAA1949 | 6.87E-24 | 98.8 | 213.6 | 160.2 | 74.7 |

Table 8. Biopsy Expression Profiling of Kidney Transplants: 3-Way Classifier AR vs. ADNR vs. TX (TGCG Samples)

| # | Probeset ID | Entrez Gene | Gene Symbol | Gene Title | p-value (Final Phenotype) | ADNR - Mean | AR - Mean | TX - Mean |
|---|---|---|---|---|---|---|---|---|
| 1 | 242956_PM_at | 3417 | IDH1 | Isocitrate dehydrogenase 1 (NADP+), soluble | 2.95E-22 | 32.7 | 29.9 | 53.6 |
| 2 | 208948_PM_s_at | 6780 | STAU1 | staufen, RNA binding protein, homolog 1 (Drosophila) | 1.56E-29 | 1807.9 | 1531.8 | 2467.4 |
| 3 | 213037_PM_x_at | 6780 | STAU1 | staufen, RNA binding protein, homolog 1 (Drosophila) | 4.77E-27 | 1699.0 | 1466.8 | 2264.9 |
| 4 | 207320_PM_x_at | 6780 | STAU1 | staufen, RNA binding protein, homolog 1 (Drosophila) | 6.17E-28 | 1425.1 | 1194.6 | 1945.3 |
| 5 | 1555832_PM_s_at | 1316 | KLF6 | Kruppel-like factor 6 | 5.82E-23 | 899.4 | 1076.8 | 575.1 |
| 6 | 202376_PM_at | 12 | SERPINA3 | serpin peptidase inhibitor, clade A (alpha-1 antiproteinase, antitrypsin), member 3 | 1.05E-25 | 186.2 | 387.1 | 51.7 |
| 7 | 226621_PM_at | 9180 | OSMR | oncostatin M receptor | 1.28E-27 | 545.6 | 804.5 | 312.1 |
| 8 | 218983_PM_at | 51279 | C1RL | complement component 1, r subcomponent-like | 9.46E-25 | 167.1 | 244.5 | 99.4 |
| 9 | 215005_PM_at | 54550 | NECAB2 | N-terminal EF-hand calcium binding protein 2 | 1.95E-25 | 36.7 | 23.4 | 65.7 |
| 10 | 202720_PM_at | 26136 | TES | testis derived transcript (3 LIM domains) | 4.32E-24 | 285.4 | 379.0 | 204.2 |
| 11 | 240320_PM_at | 100131781 | C14orf164 | chromosome 14 open reading frame 164 | 1.64E-23 | 204.9 | 84.2 | 550.0 |
| 12 | 243418_PM_at | --- | --- | --- | 1.81E-24 | 56.3 | 31.1 | 104.8 |
| 13 | 205466_PM_s_at | 9957 | HS3ST1 | heparan sulfate (glucosamine) 3-O-sulfotransferase 1 | 5.64E-24 | 73.6 | 123.8 | 42.1 |
| 14 | 201042_PM_at | 7052 | TGM2 | transglutaminase 2 (C polypeptide, protein-glutamine-gamma-glutamyltransferase) | 3.87E-28 | 131.8 | 236.5 | 80.1 |
| 15 | 202018_PM_s_at | 4057 | LTF | lactotransferrin | 4.00E-25 | 392.3 | 1332.4 | 117.7 |
| 16 | 212503_PM_s_at | 22982 | DIP2C | DIP2 disco-interacting protein 2 homolog C (Drosophila) | 6.62E-27 | 559.7 | 389.2 | 755.0 |
| 17 | 215049_PM_x_at | 9332 | CD163 | CD163 molecule | 4.65E-23 | 232.8 | 481.3 | 112.9 |

(continued)

| # | Probeset ID | Entrez Gene | Gene Symbol | Gene Title | p-value (Final Phenotype) | ADNR - Mean | AR - Mean | TX - Mean |
|---|---|---|---|---|---|---|---|---|
| 18 | 209515_PM_s_at | 5873 | RAB27A | RAB27A, member RAS oncogene family | 2.11E-23 | 127.3 | 192.2 | 85.2 |
| 19 | 221932_PM_s_at | 51218 | GLRX5 | glutaredoxin 5 | 2.08E-22 | 1351.5 | 1145.8 | 1599.3 |
| 20 | 202207_PM_at | 10123 | ARL4C | ADP-ribosylation factor-like 4C | 1.83E-29 | 106.9 | 258.6 | 57.2 |
| 21 | 227697_PM_at | 9021 | SOCS3 | suppressor of cytokine signaling 3 | 2.93E-23 | 35.9 | 69.1 | 19.9 |
| 22 | 227072_PM_at | 25914 | RTTN | rotatin | 4.26E-23 | 101.1 | 74.5 | 132.9 |
| 23 | 201136_PM_at | 5355 | PLP2 | proteolipid protein 2 (colonic epithelium-enriched) | 2.71E-22 | 187.7 | 274.0 | 131.7 |
| 24 | 212504_PM_at | 22982 | DIP2C | DIP2 disco-interacting protein 2 homolog C (Drosophila) | 2.57E-25 | 334.5 | 227.2 | 452.5 |
| 25 | 200986_PM_at | 710 | SERPING1 | serpin peptidase inhibitor, clade G (C1 inhibitor), member 1 | 2.88E-26 | 442.7 | 731.5 | 305.3 |
| 26 | 203233_PM_at | 3566 | IL4R | interleukin 4 receptor | 6.30E-23 | 97.6 | 138.5 | 72.0 |
| 27 | 229295_PM_at | 150166 /// 23765 | IL17RA /// LOC150166 | interleukin 17 receptor A /// hypothetical protein LOC150166 | 6.40E-25 | 76.4 | 131.8 | 50.0 |
| 28 | 231358_PM_at | 83876 | MRO | maestro | 1.11E-22 | 199.7 | 81.2 | 422.1 |
| 29 | 201666_PM_at | 7076 | TIMP1 | TIMP metallopeptidase inhibitor 1 | 1.54E-22 | 1035.3 | 1879.4 | 648.0 |
| 30 | 209774_PM_x_at | 2920 | CXCL2 | chemokine (C-X-C motif) ligand 2 | 1.50E-26 | 24.9 | 52.9 | 15.5 |
| 31 | 217733_PM_s_at | 9168 | TMSB10 | thymosin beta 10 | 9.34E-27 | 4414.7 | 6331.3 | 3529.0 |
| 32 | 222939_PM_s_at | 117247 | SLC16A10 | solute carrier family 16, member 10 (aromatic amino acid transporter) | 2.55E-22 | 156.6 | 93.7 | 229.6 |
| 33 | 204924_PM_at | 7097 | TLR2 | toll-like receptor 2 | 2.11E-22 | 96.8 | 162.0 | 66.6 |
| 34 | 225415_PM_at | 151636 | DTX3L | deltex 3-like (Drosophila) | 3.04E-24 | 230.2 | 376.4 | 166.9 |
| 35 | 202206_PM_at | 10123 | ARL4C | ADP-ribosylation factor-like 4C | 1.16E-22 | 60.8 | 128.5 | 36.0 |

(continued)

| # | Probeset ID | Entrez Gene | Gene Symbol | Gene Title | p-value (Final Phenotype) | ADNR - Mean | AR - Mean | TX - Mean |
|---|---|---|---|---|---|---|---|---|
| 36 | 213857_PM_s_at | 961 | CD47 | CD47 molecule | 2.56E-27 | 589.6 | 858.0 | 481.2 |
| 37 | 235529_PM_x_at | 25939 | SAMHD1 | SAM domain and HD domain 1 | 4.49E-24 | 189.3 | 379.9 | 128.0 |
| 38 | 206693_PM_at | 3574 | IL7 | interleukin 7 | 3.12E-22 | 37.3 | 57.0 | 28.9 |
| 39 | 219033_PM_at | 79668 | PARP8 | poly (ADP-ribose) polymerase family, member 8 | 1.88E-22 | 47.9 | 79.2 | 35.7 |
| 40 | 201721_PM_s_at | 7805 | LAPTM5 | lysosomal protein transmembrane 5 | 3.72E-24 | 396.5 | 934.6 | 249.4 |
| 41 | 204336_PM_s_at | 10287 | RGS19 | regulator of G-protein signaling 19 | 3.52E-25 | 95.2 | 187.7 | 67.0 |
| 42 | 235964_PM_x_at | 25939 | SAMHD1 | SAM domain and HD domain 1 | 2.45E-23 | 172.9 | 345.9 | 117.5 |
| 43 | 208091_PM_s_at | 81552 | VOPP1 | vesicular, overexpressed in cancer, prosurvival protein 1 | 9.47E-25 | 495.5 | 713.9 | 409.7 |
| 44 | 204446_PM_s_at | 240 | ALOX5 | arachidonate 5-lipoxygenase | 6.25E-31 | 91.9 | 323.9 | 54.7 |
| 45 | 212703_PM_at | 83660 | TLN2 | talin 2 | 6.13E-23 | 270.8 | 159.7 | 357.5 |
| 46 | 213414_PM_s_at | 6223 | RPS19 | ribosomal protein S19 | 1.57E-22 | 4508.2 | 5432.1 | 4081.7 |
| 47 | 1565681_PM_s_at | 22982 | DIP2C | DIP2 disco-interacting protein 2 homolog C (Drosophila) | 2.57E-22 | 66.4 | 35.7 | 92.5 |
| 48 | 225764_PM_at | 2120 | ETV6 | ets variant 6 | 2.63E-23 | 92.6 | 133.0 | 77.0 |
| 49 | 227184_PM_at | 5724 | PTAFR | platelet-activating factor receptor | 1.73E-24 | 89.8 | 191.4 | 62.7 |
| 50 | 221840_PM_at | 5791 | PTPRE | protein tyrosine phosphatase, receptor type, E | 8.52E-23 | 71.0 | 133.2 | 51.8 |
| 51 | 225799_PM_at | 112597 /// 541471 | LOC541471 /// NCRNA00152 | hypothetical LOC541471 /// non-protein coding RNA 152 | 1.18E-23 | 230.5 | 444.3 | 172.0 |
| 52 | 202957_PM_at | 3059 | HCLS1 | hematopoietic cell-specific Lyn substrate 1 | 1.94E-25 | 119.2 | 299.5 | 82.2 |

(continued)

| # | Probeset ID | Entrez Gene | Gene Symbol | Gene Title | p-value (Final Phenotype) | ADNR - Mean | AR - Mean | TX - Mean |
|---|---|---|---|---|---|---|---|---|
| 53 | 229383_PM_at | 55016 | 1-Mar | membrane-associated ring finger (C3HC4) 1 | 8.09E-25 | 33.8 | 88.0 | 22.9 |
| 54 | 33304_PM_at | 3669 | ISG20 | interferon stimulated exonuclease gene 20kDa | 2.83E-27 | 33.2 | 101.3 | 22.1 |
| 55 | 222062_PM_at | 9466 | IL27RA | interleukin 27 receptor, alpha | 1.79E-22 | 34.9 | 65.8 | 26.4 |
| 56 | 219574_PM_at | 55016 | 1-Mar | membrane-associated ring finger (C3HC4) 1 | 6.65E-25 | 51.5 | 126.0 | 36.2 |
| 57 | 202748_PM_at | 2634 | GBP2 | guanylate binding protein 2, interferon-inducible | 7.51E-26 | 196.7 | 473.0 | 141.0 |
| 58 | 210895_PM_s_at | 942 | CD86 | CD86 molecule | 3.80E-23 | 32.3 | 85.0 | 21.6 |
| 59 | 202208_PM_s_at | 10123 | ARL4C | ADP-ribosylation factor-like 4C | 1.24E-23 | 54.0 | 99.6 | 42.2 |
| 60 | 221666_PM_s_at | 29108 | PYCARD | PYD and CARD domain containing | 5.55E-24 | 60.7 | 132.8 | 44.7 |
| 61 | 227125_PM_at | 3455 | IFNAR2 | interferon (alpha, beta and omega) receptor 2 | 3.57E-24 | 70.0 | 126.2 | 55.8 |
| 62 | 226525_PM_at | 9262 | STK17B | serine/threonine kinase 17b | 3.43E-24 | 146.8 | 338.7 | 107.6 |
| 63 | 210644_PM_s_at | 3903 | LAIR1 | leukocyte-associated immunoglobulin-like receptor 1 | 2.96E-24 | 29.7 | 74.6 | 21.2 |
| 64 | 230391_PM_at | 8832 | CD84 | CD84 molecule | 1.89E-22 | 47.9 | 130.1 | 32.5 |
| 65 | 242907_PM_at | --- | --- | --- | 2.54E-22 | 59.3 | 165.1 | 39.8 |
| 66 | 1553906_PM_s_at | 221472 | FGD2 | FYVE, RhoGEF and PH domain containing 2 | 1.67E-26 | 104.6 | 321.0 | 71.9 |
| 67 | 223922_PM_x_at | 64231 | MS4A6A | membrane-spanning 4-domains, subfamily A, member 6A | 8.68E-24 | 289.2 | 656.8 | 214.5 |
| 68 | 230550_PM_at | 64231 | MS4A6A | membrane-spanning 4-domains, subfamily A, member 6A | 7.63E-24 | 44.8 | 124.3 | 30.9 |
| 69 | 202953_PM_at | 713 | C1QB | complement component 1, q subcomponent, B chain | 2.79E-22 | 215.8 | 638.4 | 142.5 |
| 70 | 213733_PM_at | 4542 | MYO1F | myosin IF | 2.25E-23 | 26.8 | 52.7 | 20.9 |
| 71 | 204774_PM_at | 2123 | EVI2A | ecotropic viral integration site 2A | 5.10E-24 | 63.6 | 168.5 | 44.9 |

| # | Probeset ID | Entrez Gene | Gene Symbol | Gene Title | p-value (Final Phenotype) | ADNR - Mean | AR - Mean | TX - Mean |
|---|---|---|---|---|---|---|---|---|
| 72 | 211366_PM_x_at | 834 | CASP1 | caspase 1, apoptosis-related cysteine peptidase (interleukin 1, beta, convertase) | 3.40E-24 | 115.3 | 261.0 | 87.1 |
| 73 | 204698_PM_at | 3669 | ISG20 | interferon stimulated exonuclease gene 20kDa | 1.27E-28 | 41.5 | 165.1 | 27.6 |
| 74 | 201762_PM_s_at | 5721 | PSME2 | proteasome (prosome, macropain) activator subunit 2 (PA28 beta) | 2.31E-22 | 1251.6 | 1825.1 | 1091.0 |
| 75 | 204470_PM_at | 2919 | CXCL1 | chemokine (C-X-C motif) ligand 1 (melanoma growth stimulating activity, alpha) | 2.81E-24 | 22.9 | 63.7 | 16.2 |
| 76 | 242827_PM_x_at | --- | --- | --- | 9.00E-23 | 22.1 | 52.3 | 16.3 |
| 77 | 209970_PM_x_at | 834 | CASP1 | caspase 1, apoptosis-related cysteine peptidase (interleukin 1, beta, convertase) | 4.40E-25 | 116.0 | 266.6 | 88.7 |
| 78 | 228532_PM_at | 128346 | C1orf162 | chromosome 1 open reading frame 162 | 1.57E-22 | 82.6 | 217.7 | 60.0 |
| 79 | 232617_PM_at | 1520 | CTSS | cathepsin S | 2.83E-23 | 209.6 | 537.9 | 154.8 |
| 80 | 203761_PM_at | 6503 | SLA | Src-like-adaptor | 3.80E-23 | 69.6 | 179.2 | 51.4 |
| 81 | 219666_PM_at | 64231 | MS4A6A | membrane-spanning 4-domains, subfamily A, member 6A | 4.29E-23 | 159.2 | 397.6 | 118.7 |
| 82 | 223280_PM_x_at | 64231 | MS4A6A | membrane-spanning 4-domains, subfamily A, member 6A | 2.53E-24 | 269.0 | 711.8 | 199.5 |
| 83 | 225701_PM_at | 80709 | AKNA | AT-hook transcription factor | 2.87E-29 | 37.7 | 102.8 | 29.0 |
| 84 | 224356_PM_x_at | 64231 | MS4A6A | membrane-spanning 4-domains, subfamily A, member 6A | 1.56E-23 | 150.6 | 399.6 | 111.2 |
| 85 | 202643_PM_s_at | 7128 | TNFAIP3 | tumor necrosis factor, alpha-induced protein 3 | 9.92E-24 | 43.7 | 92.8 | 34.8 |
| 86 | 202644_PM_s_at | 7128 | TNFAIP3 | tumor necrosis factor, alpha-induced protein 3 | 2.12E-27 | 169.8 | 380.4 | 136.6 |
| 87 | 213566_PM_at | 6039 | RNASE6 | ribonuclease, RNase A family, k6 | 1.55E-23 | 180.9 | 482.0 | 134.3 |
| 88 | 219386_PM_s_at | 56833 | SLAMF8 | SLAM family member 8 | 1.22E-22 | 28.2 | 92.1 | 19.4 |

| # | Probeset ID | Entrez Gene | Gene Symbol | Gene Title | p-value (Final Phenotype) | ADNR - Mean | AR - Mean | TX - Mean |
|---|---|---|---|---|---|---|---|---|
| 89 | 203416_PM_at | 963 | CD53 | CD53 molecule | 3.13E-23 | 215.9 | 603.0 | 157.8 |
| 90 | 200003_PM_s_at | 6158 | RPL28 | ribosomal protein L28 | 1.73E-22 | 4375.2 | 5531.2 | 4069.9 |
| 91 | 206420_PM_at | 10261 | IGSF6 | immunoglobulin superfamily, member 6 | 5.65E-23 | 45.1 | 131.5 | 32.7 |
| 92 | 217028_PM_at | 7852 | CXCR4 | chemokine (C-X-C motif) receptor 4 | 6.84E-27 | 100.8 | 304.4 | 75.3 |
| 93 | 232024_PM_at | 26157 | GIMAP2 | GTPase, IMAP family member 2 | 2.94E-24 | 90.2 | 197.7 | 72.5 |
| 94 | 238327_PM_at | 440836 | ODF3B | outer dense fiber of sperm tails 3B | 1.75E-27 | 32.8 | 81.4 | 26.1 |
| 95 | 209083_PM_at | 11151 | CORO1A | coronin, actin binding protein, 1A | 2.78E-27 | 46.9 | 163.8 | 34.3 |
| 96 | 232724_PM_at | 64231 | MS4A6A | membrane-spanning 4-domains, subfamily A, member 6A | 6.28E-23 | 24.8 | 47.6 | 20.7 |
| 97 | 211742_PM_s_at | 2124 | EVI2B | ecotropic viral integration site 2B | 1.56E-22 | 73.9 | 236.5 | 53.2 |
| 98 | 202659_PM_at | 5699 | PSMB10 | proteasome (prosome, macropain) subunit, beta type, 10 | 2.29E-24 | 180.7 | 355.6 | 151.5 |
| 99 | 226991_PM_at | 4773 | NFATC2 | nuclear factor of activated T-cells, cytoplasmic, calcineurin-dependent 2 | 2.42E-23 | 37.9 | 87.0 | 30.3 |
| 100 | 210538_PM_s_at | 330 | BIRC3 | baculoviral IAP repeat-containing 3 | 5.40E-26 | 106.7 | 276.7 | 84.6 |
| 101 | 205269_PM_at | 3937 | LCP2 | lymphocyte cytosolic protein 2 (SH2 domain containing leukocyte protein of 76kDa) | 7.35E-25 | 30.2 | 92.2 | 22.9 |
| 102 | 211368_PM_s_at | 834 | CASP1 | caspase 1, apoptosis-related cysteine peptidase (interleukin 1, beta, convertase) | 9.43E-27 | 102.6 | 274.3 | 81.8 |
| 103 | 205798_PM_at | 3575 | IL7R | interleukin 7 receptor | 1.57E-24 | 44.1 | 136.8 | 33.4 |
| 104 | 228442_PM_at | 4773 | NFATC2 | nuclear factor of activated T-cells, cytoplasmic, calcineurin-dependent 2 | 5.58E-23 | 39.0 | 84.8 | 32.0 |
| 105 | 213603_PM_s_at | 5880 | RAC2 | ras-related C3 botulinum toxin substrate 2 (rho family, small GTP binding protein Rac2) | 3.38E-25 | 113.9 | 366.5 | 86.5 |

| # | Probeset ID | Entrez Gene | Gene Symbol | Gene Title | p-value (Final Phenotype) | ADNR - Mean | AR - Mean | TX - Mean |
|---|---|---|---|---|---|---|---|---|
| 106 | 228964_PM_at | 639 | PRDM1 | PR domain containing 1, with ZNF domain | 1.42E-23 | 21.2 | 52.1 | 17.0 |
| 107 | 209606_PM_at | 9595 | CYTIP | cytohesin 1 interacting protein | 1.66E-26 | 41.4 | 114.3 | 32.9 |
| 108 | 214022_PM_s_at | 8519 | IFITM1 | interferon induced transmembrane protein 1 (9-27) | 1.61E-23 | 799.1 | 1514.7 | 683.3 |
| 109 | 202307_PM_s_at | 6890 | TAP1 | transporter 1, ATP-binding cassette, sub-family B (MDR/TAP) | 8.61E-26 | 172.4 | 420.6 | 141.0 |
| 110 | 204882_PM_at | 9938 | ARHGAP25 | Rho GTPase activating protein 25 | 8.14E-23 | 51.5 | 107.3 | 43.0 |
| 111 | 227344_PM_at | 10320 | IKZF1 | IKAROS family zinc finger 1 (Ikaros) | 5.30E-26 | 17.8 | 40.0 | 14.9 |
| 112 | 205270_PM_s_at | 3937 | LCP2 | lymphocyte cytosolic protein 2 (SH2 domain containing leukocyte protein of 76kDa) | 3.75E-24 | 56.8 | 162.6 | 44.6 |
| 113 | 223640_PM_at | 10870 | HCST | hematopoietic cell signal transducer | 5.55E-23 | 91.0 | 234.0 | 72.7 |
| 114 | 226218_PM_at | 3575 | IL7R | interleukin 7 receptor | 2.15E-23 | 55.6 | 197.0 | 41.4 |
| 115 | 226219_PM_at | 257106 | ARHGAP30 | Rho GTPase activating protein 30 | 1.87E-26 | 46.4 | 127.9 | 37.5 |
| 116 | 38149_PM_at | 9938 | ARHGAP25 | Rho GTPase activating protein 25 | 1.20E-23 | 51.3 | 108.9 | 43.2 |
| 117 | 213975_PM_s_at | 4069 | LYZ | lysozyme | 2.70E-22 | 458.4 | 1626.0 | 338.1 |
| 118 | 238668_PM_at | --- | --- | --- | 1.35E-23 | 18.2 | 49.2 | 14.5 |
| 119 | 200887_PM_s_at | 6772 | STAT1 | signal transducer and activator of transcription 1, 91kDa | 1.27E-22 | 1141.7 | 2278.6 | 972.9 |
| 120 | 1555756_PM_a_at | 64581 | CLEC7A | C-type lectin domain family 7, member A | 2.11E-22 | 16.6 | 45.4 | 13.2 |
| 121 | 205039_PM_s_at | 10320 | IKZF1 | IKAROS family zinc finger 1 (Ikaros) | 6.73E-23 | 29.1 | 65.9 | 24.2 |
| 122 | 206011_PM_at | 834 | CASP1 | caspase 1, apoptosis-related cysteine peptidase (interleukin 1, beta, convertase) | 6.34E-25 | 74.5 | 198.0 | 60.7 |
| 123 | 221698_PM_s_at | 64581 | CLEC7A | C-type lectin domain family 7, member A | 9.79E-24 | 61.5 | 164.6 | 49.7 |

| # | Probeset ID | Entrez Gene | Gene Symbol | Gene Title | p-value (Final Phenotype) | ADNR - Mean | AR - Mean | TX - Mean |
|---|---|---|---|---|---|---|---|---|
| 124 | 227346_PM_at | 10320 | IKZF1 | IKAROS family zinc finger 1 (Ikaros) | 1.51E-26 | 24.9 | 79.7 | 19.8 |
| 125 | 230499_PM_at | --- | --- | --- | 8.55E-23 | 29.7 | 68.7 | 24.7 |
| 126 | 229391_PM_s_at | 441168 | FAM26F | family with sequence similarity 26, member F | 2.74E-23 | 98.6 | 379.7 | 73.7 |
| 127 | 205159_PM_at | 1439 | CSF2RB | colony stimulating factor 2 receptor, beta, low-affinity (granulocyte-macrophage) | 1.74E-23 | 33.7 | 107.5 | 26.3 |
| 128 | 205639_PM_at | 313 | AOAH | acyloxyacyl hydrolase (neutrophil) | 4.43E-23 | 30.3 | 72.6 | 25.2 |
| 129 | 204563_PM_at | 6402 | SELL | selectin L | 1.00E-23 | 40.7 | 134.7 | 31.7 |
| 130 | 201288_PM_at | 397 | ARHGDIB | Rho GDP dissociation inhibitor (GDI) beta | 1.92E-22 | 354.9 | 686.8 | 308.1 |
| 131 | 209969_PM_s_at | 6772 | STAT1 | signal transducer and activator of transcription 1, 91kDa | 5.49E-24 | 395.8 | 1114.5 | 320.8 |
| 132 | 229390_PM_at | 441168 | FAM26F | family with sequence similarity 26, member F | 3.91E-25 | 103.8 | 520.0 | 75.9 |
| 133 | 242916_PM_at | 11064 | CEP110 | centrosomal protein 110kDa | 1.99E-23 | 30.8 | 68.1 | 26.2 |
| 134 | 207651_PM_at | 29909 | GPR171 | G protein-coupled receptor 171 | 1.90E-29 | 25.8 | 89.9 | 20.9 |
| 135 | 229937_PM_x_at | 10859 | LILRB1 | Leukocyte immunoglobulin-like receptor, subfamily B (with TM and ITIM domains), member | 1.67E-24 | 23.5 | 79.9 | 18.5 |
| 136 | 232543_PM_x_at | 64333 | ARHGAP9 | Rho GTPase activating protein 9 | 3.66E-26 | 31.7 | 99.1 | 25.8 |
| 137 | 203332_PM_s_at | 3635 | INPP5D | inositol polyphosphate-5-phosphatase, 145kDa | 3.66E-24 | 27.9 | 60.5 | 24.0 |
| 138 | 213160_PM_at | 1794 | DOCK2 | dedicator of cytokinesis 2 | 1.03E-24 | 33.7 | 92.6 | 27.8 |
| 139 | 204912_PM_at | 3587 | IL10RA | interleukin 10 receptor, alpha | 1.28E-24 | 57.0 | 178.7 | 46.1 |
| 140 | 204205_PM_at | 60489 | APOBEC3G | apolipoprotein B mRNA editing enzyme, catalytic polypeptide-like 3G | 6.40E-27 | 95.4 | 289.4 | 78.7 |
| 141 | 206513_PM_at | 9447 | AIM2 | absent in melanoma 2 | 9.67E-23 | 17.2 | 50.7 | 13.9 |
| 142 | 203741_PM_s_at | 113 | ADCY7 | adenylate cyclase 7 | 2.34E-22 | 23.6 | 59.3 | 19.7 |

(continued)

| # | Probeset ID | Entrez Gene | Gene Symbol | Gene Title | p-value (Final Phenotype) | ADNR - Mean | AR - Mean | TX - Mean |
|---|---|---|---|---|---|---|---|---|
| 143 | 206118_PM_at | 6775 | STAT4 | signal transducer and activator of transcription 4 | 8.28E-26 | 21.0 | 49.5 | 18.1 |
| 144 | 227677_PM_at | 3718 | JAK3 | Janus kinase 3 | 6.48E-24 | 18.6 | 51.7 | 15.5 |
| 145 | 227353_PM_at | 147138 | TMC8 | transmembrane channel-like 8 | 6.49E-29 | 19.8 | 64.2 | 16.6 |
| 146 | 1552701_PM_a_at | 114769 | CARD16 | caspase recruitment domain family, member 16 | 2.52E-23 | 143.8 | 413.6 | 119.8 |
| 147 | 1552703_PM_s_at | 114769 /// 834 | CARD16 /// CASP1 | caspase recruitment domain family, member 16 /// caspase 1, apoptosis-related cysteine | 7.57E-24 | 64.6 | 167.8 | 54.9 |
| 148 | 229437_PM_at | 114614 | MIR155HG | MIR155 host gene (non-protein coding) | 3.90E-27 | 15.4 | 50.4 | 12.9 |
| 149 | 204319_PM_s_at | 6001 | RGS10 | regulator of G-protein signaling 10 | 6.54E-24 | 159.7 | 360.4 | 139.4 |
| 150 | 204118_PM_at | 962 | CD48 | CD48 molecule | 3.85E-23 | 82.9 | 341.5 | 65.2 |
| 151 | 1559584_PM_a_at | 283897 | C16orf54 | chromosome 16 open reading frame 54 | 2.86E-24 | 31.3 | 95.8 | 26.1 |
| 152 | 212588_PM_at | 5788 | PTPRC | protein tyrosine phosphatase, receptor type, C | 2.46E-22 | 94.4 | 321.0 | 76.4 |
| 153 | 219014_PM_at | 51316 | PLAC8 | placenta-specific 8 | 2.03E-23 | 38.8 | 164.1 | 30.7 |
| 154 | 235735_PM_at | --- | --- | --- | 1.39E-25 | 13.0 | 34.9 | 11.2 |
| 155 | 203932_PM_at | 3109 | HLA-DMB | major histocompatibility complex, class II, DM beta | 6.25E-23 | 422.4 | 853.9 | 376.0 |
| 156 | 223502_PM_s_at | 10673 | TNFSF13B | tumor necrosis factor (ligand) superfamily, member 13b | 2.62E-23 | 73.0 | 244.3 | 60.0 |
| 157 | 1405_PM_i_at | 6352 | CCL5 | chemokine (C-C motif) ligand 5 | 2.22E-25 | 68.0 | 295.7 | 54.6 |
| 158 | 226474_PM_at | 84166 | NLRC5 | NLR family, CARD domain containing 5 | 1.33E-23 | 64.4 | 173.5 | 55.1 |
| 159 | 204220_PM_at | 9535 | GMFG | glia maturation factor, gamma | 1.56E-23 | 147.0 | 339.3 | 128.9 |
| 160 | 204923_PM_at | 54440 | SASH3 | SAM and SH3 domain containing 3 | 4.56E-23 | 25.4 | 68.2 | 21.7 |
| 161 | 206082_PM_at | 10866 | HCP5 | HLA complex P5 | 1.23E-23 | 76.2 | 185.1 | 66.6 |
| 162 | 204670_PM_x_at | 3123 /// 3126 | HLA-DRB1 /// HLA-DRB4 | major histocompatibility complex, class II, DR beta 1 /// major histocompatibility comp | 1.31E-23 | 2461.9 | 4344.6 | 2262.0 |

(continued)

| # | Probeset ID | Entrez Gene | Gene Symbol | Gene Title | p-value (Final Phenotype) | ADNR - Mean | AR - Mean | TX - Mean |
|---|---|---|---|---|---|---|---|---|
| 163 | 228869_PM_at | 124460 | SNX20 | sorting nexin 20 | 2.59E-22 | 25.7 | 67.3 | 22.2 |
| 164 | 205831_PM_at | 914 | CD2 | CD2 molecule | 2.30E-27 | 40.4 | 162.5 | 33.9 |
| 165 | 206978_PM_at | 729230 | CCR2 | chemokine (C-C motif) receptor 2 | 4.46E-23 | 32.1 | 100.7 | 27.3 |
| 166 | 224451_PM_x_at | 64333 | ARHGAP9 | Rho GTPase activating protein 9 | 4.23E-24 | 34.2 | 103.4 | 29.4 |
| 167 | 204279_PM_at | 5698 | PSMB9 | proteasome (prosome, macropain) subunit, beta type, 9 (large multifunctional peptidase) | 2.81E-23 | 241.6 | 637.4 | 211.3 |
| 168 | 209795_PM_at | 969 | CD69 | CD69 molecule | 5.81E-27 | 17.6 | 57.6 | 15.2 |
| 169 | 229625_PM_at | 115362 | GBP5 | guanylate binding protein 5 | 5.85E-24 | 29.3 | 133.3 | 24.0 |
| 170 | 213416_PM_at | 3676 | ITGA4 | integrin, alpha 4 (antigen CD49D, alpha 4 subunit of VLA-4 receptor) | 1.19E-23 | 26.3 | 78.1 | 22.8 |
| 171 | 206804_PM_at | 917 | CD3G | CD3g molecule, gamma (CD3-TCR complex) | 1.50E-27 | 19.7 | 60.6 | 17.3 |
| 172 | 222895_PM_s_at | 64919 | BCL11B | B-cell CLL/lymphoma 11B (zinc finger protein) | 7.01E-23 | 22.0 | 64.5 | 19.1 |
| 173 | 211582_PM_x_at | 7940 | LST1 | leukocyte specific transcript 1 | 2.13E-22 | 57.5 | 183.8 | 49.4 |
| 174 | 1555852_PM_at | 100507463 | LOC100507746 3 | hypothetical LOC100507463 | 8.92E-26 | 78.9 | 202.6 | 70.9 |
| 175 | 213539_PM_at | 915 | CD3D | CD3d molecule, delta (CD3-TCR complex) | 9.01E-27 | 70.8 | 335.1 | 60.0 |
| 176 | 239237_PM_at | --- | --- | --- | 9.82E-24 | 15.9 | 34.9 | 14.5 |
| 177 | 229723_PM_at | 117289 | TAGAP | T-cell activation RhoGTPase activating protein | 5.21E-24 | 29.2 | 82.9 | 26.2 |
| 178 | 204655_PM_at | 6352 | CCL5 | chemokine (C-C motif) ligand 5 | 7.63E-24 | 77.5 | 339.4 | 66.8 |
| 179 | 229041_PM_s_at | --- | --- | --- | 1.64E-24 | 36.5 | 132.1 | 32.4 |
| 180 | 205267_PM_at | 5450 | POU2AF1 | POU class 2 associating factor 1 | 4.47E-23 | 17.9 | 86.8 | 15.7 |
| 181 | 226878_PM_at | 3111 | HLA-DOA | major histocompatibility complex, class II, DO alpha | 2.59E-25 | 102.0 | 288.9 | 94.3 |

| # | Probeset ID | Entrez Gene | Gene Symbol | Gene Title | p-value (Final Phenotype) | ADNR - Mean | AR - Mean | TX - Mean |
|---|---|---|---|---|---|---|---|---|
| 182 | 205488_PM_at | 3001 | GZMA | granzyme A (granzyme 1, cytotoxic T-lymphocyte-associated serine esterase 3) | 4.14E-25 | 37.3 | 164.8 | 33.4 |
| 183 | 201137_PM_s_at | 3115 | HLA-DPB1 | major histocompatibility complex, class II, DP beta 1 | 2.17E-22 | 1579.1 | 3863.7 | 1475.9 |
| 184 | 204891_PM_s_at | 3932 | LCK | lymphocyte-specific protein tyrosine kinase | 7.95E-28 | 19.3 | 74.2 | 17.8 |
| 185 | 231776_PM_at | 8320 | EOMES | eomesodermin | 1.71E-23 | 24.0 | 63.9 | 22.4 |
| 186 | 211339_PM_s_at | 3702 | ITK | IL2-inducible T-cell kinase | 7.40E-23 | 16.7 | 44.4 | 15.7 |
| 187 | 223322_PM_at | 83593 | RASSF5 | Ras association (RalGDS/AF-6) domain family member 5 | 5.21E-26 | 41.5 | 114.4 | 39.2 |
| 188 | 205758_PM_at | 925 | CD8A | CD8a molecule | 2.80E-25 | 24.2 | 105.8 | 22.2 |
| 189 | 231124_PM_x_at | 4063 | LY9 | lymphocyte antigen 9 | 2.81E-23 | 16.7 | 45.8 | 15.7 |
| 190 | 211796_PM_s_at | 28638 /// 28639 | TRBC1 /// TRBC2 | T cell receptor beta constant 1 /// T cell receptor beta constant 2 | 4.38E-25 | 69.2 | 431.5 | 63.6 |
| 191 | 210915_PM_x_at | 28638 | TRBC2 | T cell receptor beta constant 2 | 1.91E-27 | 39.7 | 230.7 | 37.5 |
| 192 | 205821_PM_at | 22914 | KLRK1 | killer cell lectin-like receptor subfamily K, member 1 | 1.18E-25 | 30.3 | 111.5 | 31.3 |
| 193 | 236295_PM_s_at | 197358 | NLRC3 | NLR family, CARD domain containing 3 | 6.22E-26 | 19.0 | 52.5 | 18.6 |
| 194 | 213193_PM_x_at | 28639 | TRBC1 | T cell receptor beta constant 1 | 4.22E-25 | 95.3 | 490.9 | 92.1 |
| 195 | 211656_PM_x_at | 100133583 /// 3119 | HLA-DQB1 /// LOC10013358 3 | major histocompatibility complex, class II, DQ beta 1 /// HLA class II histocompatibili | 5.98E-25 | 211.3 | 630.2 | 208.2 |
| 196 | 209670_PM_at | 28755 | TRAC | T cell receptor alpha constant | 1.44E-24 | 37.9 | 149.9 | 38.5 |

| # | Probeset ID | Entrez Gene | Gene Symbol | Gene Title | p-value (Final Phenotype) | ADNR - Mean | AR - Mean | TX - Mean |
|---|---|---|---|---|---|---|---|---|
| 197 | 213888_PM_s_at | 80342 | TRAF3IP3 | TRAF3 interacting protein 3 | 1.66E-22 | 30.8 | 101.9 | 30.5 |

Table 9. Biopsy Expression Profiling of Kidney Transplants: 2-Way Classifier CAN vs. TX

| # | Probeset ID | Entrez Gene | Gene Symbol | Gene Title | p-value (Final Phenotype) | CAN - Mean | TX- Mean |
|---|---|---|---|---|---|---|---|
| 1 | 204698_PM_at | 3669 | ISG20 | interferon stimulated exonuclease gene 20kDa | 1.93E-19 | 96.1 | 27.6 |
| 2 | 33304_PM_at | 3669 | ISG20 | interferon stimulated exonuclease gene 20kDa | 2.02E-19 | 63.4 | 22.1 |
| 3 | 217022_PM_s_at | 100126583 /// 3493 /// 3494 | IGHA1 /// IGHA2 /// LOC10012658 3 | immunoglobulin heavy constant alpha 1 /// immunoglobulin heavy constant alpha 2 (A2m ma | 4.31E-19 | 494.6 | 49.4 |
| 4 | 202957_PM_at | 3059 | HCLS1 | hematopoietic cell-specific Lyn substrate 1 | 5.13E-19 | 229.9 | 82.2 |
| 5 | 203761_PM_at | 6503 | SLA | Src-like-adaptor | 1.10E-18 | 138.4 | 51.4 |
| 6 | 204446_PM_s_at | 240 | ALOX5 | arachidonate 5-lipoxygenase | 1.36E-18 | 216.7 | 54.7 |
| 7 | 209198_PM_s_at | 23208 | SYT11 | synaptotagmin XI | 1.93E-18 | 41.8 | 22.8 |
| 8 | 228964_PM_at | 639 | PRDM1 | PR domain containing 1, with ZNF domain | 2.37E-18 | 41.5 | 17.0 |
| 9 | 201042_PM_at | 7052 | TGM2 | transglutaminase 2 (C polypeptide, protein-glutamine-gamma-glutamyltransferase) | 3.13E-18 | 172.6 | 80.1 |
| 10 | 226219_PM_at | 257106 | ARHGAP30 | Rho GTPase activating protein 30 | 7.21E-18 | 91.8 | 37.5 |
| 11 | 225701_PM_at | 80709 | AKNA | AT-hook transcription factor | 7.27E-18 | 73.2 | 29.0 |
| 12 | 202207_PM_at | 10123 | ARL4C | ADP-ribosylation factor-like 4C | 7.98E-18 | 190.4 | 57.2 |
| 13 | 219574_PM_at | 55016 | MAR1 | membrane-associated ring finger (C3HC4) 1 | 8.98E-18 | 87.5 | 36.2 |
| 14 | 209083_PM_at | 12-Jul | CORO1A | coronin, actin binding protein, 1A | 1.06E-17 | 107.1 | 34.3 |
| 15 | 226621_PM_at | 9180 | OSMR | oncostatin M receptor | 1.85E-17 | 682.9 | 312.1 |
| 16 | 1405_PM_i_at | 6352 | CCL5 | chemokine (C-C motif) ligand 5 | 2.19E-17 | 195.6 | 54.6 |
| 17 | 213160_PM_at | 1794 | DOCK2 | dedicator of cytokinesis 2 | 2.75E-17 | 66.0 | 27.8 |
| 18 | 227346_PM_at | 10320 | IKZF1 | IKAROS family zinc finger 1 (Ikaros) | 2.92E-17 | 53.3 | 19.8 |
| 19 | 204205_PM_at | 60489 | APOBEC3G | apolipoprotein B mRNA editing enzyme, catalytic polypeptide-like 3G | 2.92E-17 | 192.0 | 78.7 |
| 20 | 218322_PM_s_at | 51703 | ACSL5 | acyl-CoA synthetase long-chain family member 5 | 3.15E-17 | 84.5 | 48.1 |

EP 3 825 417 A2

| # | Probeset ID | Entrez Gene | Gene Symbol | Gene Title | p-value (Final Phenotype) | CAN - Mean | TX- Mean |
|---|---|---|---|---|---|---|---|
| 21 | 238327_PM_at | 440836 | ODF3B | outer dense fiber of sperm tails 3B | 3.42E-17 | 58.5 | 26.1 |
| 22 | 218983_PM_at | 51279 | C1RL | complement component 1, r subcomponent-like | 4.33E-17 | 206.9 | 99.4 |
| 23 | 210538_PM_s_at | 330 | BIRC3 | baculoviral IAP repeat-containing 3 | 4.49E-17 | 199.1 | 84.6 |
| 24 | 207651_PM_at | 29909 | GPR171 | G protein-coupled receptor 171 | 5.48E-17 | 57.0 | 20.9 |
| 25 | 201601_PM_x_at | 8519 | IFITM1 | interferon induced transmembrane protein 1 (9-27) | 6.07E-17 | 2202.5 | 1251.1 |
| 26 | 226878_PM_at | 3111 | HLA-DOA | major histocompatibility complex, class II, DO alpha | 6.12E-17 | 201.4 | 94.3 |
| 27 | 1555756_PM_a_at | 64581 | CLEC7A | C-type lectin domain family 7, member A | 6.22E-17 | 28.9 | 13.2 |
| 28 | 1559584_PM_a_at | 283897 | C16orf54 | chromosome 16 open reading frame 54 | 6.73E-17 | 71.5 | 26.1 |
| 29 | 209795_PM_at | 969 | CD69 | CD69 molecule | 9.46E-17 | 40.6 | 15.2 |
| 30 | 230550_PM_at | 64231 | MS4A6A | membrane-spanning 4-domains, subfamily A, member 6A | 1.20E-16 | 88.0 | 30.9 |
| 31 | 1553906_PM_s_at | 221472 | FGD2 | FYVE, RhoGEF and PH domain containing 2 | 1.34E-16 | 219.3 | 71.9 |
| 32 | 205798_PM_at | 3575 | IL7R | interleukin 7 receptor | 1.55E-16 | 106.3 | 33.4 |
| 33 | 1555852_PM_at | 100507463 | LOC10050746 3 | hypothetical LOC100507463 | 1.81E-16 | 154.2 | 70.9 |
| 34 | 224916_PM_at | 340061 | TMEM173 | transmembrane protein 173 | 1.84E-16 | 67.8 | 40.0 |
| 35 | 211368_PM_s_at | 834 | CASP1 | caspase 1, apoptosis-related cysteine peptidase (interleukin 1, beta, convertase) | 1.85E-16 | 191.4 | 81.8 |
| 36 | 226474_PM_at | 84166 | NLRC5 | NLR family, CARD domain containing 5 | 1.85E-16 | 129.8 | 55.1 |
| 37 | 201137_PM_s_at | 3115 | HLA-DPB1 | major histocompatibility complex, class II, DP beta 1 | 1.90E-16 | 3151.7 | 1475.9 |

(continued)

| # | Probeset ID | Entrez Gene | Gene Symbol | Gene Title | p-value (Final Phenotype) | CAN - Mean | TX- Mean |
|---|---|---|---|---|---|---|---|
| 38 | 210785_PM_s_at | 9473 | C1orf38 | chromosome 1 open reading frame 38 | 2.07E-16 | 39.9 | 16.4 |
| 39 | 215121_PM_x_at | 100290481 /// 28823 /// 28834 | IGLC7 /// IGLV1-44 /// LOC10029048 1 | immunoglobulin lambda constant 7 /// immunoglobulin lambda variable 1-44 /// immunoglob | 2.13E-16 | 1546.8 | 250.4 |
| 40 | 1555832_PM_s_at | 1316 | KLF6 | Kruppel-like factor 6 | 2.35E-16 | 1003.3 | 575.1 |
| 41 | 221932_PM_s_at | 51218 | GLRX5 | glutaredoxin 5 | 2.49E-16 | 1218.1 | 1599.3 |
| 42 | 207677_PM_s_at | 4689 | NCF4 | neutrophil cytosolic factor 4, 40kDa | 2.65E-16 | 39.5 | 19.2 |
| 43 | 202720_PM_at | 26136 | TES | testis derived transcript (3 LIM domains) | 2.68E-16 | 357.9 | 204.2 |
| 44 | 220005_PM_at | 53829 | P2RY13 | purinergic receptor P2Y, G-protein coupled, 13 | 2.72E-16 | 29.6 | 14.8 |
| 45 | 200904_PM_at | 3133 | HLA-E | major histocompatibility complex, class I, E | 2.73E-16 | 1607.7 | 994.7 |
| 46 | 222294_PM_s_at | 5873 | RAB27A | RAB27A, member RAS oncogene family | 2.91E-16 | 263.0 | 146.4 |
| 47 | 205831_PM_at | 914 | CD2 | CD2 molecule | 3.32E-16 | 100.9 | 33.9 |
| 48 | 227344_PM_at | 10320 | IKZF1 | IKAROS family zinc finger 1 (Ikaros) | 3.39E-16 | 28.5 | 14.9 |
| 49 | 209374_PM_s_at | 3507 | IGHM | immunoglobulin heavy constant mu | 3.73E-16 | 301.0 | 45.0 |
| 50 | 202307_PM_s_at | 6890 | TAP1 | transporter 1, ATP-binding cassette, sub-family B (MDR/TAP) | 4.84E-16 | 280.0 | 141.0 |
| 51 | 223218_PM_s_at | 64332 | NFKBIZ | nuclear factor of kappa light polypeptide gene enhancer in B-cells inhibitor, zeta | 5.05E-16 | 399.9 | 159.1 |
| 52 | 229437_PM_at | 114614 | MIR155HG | MIR155 host gene (non-protein coding) | 5.85E-16 | 28.5 | 12.9 |
| 53 | 213603_PM_s_at | 5880 | RAC2 | ras-related C3 botulinum toxin substrate 2 (rho family, small GTP binding protein Rac2) | 5.98E-16 | 250.3 | 86.5 |

| # | Probeset ID | Entrez Gene | Gene Symbol | Gene Title | p-value (Final Phenotype) | CAN - Mean | TX- Mean |
|---|---|---|---|---|---|---|---|
| 54 | 214669_PM_x_at | 3514 /// 50802 | IGK@ /// IGKC | immunoglobulin kappa locus /// immunoglobulin kappa constant | 6.32E-16 | 3449.1 | 587.1 |
| 55 | 211430_PM_s_at | 28396 /// 3500 /// 3507 | IGHG1 /// IGHM /// IGHV4-31 | immunoglobulin heavy constant gamma 1 (G1m marker) /// immunoglobulin heavy constant mu | 6.39E-16 | 2177.7 | 266.9 |
| 56 | 228471_PM_at | 91526 | ANKRD44 | ankyrin repeat domain 44 | 6.42E-16 | 184.6 | 86.5 |
| 57 | 209138_PM_x_at | 3535 | IGL@ | Immunoglobulin lambda locus | 7.54E-16 | 2387.0 | 343.2 |
| 58 | 227353_PM_at | 147138 | TMC8 | transmembrane channel-like 8 | 8.01E-16 | 42.7 | 16.6 |
| 59 | 200986_PM_at | 710 | SERPING1 | serpin peptidase inhibitor, clade G (C1 inhibitor), member 1 | 8.10E-16 | 590.2 | 305.3 |
| 60 | 212203_PM_x_at | 10410 | IFITM3 | interferon induced transmembrane protein 3 (1-8U) | 8.17E-16 | 4050.1 | 2773.8 |
| 61 | 221651_PM_x_at | 3514 /// 50802 | IGK@ /// IGKC | immunoglobulin kappa locus /// immunoglobulin kappa constant | 9.60E-16 | 3750.2 | 621.2 |
| 62 | 214836_PM_x_at | 28299 /// 3514 /// 50802 | IGK@ /// IGKC /// IGKV1-5 | immunoglobulin kappa locus /// immunoglobulin kappa constant /// immunoglobulin kappa v | 9.72E-16 | 544.2 | 109.1 |
| 63 | 1552703_PM_s_at | 114769 /// 834 | CARD16 /// CASP1 | caspase recruitment domain family, member 16 /// caspase 1, apoptosis-related cysteine | 1.12E-15 | 120.6 | 54.9 |
| 64 | 202901_PM_x_at | 1520 | CTSS | cathepsin S | 1.13E-15 | 130.9 | 45.1 |
| 65 | 215379_PM_x_at | 28823 /// 28834 | IGLC7 /// IGLV1-44 | immunoglobulin lambda constant 7 /// immunoglobulin lambda variable 1-44 | 1.16E-15 | 1453.0 | 248.1 |
| 66 | 222939_PM_s_at | 117247 | SLC16A10 | solute carrier family 16, member 10 (aromatic amino acid transporter) | 1.22E-15 | 115.1 | 229.6 |
| 67 | 232617_PM_at | 1520 | CTSS | cathepsin S | 1.22E-15 | 392.2 | 154.8 |
| 68 | 235964_PM_x_at | 25939 | SAMHD1 | SAM domain and HD domain 1 | 1.26E-15 | 270.1 | 117.5 |

| # | Probeset ID | Entrez Gene | Gene Symbol | Gene Title | p-value (Final Phenotype) | CAN - Mean | TX- Mean |
|---|---|---|---|---|---|---|---|
| 69 | 205159_PM_at | 1439 | CSF2RB | colony stimulating factor 2 receptor, beta, low-affinity (granulocyte-macrophage) | 1.28E-15 | 70.4 | 26.3 |
| 70 | 224451_PM_x_at | 64333 | ARHGAP9 | Rho GTPase activating protein 9 | 1.34E-15 | 71.8 | 29.4 |
| 71 | 214677_PM_x_at | 100287927 /// 3535 | IGL@ /// LOC10028792 7 | Immunoglobulin lambda locus /// Hypothetical protein LOC100287927 | 1.35E-15 | 2903.1 | 433.7 |
| 72 | 217733_PM_s_at | 9168 | TMSB10 | thymosin beta 10 | 1.37E-15 | 5555.2 | 3529.0 |
| 73 | 38149_PM_at | 9938 | ARHGAP25 | Rho GTPase activating protein 25 | 1.46E-15 | 83.2 | 43.2 |
| 74 | 221671_PM_x_at | 3514 /// 50802 | IGK@ /// IGKC | immunoglobulin kappa locus /// immunoglobulin kappa constant | 1.57E-15 | 3722.5 | 642.9 |
| 75 | 214022_PM_s_at | 8519 | IFITM1 | interferon induced transmembrane protein 1 (9-27) | 1.59E-15 | 1236.6 | 683.3 |
| 76 | 223217_PM_s_at | 64332 | NFKBIZ | nuclear factor of kappa light polypeptide gene enhancer in B-cells inhibitor, zeta | 1.61E-15 | 196.6 | 79.7 |
| 77 | 206118_PM_at | 6775 | STAT4 | signal transducer and activator of transcription 4 | 1.67E-15 | 37.7 | 18.1 |
| 78 | 221666_PM_s_at | 29108 | PYCARD | PYD and CARD domain containing | 1.82E-15 | 95.5 | 44.7 |
| 79 | 207375_PM_s_at | 3601 | IL15RA | interleukin 15 receptor, alpha | 1.94E-15 | 51.2 | 28.2 |
| 80 | 209197_PM_at | 23208 | SYT11 | synaptotagmin XI | 2.02E-15 | 38.2 | 24.9 |
| 81 | 243366_PM_s_at | --- | --- | --- | 2.05E-15 | 52.5 | 22.0 |
| 82 | 224795_PM_x_at | 3514 /// 50802 | IGK@ /// IGKC | immunoglobulin kappa locus /// immunoglobulin kappa constant | 2.18E-15 | 3866.2 | 670.5 |
| 83 | 36711_PM_at | 23764 | MAFF | v-maf musculoaponeurotic fibrosarcoma oncogene homolog F (avian) | 2.26E-15 | 113.0 | 40.7 |
| 84 | 227125_PM_at | 3455 | IFNAR2 | interferon (alpha, beta and omega) receptor 2 | 2.27E-15 | 96.7 | 55.8 |

| # | Probeset ID | Entrez Gene | Gene Symbol | Gene Title | p-value (Final Phenotype) | CAN - Mean | TX- Mean |
|---|---|---|---|---|---|---|---|
| 85 | 235735_PM_at | --- | --- | --- | 2.58E-15 | 24.5 | 11.2 |
| 86 | 209515_PM_s_at | 5873 | RAB27A | RAB27A, member RAS oncogene family | 2.61E-15 | 160.5 | 85.2 |
| 87 | 204670_PM_x_at | 3123 /// 3126 | HLA-DRB1 /// HLA-DRB4 | major histocompatibility complex, class II, DR beta 1 /// major histocompatibility comp | 2.61E-15 | 3694.9 | 2262.0 |
| 88 | 205269_PM_at | 3937 | LCP2 | lymphocyte cytosolic protein 2 (SH2 domain containing | 2.85E-15 | 58.9 | 22.9 |
| | | | | leukocyte protein of 76kDa) | | | |
| 89 | 226525_PM_at | 9262 | STK17B | serine/threonine kinase 17b | 3.00E-15 | 259.1 | 107.6 |
| 90 | 229295_PM_at | 150166 /// 23765 | IL17RA /// LOC150166 | interleukin 17 receptor A /// hypothetical protein LOC150166 | 3.02E-15 | 98.4 | 50.0 |
| 91 | 206513_PM_at | 9447 | AIM2 | absent in melanoma 2 | 3.18E-15 | 30.5 | 13.9 |
| 92 | 209774_PM_x_at | 2920 | CXCL2 | chemokine (C-X-C motif) ligand 2 | 3.45E-15 | 38.2 | 15.5 |
| 93 | 211656_PM_x_at | 100133583 /// 3119 | HLA-DQB1 /// LOC10013358 3 | major histocompatibility complex, class II, DQ beta 1 /// HLA class II histocompatibili | 3.51E-15 | 459.8 | 208.2 |
| 94 | 206011_PM_at | 834 | CASP1 | caspase 1, apoptosis-related cysteine peptidase (interleukin 1, beta, convertase) | 3.56E-15 | 146.2 | 60.7 |
| 95 | 202803_PM_s_at | 3689 | ITGB2 | integrin, beta 2 (complement component 3 receptor 3 and 4 subunit) | 3.68E-15 | 182.6 | 65.2 |
| 96 | 221698_PM_s_at | 64581 | CLEC7A | C-type lectin domain family 7, member A | 3.69E-15 | 112.8 | 49.7 |
| 97 | 229937_PM_x_at | 10859 | LILRB1 | Leukocyte immunoglobulin-like receptor, subfamily B (with TM and ITIM domains), member | 3.75E-15 | 50.0 | 18.5 |
| 98 | 235529_PM_x_at | 25939 | SAMHD1 | SAM domain and HD domain 1 | 3.99E-15 | 289.1 | 128.0 |
| 99 | 223322_PM_at | 83593 | RASSF5 | Ras association (RalGDS/AF-6) domain family member 5 | 4.03E-15 | 79.3 | 39.2 |

| # | Probeset ID | Entrez Gene | Gene Symbol | Gene Title | p-value (Final Phenotype) | CAN - Mean | TX-Mean |
|---|---|---|---|---|---|---|---|
| 100 | 211980_PM_at | 1282 | COL4A1 | collagen, type IV, alpha 1 | 4.75E-15 | 1295.8 | 774.7 |
| 101 | 201721_PM_s_at | 7805 | LAPTM5 | lysosomal protein transmembrane 5 | 4.83E-15 | 661.3 | 249.4 |
| 102 | 242916_PM_at | 11064 | CEP110 | centrosomal protein 110kDa | 4.89E-15 | 51.2 | 26.2 |
| 103 | 206978_PM_at | 729230 | CCR2 | chemokine (C-C motif) receptor 2 | 5.01E-15 | 68.6 | 27.3 |
| 104 | 244353_PM_s_at | 154091 | SLC2A12 | solute carrier family 2 (facilitated glucose transporter), member 12 | 5.72E-15 | 51.6 | 100.5 |
| 105 | 215049_PM_x_at | 9332 | CD163 | CD163 molecule | 6.21E-15 | 344.5 | 112.9 |
| 106 | 1552510_PM_at | 142680 | SLC34A3 | solute carrier family 34 (sodium phosphate), member 3 | 6.40E-15 | 95.6 | 206.6 |
| 107 | 225636_PM_at | 6773 | STAT2 | signal transducer and activator of transcription 2, 113kDa | 6.63E-15 | 711.5 | 485.3 |
| 108 | 229390_PM_at | 441168 | FAM26F | family with sequence similarity 26, member F | 6.73E-15 | 272.4 | 75.9 |
| 109 | 235229_PM_at | --- | --- | --- | 6.90E-15 | 135.0 | 41.9 |
| 110 | 226218_PM_at | 3575 | IL7R | interleukin 7 receptor | 7.22E-15 | 147.1 | 41.4 |
| 111 | 217028_PM_at | 7852 | CXCR4 | chemokine (C-X-C motif) receptor 4 | 7.40E-15 | 208.4 | 75.3 |
| 112 | 204655_PM_at | 6352 | CCL5 | chemokine (C-C motif) ligand 5 | 8.57E-15 | 223.4 | 66.8 |
| 113 | 227184_PM_at | 5724 | PTAFR | platelet-activating factor receptor | 8.78E-15 | 134.4 | 62.7 |
| 114 | 202748_PM_at | 2634 | GBP2 | guanylate binding protein 2, interferon-inducible | 8.91E-15 | 306.7 | 141.0 |
| 115 | 226991_PM_at | 4773 | NFATC2 | nuclear factor of activated T-cells, cytoplasmic, calcineurin-dependent 2 | 9.05E-15 | 66.7 | 30.3 |
| 116 | 216565_PM_x_at | --- | --- | --- | 9.49E-15 | 1224.6 | 779.1 |
| 117 | 203104_PM_at | 1436 | CSF1R | colony stimulating factor 1 receptor | 9.57E-15 | 42.7 | 22.1 |
| 118 | 238668_PM_at | --- | --- | --- | 9.84E-15 | 33.6 | 14.5 |

| # | Probeset ID | Entrez Gene | Gene Symbol | Gene Title | p-value (Final Phenotype) | CAN - Mean | TX- Mean |
|---|---|---|---|---|---|---|---|
| 119 | 204923_PM_at | 54440 | SASH3 | SAM and SH3 domain containing 3 | 9.93E-15 | 47.6 | 21.7 |
| 120 | 230036_PM_at | 219285 | SAMD9L | sterile alpha motif domain containing 9-like | 1.02E-14 | 128.0 | 72.7 |
| 121 | 211742_PM_s_at | 2124 | EVI2B | ecotropic viral integration site 2B | 1.03E-14 | 166.2 | 53.2 |
| 122 | 236782_PM_at | 154075 | SAMD3 | sterile alpha motif domain containing 3 | 1.11E-14 | 23.3 | 13.3 |
| 123 | 232543_PM_x_at | 64333 | ARHGAP9 | Rho GTPase activating protein 9 | 1.13E-14 | 62.3 | 25.8 |
| 124 | 231124_PM_x_at | 4063 | LY9 | lymphocyte antigen 9 | 1.18E-14 | 33.7 | 15.7 |
| 125 | 215946_PM_x_at | 3543 /// 91316 /// 91353 | IGLL1 /// IGLL3P /// LOC91316 | immunoglobulin lambda-like polypeptide 1 /// immunoglobulin lambda-like polypeptide 3, | 1.22E-14 | 187.5 | 52.1 |
| 126 | 208306_PM_x_at | 3123 | HLA-DRB1 | Major histocompatibility complex, class II, DR beta 1 | 1.25E-14 | 3695.8 | 2255.0 |
| 127 | 217235_PM_x_at | 28816 | IGLV2-11 | immunoglobulin lambda variable 2-11 | 1.29E-14 | 196.8 | 37.8 |
| 128 | 209546_PM_s_at | 8542 | APOL1 | apolipoprotein L, 1 | 1.33E-14 | 206.8 | 114.1 |
| 129 | 203416_PM_at | 963 | CD53 | CD53 molecule | 1.34E-14 | 422.7 | 157.8 |
| 130 | 211366_PM_x_at | 834 | CASP1 | caspase 1, apoptosis-related cysteine peptidase (interleukin 1, beta, convertase) | 1.35E-14 | 186.0 | 87.1 |
| 131 | 200797_PM_s_at | 4170 | MCL1 | myeloid cell leukemia sequence 1 (BCL2-related) | 1.38E-14 | 793.7 | 575.9 |
| 132 | 31845_PM_at | 2000 | ELF4 | E74-like factor 4 (ets domain transcription factor) | 1.40E-14 | 60.7 | 34.3 |
| 133 | 221841_PM_s_at | 9314 | KLF4 | Kruppel-like factor 4 (gut) | 1.48E-14 | 132.3 | 65.2 |
| 134 | 229391_PM_s_at | 441168 | FAM26F | family with sequence similarity 26, member F | 1.49E-14 | 212.1 | 73.7 |

| # | Probeset ID | Entrez Gene | Gene Symbol | Gene Title | p-value (Final Phenotype) | CAN - Mean | TX- Mean |
|---|---|---|---|---|---|---|---|
| 135 | 203645_PM_s_at | 9332 | CD163 | CD163 molecule | 1.51E-14 | 274.9 | 85.0 |
| 136 | 211643_PM_x_at | 100510044 /// 28875 /// 3514 /// 50802 | IGK@ /// IGKC /// IGKV3D-15 /// LOC10051004 4 | immunoglobulin kappa locus /// immunoglobulin kappa constant /// immunoglobulin kappa v | 1.61E-14 | 131.1 | 32.6 |
| 137 | 205488_PM_at | 3001 | GZMA | granzyme A (granzyme 1, cytotoxic T-lymphocyte-associated serine esterase 3) | 1.82E-14 | 102.3 | 33.4 |
| 138 | 201464_PM_x_at | 3725 | JUN | jun proto-oncogene | 1.90E-14 | 424.7 | 244.5 |
| 139 | 204774_PM_at | 2123 | EVI2A | ecotropic viral integration site 2A | 1.95E-14 | 114.7 | 44.9 |
| 140 | 204336_PM_s_at | 10287 | RGS19 | regulator of G-protein signaling 19 | 2.01E-14 | 135.7 | 67.0 |
| 141 | 244654_PM_at | 64005 | MYO1G | myosin IG | 2.03E-14 | 26.8 | 14.9 |
| 142 | 228442_PM_at | 4773 | NFATC2 | nuclear factor of activated T-cells, cytoplasmic, calcineurin-dependent 2 | 2.06E-14 | 62.8 | 32.0 |
| 143 | 206804_PM_at | 917 | CD3G | CD3g molecule, gamma (CD3-TCR complex) | 2.18E-14 | 36.4 | 17.3 |
| 144 | 201315_PM_x_at | 10581 | IFITM2 | interferon induced transmembrane protein 2 (1-8D) | 2.21E-14 | 3303.7 | 2175.3 |
| 145 | 203561_PM_at | 2212 | FCGR2A | Fc fragment of IgG, low affinity IIa, receptor (CD32) | 2.22E-14 | 66.4 | 29.2 |
| 146 | 219117_PM_s_at | 51303 | FKBP11 | FK506 binding protein 11, 19 kDa | 2.31E-14 | 341.3 | 192.9 |
| 147 | 242827_PM_x_at | --- | --- | --- | 2.37E-14 | 38.9 | 16.3 |
| 148 | 214768_PM_x_at | 28299 /// 3514 /// 50802 | IGK@ /// IGKC III IGKV1-5 | immunoglobulin kappa locus /// immunoglobulin kappa constant /// immunoglobulin kappa v | 2.38E-14 | 116.7 | 21.1 |
| 149 | 227253_PM_at | 1356 | CP | ceruloplasmin (ferroxidase) | 2.49E-14 | 44.7 | 22.0 |
| 150 | 209619_PM_at | 972 | CD74 | CD74 molecule, major histocompatibility complex, class II invariant chain | 2.51E-14 | 1502.3 | 864.9 |

EP 3 825 417 A2

| # | Probeset ID | Entrez Gene | Gene Symbol | Gene Title | p-value (Final Phenotype) | CAN - Mean | TX- Mean |
|---|---|---|---|---|---|---|---|
| 151 | 208966_PM_x_at | 3428 | IFI16 | interferon, gamma-inducible protein 16 | 2.65E-14 | 644.9 | 312.6 |
| 152 | 239237_PM_at | --- | --- | --- | 2.79E-14 | 25.3 | 14.5 |
| 153 | 213566_PM_at | 6039 | RNASE6 | ribonuclease, RNase A family, k6 | 2.82E-14 | 341.1 | 134.3 |
| 154 | 201288_PM_at | 397 | ARHGDIB | Rho GDP dissociation inhibitor (GDI) beta | 2.86E-14 | 542.2 | 308.1 |
| 155 | 209606_PM_at | 9595 | CYTIP | cytohesin 1 interacting protein | 2.90E-14 | 79.0 | 32.9 |
| 156 | 205758_PM_at | 925 | CD8A | CD8a molecule | 2.91E-14 | 60.3 | 22.2 |
| 157 | 202953_PM_at | 713 | C1QB | complement component 1, q subcomponent, B chain | 3.00E-14 | 401.1 | 142.5 |
| 158 | 203233_PM_at | 3566 | IL4R | interleukin 4 receptor | 3.06E-14 | 116.7 | 72.0 |
| 159 | 205270_PM_s_at | 3937 | LCP2 | lymphocyte cytosolic protein 2 (SH2 domain containing leukocyte protein of 76kDa) | 3.12E-14 | 104.4 | 44.6 |
| 160 | 223658_PM_at | 9424 | KCNK6 | potassium channel, subfamily K, member 6 | 3.18E-14 | 35.9 | 22.0 |
| 161 | 202637_PM_s_at | 3383 | ICAM1 | intercellular adhesion molecule 1 | 3.18E-14 | 89.1 | 45.7 |
| 162 | 202935_PM_s_at | 6662 | SOX9 | SRY (sex determining region Y)-box 9 | 3.18E-14 | 117.0 | 46.1 |
| 163 | 217986_PM_s_at | 11177 | BAZ1A | bromodomain adjacent to zinc finger domain, 1A | 3.21E-14 | 116.4 | 62.5 |
| 164 | 210915_PM_x_at | 28638 | TRBC2 | T cell receptor beta constant 2 | 3.27E-14 | 129.7 | 37.5 |
| 165 | 223343_PM_at | 58475 | MS4A7 | membrane-spanning 4-domains, subfamily A, member 7 | 3.38E-14 | 346.0 | 128.3 |
| 166 | 1552701_PM_a_at | 114769 | CARD16 | caspase recruitment domain family, member 16 | 3.60E-14 | 273.3 | 119.8 |
| 167 | 226659_PM_at | 50619 | DEF6 | differentially expressed in FDCP 6 homolog (mouse) | 3.63E-14 | 35.2 | 22.2 |

(continued)

| # | Probeset ID | Entrez Gene | Gene Symbol | Gene Title | p-value (Final Phenotype) | CAN - Mean | TX- Mean |
|---|---|---|---|---|---|---|---|
| 168 | 213502_PM_x_at | 91316 | LOC91316 | glucuronidase, beta/immunoglobulin lambda-like polypeptide 1 pseudogene | 3.63E-14 | 1214.7 | 419.3 |
| 169 | 219332_PM_at | 79778 | MICALL2 | MICAL-like 2 | 3.71E-14 | 68.7 | 44.4 |
| 170 | 204891_PM_s_at | 3932 | LCK | lymphocyte-specific protein tyrosine kinase | 3.74E-14 | 43.4 | 17.8 |
| 171 | 224252_PM_s_at | 53827 | FXYD5 | FXYD domain containing ion transport regulator 5 | 3.76E-14 | 73.8 | 32.5 |
| 172 | 242878_PM_at | --- | --- | --- | 3.90E-14 | 53.2 | 30.1 |
| 173 | 224709_PM_s_at | 56990 | CDC42SE2 | CDC42 small effector 2 | 4.07E-14 | 1266.2 | 935.7 |
| 174 | 40420_PM_at | 6793 | STK10 | serine/threonine kinase 10 | 4.32E-14 | 42.0 | 24.4 |
| 175 | 218084_PM_x_at | 53827 | FXYD5 | FXYD domain containing ion transport regulator 5 | 4.52E-14 | 89.2 | 39.1 |
| 176 | 218232_PM_at | 712 | C1QA | complement component 1, q subcomponent, A chain | 4.63E-14 | 197.0 | 85.8 |
| 177 | 202208_PM_s_at | 10123 | ARL4C | ADP-ribosylation factor-like 4C | 4.63E-14 | 77.0 | 42.2 |
| 178 | 220146_PM_at | 51284 | TLR7 | toll-like receptor 7 | 4.93E-14 | 31.6 | 17.8 |
| 179 | 228752_PM_at | 84766 | EFCAB4B | EF-hand calcium binding domain 4B | 5.05E-14 | 20.6 | 12.1 |
| 180 | 208948_PM_s_at | 6780 | STAU1 | staufen, RNA binding protein, homolog 1 (Drosophila) | 5.23E-14 | 1766.0 | 2467.4 |
| 181 | 211645_PM_x_at | --- | --- | --- | 5.24E-14 | 166.7 | 27.4 |
| 182 | 236295_PM_s_at | 197358 | NLRC3 | NLR family, CARD domain containing 3 | 5.28E-14 | 37.0 | 18.6 |
| 183 | 224927_PM_at | 170954 | KIAA1949 | KIAA1949 | 5.44E-14 | 160.2 | 74.7 |
| 184 | 225258_PM_at | 54751 | FBLIM1 | filamin binding LIM protein 1 | 6.03E-14 | 228.7 | 125.4 |

| # | Probeset ID | Entrez Gene | Gene Symbol | Gene Title | p-value (Final Phenotype) | CAN - Mean | TX- Mean |
|---|---|---|---|---|---|---|---|
| 185 | 202898_PM_at | 9672 | SDC3 | syndecan 3 | 6.07E-14 | 64.8 | 32.0 |
| 186 | 218789_PM_s_at | 54494 | C11orf71 | chromosome 11 open reading frame 71 | 6.12E-14 | 175.8 | 280.8 |
| 187 | 204912_PM_at | 3587 | IL10RA | interleukin 10 receptor, alpha | 6.25E-14 | 117.2 | 46.1 |
| 188 | 211582_PM_x_at | 7940 | LST1 | leukocyte specific transcript 1 | 6.48E-14 | 121.2 | 49.4 |
| 189 | 214617_PM_at | 5551 | PRF1 | perforin 1 (pore forming protein) | 6.77E-14 | 85.6 | 40.8 |
| 190 | 231887_PM_s_at | 27143 | KIAA1274 | KIAA1274 | 7.00E-14 | 45.6 | 30.0 |
| 191 | 223773_PM_s_at | 85028 | SNHG12 | small nucleolar RNA host gene 12 (non-protein coding) | 7.00E-14 | 174.8 | 93.2 |
| 192 | 202644_PM_s_at | 7128 | TNFAIP3 | tumor necrosis factor, alpha-induced protein 3 | 7.11E-14 | 278.2 | 136.6 |
| 193 | 211796_PM_s_at | 28638 /// 28639 | TRBC1 /// TRBC2 | T cell receptor beta constant 1 /// T cell receptor beta constant 2 | 7.13E-14 | 250.2 | 63.6 |
| 194 | 206254_PM_at | 1950 | EGF | epidermal growth factor | 7.38E-14 | 176.6 | 551.3 |
| 195 | 216207_PM_x_at | 28299 /// 28904 /// 3514 /// 652493 /// 652694 | IGKC /// IGKV1-5 /// IGKV1D-8 /// LOC652493 /// LOC652694 | immunoglobulin kappa constant /// immunoglobulin kappa variable 1-5 /// immunoglobulin | 7.51E-14 | 266.3 | 50.9 |
| 196 | 232311_PM_at | 567 | B2M | Beta-2-microglobulin | 7.73E-14 | 83.7 | 35.2 |
| 197 | 205466_PM_s_at | 9957 | HS3ST1 | heparan sulfate (glucosamine) 3-O-sulfotransferase 1 | 7.84E-14 | 96.0 | 42.1 |
| 198 | 203332_PM_s_at | 3635 | INPP5D | inositol polyphosphate-5-phosphatase, 145kDa | 7.89E-14 | 42.6 | 24.0 |
| 199 | 64064_PM_at | 55340 | GIMAP5 | GTPase, IMAP family member 5 | 7.98E-14 | 170.6 | 111.4 |
| 200 | 211644_PM_x_at | 3514 /// 50802 | IGK@ /// IGKC | immunoglobulin kappa locus /// immunoglobulin kappa constant | 8.04E-14 | 246.9 | 47.5 |

**Table 10: Biopsy Expression Profiling of Kidney Transplants: 2-Way Classifier AR vs. TX**

| AR vs TX | Fold Changes | p-values |
|---|---|---|
| MAPT | -3.0916 | 2.99053E-20 |
| GATSL3 /// TBC1D10A | -2.11118 | 3.16645E-17 |
| LOC100288617 | -2.04457 | 1.22069E-16 |
| ACSM2A /// ACSM2B | -1.95127 | 1.57754E-16 |
| AKR7A3 | -2.05229 | 1.75126E-16 |
| FTCD | -2.71817 | 4.97004E-16 |
| GPHN | -1.81578 | 1.17623E-15 |
| SLC13A2 | -2.18768 | 3.58849E-15 |
| DNAJC6 | -1.76929 | 5.20867E-15 |
| SLC17A3 | -1.84587 | 6.8169E-15 |
| RGN | -2.37098 | 7.84126E-15 |
| RDH12 | -4.26697 | 9.20572E-15 |
| CRYAA | -2.74677 | 1.32872E-14 |
| SLC7A13 | -4.66793 | 1.86514E-14 |
| ALB | -6.82725 | 1.89192E-14 |
| HAGH | -1.81366 | 7.33148E-14 |
| ALDH6A1 | -2.20224 | 8.91056E-14 |
| HBA1 /// HBA2 | -1.88978 | 9.34894E-14 |
| CLYBL | -1.8789 | 9.81129E-14 |
| FLJ42875 | -1.97495 | 1.45096E-13 |
| FBP1 | -2.28393 | 2.00607E-13 |
| FABP1 | -2.86164 | 2.18592E-13 |
| SLC2A5 | -2.17033 | 2.79078E-13 |
| ETNK2 | -1.92877 | 3.8779E-13 |
| ADH6 | -2.40152 | 4.1841E-13 |
| PEPD | -1.97852 | 4.63261E-13 |
| PPAPDC1A | -2.02799 | 6.60672E-13 |
| CLCNKA /// CLCNKB | -1.89433 | 7.0432E-13 |
| LPPR1 | -2.46608 | 8.09001E-13 |
| PNPLA3 | -2.89596 | 8.8556E-13 |
| GSTA1 | -1.92437 | 1.16843E-12 |
| DHDH | -3.08428 | 1.29848E-12 |
| HPD | -3.98241 | 1.30435E-12 |
| HIBADH | -1.89283 | 1.4274E-12 |
| EPHX2 | -2.11633 | 1.98928E-12 |
| MAF | -1.76205 | 2.0964E-12 |
| TMEM178 | -1.78847 | 2.20622E-12 |

(continued)

| AR vs TX | Fold Changes | p-values |
|---|---|---|
| LOC642891 | -1.73984 | 2.80875E-12 |
| LOC285733 | -1.95994 | 3.23545E-12 |
| FMO1 | -2.25513 | 4.50736E-12 |
| GGT6 | -2.66101 | 4.6681E-12 |
| FLJ22763 | -1.93955 | 5.14404E-12 |
| SLC6A13 | -2.24946 | 5.34043E-12 |
| PTGER3 | -1.81112 | 5.52121E-12 |
| SLC30A8 | -1.84747 | 6.11346E-12 |
| IGSF11 | -2.21766 | 6.61316E-12 |
| CYP3A7 | -2.91581 | 6.87921E-12 |
| LRRC43 | -1.9031 | 8.14997E-12 |
| APOH | -5.57453 | 8.27826E-12 |
| FAM83D | -2.32876 | 8.74785E-12 |
| ASPA | -2.31579 | 8.80911E-12 |
| PROZ | -2.78599 | 9.23614E-12 |
| TNNC1 | -1.79223 | 1.01194E-11 |
| PRODH2 | -3.38674 | 1.25579E-11 |
| C15orf59 | -1.77893 | 1.64304E-11 |
| GLYCTK | -1.82955 | 1.70772E-11 |
| KMO | -2.36048 | 1.9935E-11 |
| KNG1 | -2.63283 | 2.20875E-11 |
| LDHD | -2.57191 | 2.27083E-11 |
| CDHR5 | -2.63356 | 2.2819E-11 |
| HAAO | -1.84903 | 2.42843E-11 |
| ACSF2 | -2.00048 | 2.48828E-11 |
| SLC37A4 | -2.01845 | 2.87337E-11 |
| TINAG | -1.8544 | 2.91111E-11 |
| PBLD | -1.91883 | 3.1387E-11 |
| CYP46A1 | -1.93139 | 3.14729E-11 |
| CNDP1 | -2.3083 | 3.26491E-11 |
| SLC12A6 | -2.31688 | 3.56291E-11 |
| AFM | -6.66098 | 3.67152E-11 |
| C10orf72 | -1.76232 | 3.70569E-11 |
| CLCNKB | -1.96378 | 3.83317E-11 |
| SLC6A19 | -3.99223 | 3.8873E-11 |
| ATP6V0D2 | -2.33798 | 4.04264E-11 |
| RHBG | -1.80807 | 4.19595E-11 |
| PM20D1 | -2.51866 | 4.26226E-11 |

(continued)

| AR vs TX | Fold Changes | p-values |
|---|---|---|
| ACADSB | -2.10959 | 4.46915E-11 |
| CLDN8 | -2.16632 | 4.88256E-11 |
| DEPDC7 | -1.85669 | 5.00337E-11 |
| C2orf54 | -1.7955 | 5.39601E-11 |
| FAM151A | -5.32405 | 5.61188E-11 |
| SLC22A11 | -2.10205 | 5.65313E-11 |
| RALYL | -2.47687 | 5.78764E-11 |
| ACSM5 | -1.88278 | 5.80564E-11 |
| TMEM207 | -2.95205 | 6.13829E-11 |
| RUNDC3B | -2.06687 | 6.42529E-11 |
| ANGPTL3 | -2.56539 | 7.90237E-11 |
| LOC388588 | -2.00381 | 9.24089E-11 |
| APOM | -2.50604 | 9.25689E-11 |
| KCNJ15 | -2.03561 | 9.83258E-11 |
| ZYG11A | -2.40225 | 1.04754E-10 |
| AGXT | -2.6006 | 1.17202E-10 |
| UPB1 | -2.7529 | 1.20683E-10 |
| CAPN3 | -2.1059 | 1.23158E-10 |
| ENTPD5 | -1.95549 | 1.28898E-10 |
| METTL7B | -2.18054 | 1.2938E-10 |
| KHK | -2.60696 | 1.31642E-10 |
| LOC284578 | -1.90116 | 1.38269E-10 |
| PDZD3 | -1.83091 | 1.46276E-10 |
| SERPINA5 | -1.74937 | 1.6048E-10 |
| TM4SF5 | -2.64814 | 1.71797E-10 |
| NT5E | -1.77876 | 1.82759E-10 |
| CTH | -1.95197 | 1.84254E-10 |
| AQP11 | -2.0271 | 2.48812E-10 |
| SLC34A3 | -2.04588 | 2.84241E-10 |
| DMGDH | -2.55618 | 3.18184E-10 |
| SORD | -2.24476 | 3.30972E-10 |
| DPEP1 | -3.05594 | 3.61209E-10 |
| LOC153328 | -1.8068 | 3.80205E-10 |
| FOLH1 | -2.32575 | 3.80509E-10 |
| PLCXD3 | -1.76665 | 3.89412E-10 |
| OSTBETA | -2.43601 | 3.93207E-10 |
| ABCC2 | -2.53512 | 3.97325E-10 |
| ECHDC3 | -1.73755 | 3.97793E-10 |

(continued)

| AR vs TX | Fold Changes | p-values |
|---|---|---|
| CRYL1 | -1.82953 | 4.00197E-10 |
| SLC13A3 | -2.13984 | 4.04239E-10 |
| SLC16A10 | -2.44322 | 4.7162E-10 |
| C9orf66 | -3.0274 | 5.03177E-10 |
| GPR98 | -4.26595 | 6.258E-10 |
| HA02 | -3.23686 | 6.39362E-10 |
| CLPTM1 | -2.00927 | 6.66131E-10 |
| ACP5 | -2.79078 | 6.66908E-10 |
| ISOC2 | -1.8934 | 6.90662E-10 |
| FOLH1B | -2.76374 | 7.11947E-10 |
| TLN2 | -2.37363 | 7.32503E-10 |
| GALM | -1.91896 | 7.36792E-10 |
| OSTalpha | -2.78066 | 7.52226E-10 |
| SLC5A11 | -2.75896 | 8.19688E-10 |
| EPHX1 | -2.00145 | 8.56848E-10 |
| GAS2 | -1.80474 | 9.28707E-10 |
| NOX4 | -2.05807 | 1.01236E-09 |
| FGF1 | -1.82212 | 1.24912E-09 |
| FCAMR | -2.07507 | 1.27892E-09 |
| LOC100130691 | -2.59795 | 1.29088E-09 |
| ARSE | -1.80351 | 1.31681E-09 |
| RETSAT | -1.76392 | 1.36913E-09 |
| SLC22A7 | -2.26476 | 1.51477E-09 |
| C7orf10 | -2.15451 | 1.6297E-09 |
| SERPINA6 | -3.41598 | 1.63356E-09 |
| CPNE6 | -1.8087 | 1.66697E-09 |
| MAP7D2 | -1.74996 | 1.92036E-09 |
| SEPP1 | -1.90167 | 2.17198E-09 |
| AGXT2L1 | -3.23817 | 2.32327E-09 |
| C4orf12 | -2.14021 | 2.3661E-09 |
| RAB11FIP3 | -2.44555 | 2.48943E-09 |
| GK /// GK3P | -1.83394 | 2.70788E-09 |
| PC | -2.6032 | 2.83833E-09 |
| PANK1 | -2.15352 | 3.13166E-09 |
| NAGS | -2.18509 | 3.13645E-09 |
| MME | -2.67889 | 3.20928E-09 |
| SUSD2 | -2.3654 | 3.35377E-09 |
| SLC3A2 | -2.00345 | 3.38775E-09 |

(continued)

| AR vs TX | Fold Changes | p-values |
|---|---|---|
| AMDHD1 | -2.76004 | 3.69681E-09 |
| MTTP | -2.43293 | 3.87705E-09 |
| PHYH | -1.84295 | 3.99573E-09 |
| CYP17A1 | -2.01122 | 4.11531E-09 |
| BPHL | -2.37599 | 4.15467E-09 |
| ASB9 | -2.91835 | 4.72846E-09 |
| CYP2B6 /// CYP2B7P1 | -3.27876 | 4.9278E-09 |
| HADH | -1.95557 | 5.18111E-09 |
| AQP7 | -2.04383 | 5.46752E-09 |
| SLC10A2 | -2.73836 | 5.7561E-09 |
| ZMYND12 | -2.1429 | 5.83754E-09 |
| HIBCH | -1.80435 | 5.87555E-09 |
| CYP4F3 | -2.53506 | 5.96184E-09 |
| MGAM | -2.1997 | 6.22815E-09 |
| CYP4F2 /// CYP4F3 | -3.3825 | 6.27466E-09 |
| SLC22A12 | -2.11852 | 7.58307E-09 |
| QPRT | -1.87851 | 7.79844E-09 |
| THY1 | -2.21775 | 7.97833E-09 |
| SNTA1 | -1.9336 | 9.67285E-09 |
| ACOT1 /// ACOT2 | -1.78472 | 9.85738E-09 |
| SLC2A2 | -1.89882 | 1.00173E-08 |
| MYH8 | -2.11404 | 1.02047E-08 |
| SLC6A18 | -1.86164 | 1.08202E-08 |
| PRAP1 | -2.21358 | 1.09557E-08 |
| MRO | -4.28065 | 1.13633E-08 |
| EHHADH | -1.77338 | 1.14067E-08 |
| DIO1 | -3.00979 | 1.33367E-08 |
| TTC36 | -2.36726 | 1.36035E-08 |
| PSAT1 | -2.38335 | 1.3782E-08 |
| ATP6V1G3 | -2.23634 | 1.38236E-08 |
| ACE2 | -1.79037 | 1.42874E-08 |
| GJB1 | -1.96896 | 1.44201E-08 |
| SLC22A6 | -2.86071 | 1.46506E-08 |
| TRIM50 | -2.72359 | 1.48482E-08 |
| SOST | -3.04185 | 1.52557E-08 |
| ESPL1 | -2.15374 | 1.5528E-08 |
| CALB1 | -2.65581 | 1.57872E-08 |
| AZGP1 | -2.47351 | 1.60913E-08 |

(continued)

| AR vs TX | Fold Changes | p-values |
|---|---|---|
| PXMP2 | -1.95647 | 1.66904E-08 |
| TMEM120A | -1.83373 | 1.84556E-08 |
| KLK1 | -4.86671 | 1.85761E-08 |
| SLC22A8 | -3.51523 | 1.87712E-08 |
| FUT3 | -1.8837 | 1.93982E-08 |
| SLC7A8 | -1.91659 | 1.99023E-08 |
| PDK2 | -1.97685 | 2.13903E-08 |
| ANXA9 | -2.33358 | 2.23676E-08 |
| P4HA2 | -1.94378 | 2.2935E-08 |
| SLC34A1 | -2.68387 | 2.29418E-08 |
| ACY3 | -2.02646 | 2.33713E-08 |
| C2orf40 | -2.555 | 2.37292E-08 |
| FUT6 | -1.92525 | 2.41426E-08 |
| ACOT7 | -1.95494 | 2.45886E-08 |
| PNPLA1 | -1.78439 | 2.48072E-08 |
| ABAT | -1.82921 | 2.80329E-08 |
| UPP2 | -3.15846 | 3.23662E-08 |
| MOSC2 | -2.32105 | 3.32422E-08 |
| LOC389332 | -1.76102 | 3.35953E-08 |
| ALDH1B1 | -1.77365 | 3.4294E-08 |
| GPC5 | -2.35239 | 3.47079E-08 |
| AMN | -2.01448 | 3.76136E-08 |
| SLC22A13 | -1.95166 | 3.79569E-08 |
| CALML3 | -2.53824 | 3.9564E-08 |
| PDXP /// SH3BP1 | -1.90447 | 3.96765E-08 |
| RHCG | -2.56004 | 4.20488E-08 |
| VEPH1 | -1.98345 | 4.37645E-08 |
| GC | -1.79578 | 4.8255E-08 |
| RNF186 | -2.27866 | 5.26723E-08 |
| GAL3ST1 | -1.97099 | 5.32145E-08 |
| GLYAT | -2.45554 | 5.56573E-08 |
| BPI | -1.8412 | 5.56594E-08 |
| REEP6 | -2.30623 | 5.70126E-08 |
| ACOT4 | -1.9105 | 6.45152E-08 |
| MUC13 | -2.12245 | 6.98563E-08 |
| NPR3 | -2.54362 | 7.01278E-08 |
| RAB11FIP5 | -1.7414 | 7.17374E-08 |
| SLC12A3 | -2.79439 | 7.49512E-08 |

(continued)

| AR vs TX | Fold Changes | p-values |
|---|---|---|
| EGF | -3.16114 | 8.28225E-08 |
| HMGCS2 | -2.1871 | 8.66021E-08 |
| SLC5A10 | -2.39748 | 8.98765E-08 |
| TUBAL3 | -2.16082 | 9.26757E-08 |
| LOC145837 | -2.93781 | 9.38032E-08 |
| SLC7A9 | -2.43122 | 9.7596E-08 |
| LOC727944 | -2.81085 | 9.79329E-08 |
| CYP4F2 | -3.50762 | 1.00843E-07 |
| SLC5A2 | -1.91024 | 1.07955E-07 |
| DHDPSL | -1.82011 | 1.08169E-07 |
| MIOX | -3.2511 | 1.10411E-07 |
| CPN2 | -2.12523 | 1.1914E-07 |
| SLC7A7 | -2.02602 | 1.22723E-07 |
| CYP4A11 /// CYP4A22 | -1.98138 | 1.28529E-07 |
| G6PC | -3.25106 | 1.28591E-07 |
| DDC | -2.62111 | 1.28799E-07 |
| RGS7 | -1.76457 | 1.30087E-07 |
| PRNP | -1.77477 | 1.35141E-07 |
| SLC4A9 | -1.95261 | 1.3642E-07 |
| PAH | -3.20056 | 1.43122E-07 |
| ESRRG | -1.73711 | 1.52263E-07 |
| SLC26A9 | -1.74242 | 1.53483E-07 |
| LOC644242 | -1.91415 | 1.67473E-07 |
| CTXN3 | -4.3921 | 1.68953E-07 |
| UGT1A8 /// UGT1A9 | -3.14494 | 1.86107E-07 |
| PCK2 | -2.70246 | 1.87667E-07 |
| ABP1 | -2.82479 | 2.01634E-07 |
| HNF4G | -1.92184 | 2.02132E-07 |
| CYP3A4 | -2.29038 | 2.02506E-07 |
| C21orf33 | -1.76567 | 2.1446E-07 |
| CUBN | -2.30227 | 2.21237E-07 |
| FM04 | -2.66707 | 2.24563E-07 |
| GPD1 | -2.0302 | 2.35214E-07 |
| APOC3 | -2.11763 | 2.36005E-07 |
| GK3P | -2.07563 | 2.40413E-07 |
| GK | -2.20019 | 2.40894E-07 |
| ENPP6 | -2.67481 | 2.45316E-07 |
| ASS1 | -2.1157 | 2.62889E-07 |

(continued)

| AR vs TX | Fold Changes | p-values |
|---|---|---|
| DAO | -3.44454 | 2.65842E-07 |
| HPGD | -2.38564 | 2.72233E-07 |
| GCHFR | -1.75487 | 2.94943E-07 |
| TMEM174 | -3.55663 | 2.96489E-07 |
| CHDH | -2.24862 | 2.99682E-07 |
| CLEC18A /// CLEC18B /// CLEC18C | -2.16625 | 3.19031E-07 |
| UMOD | -2.26429 | 3.21104E-07 |
| HRG | -2.98761 | 3.27498E-07 |
| TSKU | -1.73688 | 3.29833E-07 |
| HSD17B14 | -2.18571 | 3.50438E-07 |
| SLC23A3 | -2.59008 | 3.53828E-07 |
| ITLN1 | -1.87748 | 3.67115E-07 |
| CAMK2G | -1.73622 | 4.07919E-07 |
| GLYATL1 | -2.38225 | 4.39935E-07 |
| UGT2B28 | -1.99594 | 5.11969E-07 |
| A2LD1 | -2.47322 | 5.71176E-07 |
| ALAD | -1.88239 | 5.71331E-07 |
| HSD11B2 | -2.18597 | 5.81499E-07 |
| GDPD3 | -2.23762 | 6.32071E-07 |
| CSDC2 | -1.98441 | 6.72915E-07 |
| AQP3 | -1.94567 | 7.02888E-07 |
| PLG | -3.86142 | 7.96637E-07 |
| PGPEP1 | -1.89438 | 8.15391E-07 |
| LPA /// PLG | -4.61775 | 8.82027E-07 |
| PAQR7 | -1.85889 | 9.01772E-07 |
| SLC27A2 | -1.92807 | 9.02555E-07 |
| C18orf56 | -2.04976 | 9.15996E-07 |
| SLC23A1 | -2.80195 | 9.52247E-07 |
| ZDHHC9 | -1.73908 | 1.08396E-06 |
| C12orf64 | -2.19674 | 1.10586E-06 |
| SLC25A10 | -2.41468 | 1.18238E-06 |
| ANK2 | -1.90934 | 1.20132E-06 |
| USP2 | -2.02703 | 1.21673E-06 |
| COLEC11 | -2.43455 | 1.36731E-06 |
| PKLR | -2.17875 | 1.37898E-06 |
| GPT2 | -2.34268 | 1.46992E-06 |
| CDHR2 | -1.91669 | 1.4866E-06 |
| ACOX2 | -2.2757 | 1.49911E-06 |

(continued)

| AR vs TX | Fold Changes | p-values |
|---|---|---|
| XPNPEP2 | -2.99801 | 1.50402E-06 |
| HEPACAM2 | -2.2925 | 1.60973E-06 |
| UBE2QL1 | -2.15896 | 1.63642E-06 |
| SLC39A5 | -2.74063 | 1.69299E-06 |
| TMEM106A | -2.02395 | 1.69787E-06 |
| ATP6V0E2 | -1.78673 | 1.86877E-06 |
| AK3L1 | -1.93512 | 1.90809E-06 |
| AP1M2 | -1.9461 | 2.03528E-06 |
| ARSF | -2.25573 | 2.07251E-06 |
| AGXT2 | -2.22933 | 2.16372E-06 |
| DAK | -2.20229 | 2.36754E-06 |
| SLC2A12 | -1.77017 | 2.43975E-06 |
| ALDOB | -1.76925 | 2.55745E-06 |
| ALDH4A1 | -2.03136 | 2.70075E-06 |
| SFXN2 | -2.60877 | 3.45507E-06 |
| DIP2C | -2.05583 | 3.64214E-06 |
| CTSL2 | -2.52968 | 3.71723E-06 |
| SLC28A2 | -1.93371 | 3.82506E-06 |
| FUZ | -1.79456 | 3.95111E-06 |
| C19orf69 | -2.09232 | 4.14502E-06 |
| REN | -1.85222 | 5.09502E-06 |
| C12orf59 | -1.75089 | 5.13955E-06 |
| TMEM132E | -2.18984 | 5.33005E-06 |
| ZGPAT | -2.44576 | 5.34764E-06 |
| PLEKHA5 | -1.96129 | 5.89183E-06 |
| GRAMD1C | -1.74771 | 6.11458E-06 |
| DUSP9 | -1.91589 | 6.38564E-06 |
| SLC22A4 | -1.89514 | 6.66544E-06 |
| SORCS1 | -1.85441 | 7.11084E-06 |
| STC2 | -1.81501 | 7.21338E-06 |
| LEAP2 | -2.38133 | 7.40637E-06 |
| KCNN2 | -2.25018 | 7.51697E-06 |
| IDH1 | -1.8165 | 7.62782E-06 |
| NAT8B | -2.42914 | 7.8931E-06 |
| SLC5A9 | -1.75921 | 9.82367E-06 |
| TM7SF2 | -1.92027 | 9.949E-06 |
| PLA2G12B | -2.13901 | 1.09806E-05 |
| CLRN3 | -1.91704 | 1.10718E-05 |

(continued)

| AR vs TX | Fold Changes | p-values |
|---|---|---|
| PCYT2 | -1.80892 | 1.18832E-05 |
| SLC4A1 | -2.48783 | 1.19999E-05 |
| GSTA3 | -2.10371 | 1.22206E-05 |
| PVALB | -2.29588 | 1.2453E-05 |
| ATP6V1C2 | -1.98979 | 1.29667E-05 |
| PIK3C2G | -1.76675 | 1.29855E-05 |
| DHRS11 | -1.74961 | 1.35061E-05 |
| NEDD4L | -1.89373 | 1.41229E-05 |
| AQP2 | -2.03409 | 1.4403E-05 |
| SLC5A12 | -2.19859 | 1.46257E-05 |
| AS3MT | -1.83726 | 1.71006E-05 |
| AHCY | -1.8639 | 1.78963E-05 |
| PIPOX | -2.48991 | 1.79528E-05 |
| CEL | -1.82327 | 1.812E-05 |
| DCXR | -2.16471 | 1.92539E-05 |
| CYP4A11 | -1.86722 | 2.10549E-05 |
| C4orf31 | -1.82811 | 2.58079E-05 |
| CYP4A22 | -2.07108 | 2.87185E-05 |
| GLTPD2 | -2.11301 | 3.06781E-05 |
| MAP7 | -2.19683 | 3.85037E-05 |
| TMEM86A | -1.89253 | 4.99658E-05 |
| SLC25A1 | -1.82031 | 5.0373E-05 |
| DPP4 | -2.22087 | 5.57627E-05 |
| IYD | -2.08101 | 5.9404E-05 |
| UGT1A1 /// UGT1A10 /// UGT1A3 /// UGT1A4 /// UGT1A5 /// UGT1A6 /// UGT1A7 /// UGT1A8 /// UGT1A9 | -1.8015 | 6.24555E-05 |
| A1CF | -2.20926 | 6.47761E-05 |
| C5orf23 | -1.93642 | 7.84432E-05 |
| NECAB2 | -3.13495 | 8.16616E-05 |
| RNLS | -1.98084 | 8.33272E-05 |
| DNMT3L | -2.65364 | 8.93726E-05 |
| NPHS2 | -1.90039 | 9.36204E-05 |
| RAB17 | -1.79062 | 0.000102017 |
| ABCC6 /// LOC100292715 | -1.9274 | 0.000110765 |
| MUC15 | -1.84539 | 0.000111654 |
| PPP1R16A | -1.89951 | 0.000117374 |
| KCNK5 | -1.76687 | 0.000126376 |
| LOC100287428 | -1.84499 | 0.000134038 |

(continued)

| AR vs TX | Fold Changes | p-values |
|---|---|---|
| APOE | -1.85436 | 0.000138448 |
| GIPC2 | -1.94676 | 0.00014941 |
| C11orf54 | -1.75689 | 0.000156408 |
| SLC17A5 | -1.74223 | 0.000163743 |
| KL | -1.8958 | 0.000167359 |
| CYP8B1 | -2.25808 | 0.000167576 |
| DPYS | -2.08317 | 0.000176199 |
| SH3GL2 | -1.91695 | 0.000186793 |
| DHRS4 /// DHRS4L2 | -2.07083 | 0.000208985 |
| ALLC | -1.78998 | 0.000234174 |
| PTGR1 | -1.75022 | 0.000261226 |
| CRHBP | -2.11391 | 0.00027295 |
| C9orf103 | -1.88471 | 0.00056122 |
| SLC28A1 | -1.77452 | 0.001183781 |
| SLC16A9 | -1.89967 | 0.001212523 |
| ABHD6 | -1.8011 | 0.001402915 |
| BHMT | -1.86117 | 0.003092475 |
| CRYM | -1.84582 | 0.004771269 |
| RENBP | -1.85157 | 0.00698823 |
| ABCC6P1 | -1.87408 | 0.008304558 |
| L2HGDH | -1.79573 | 0.025300732 |
| ACSM3 | -1.79192 | 0.029160166 |

**Table 11: Biopsy Expression Profiling of Kidney Transplants: 2-Way Classifier CAN/IFTA vs. TX**

| IFTA vs TX | Fold Changes | p-values |
|---|---|---|
| TMSB10 | 1.51781 | 3.98249E-09 |
| S100A11 | 1.56822 | 1.38989E-08 |
| SLCO2A1 | 1.63966 | 3.44566E-08 |
| HLA-DRB1 /// HLA-DRB4 | 1.58546 | 4.3499E-08 |
| HLA-DRB1 | 1.55271 | 5.38233E-08 |
| SLC2A12 | -1.70211 | 5.46662E-08 |
| VCAM1 | 1.65026 | 6.48536E-08 |
| UPP1 | 1.63064 | 1.16852E-07 |
| LDLRAD3 | 1.65949 | 1.26498E-07 |
| SH2B3 | 1.54593 | 1.36597E-07 |
| EGF | -2.89482 | 1.71342E-07 |
| MLL3 | 1.65249 | 2.14515E-07 |

(continued)

| IFTA vs TX | Fold Changes | p-values |
|---|---|---|
| TRIM50 | -2.12508 | 2.24723E-07 |
| AKNA | 1.53574 | 2.41689E-07 |
| GIMAP5 | 1.56499 | 2.67192E-07 |
| HLA-DMB | 1.57184 | 2.90879E-07 |
| CKLF | 1.55882 | 4.12568E-07 |
| SLC34A3 | -1.63066 | 5.51514E-07 |
| HS3ST1 | 1.53641 | 5.56865E-07 |
| MARCKSL1 | 1.61621 | 5.84424E-07 |
| SLC16A7 | -1.62101 | 7.39903E-07 |
| B2M | 1.50123 | 9.50899E-07 |
| TSPAN13 | 1.53438 | 9.8449E-07 |
| PARVG | 1.58576 | 1.07603E-06 |
| TUBA1A | 1.62753 | 1.08009E-06 |
| GUCY1A3 | 1.50036 | 1.08029E-06 |
| HLA-F | 1.52472 | 1.176E-06 |
| PARP12 | 1.51868 | 1.20425E-06 |
| GAB3 | 1.51079 | 1.3961E-06 |
| ELF4 | 1.63538 | 1.52125E-06 |
| FAM83D | -1.89217 | 1.53199E-06 |
| PARP14 | 1.56897 | 1.53554E-06 |
| LCP2 | 1.6147 | 1.54053E-06 |
| SAMD9L | 1.61164 | 1.55377E-06 |
| ASNS | 1.64299 | 1.56911E-06 |
| RNF213 | 1.52854 | 1.57479E-06 |
| FERMT3 | 1.57985 | 1.59445E-06 |
| APOL1 | 1.57279 | 1.60354E-06 |
| TNFAIP8L2 | 1.51502 | 1.70532E-06 |
| PSMB10 | 1.54422 | 1.75542E-06 |
| PMEPA1 | 1.66003 | 2.04838E-06 |
| RAB31 | 1.6387 | 2.08849E-06 |
| PRR24 | 1.54645 | 2.08952E-06 |
| SMPDL3A | -1.50869 | 2.10279E-06 |
| RRAS | 1.51587 | 2.26269E-06 |
| PVALB | -2.2572 | 2.72451E-06 |
| PSMB8 | 1.56268 | 2.72636E-06 |
| TM4SF5 | -1.80583 | 2.77734E-06 |
| A2LD1 | -1.77136 | 2.85983E-06 |
| NUAK1 | 1.53844 | 2.92207E-06 |

(continued)

| IFTA vs TX | Fold Changes | p-values |
|---|---|---|
| MCAM | 1.6564 | 3.11631E-06 |
| RNLS | -1.5429 | 3.14475E-06 |
| ASB9 | -1.79403 | 3.1805E-06 |
| IL15RA | 1.60325 | 3.26002E-06 |
| LOC645895 /// MYBL1 | -1.52279 | 3.36189E-06 |
| MST4 | 1.57651 | 3.39448E-06 |
| COL4A2 | 1.61415 | 3.54382E-06 |
| NPHS1 | -1.77154 | 3.58126E-06 |
| KCNN2 | -1.95169 | 3.61421E-06 |
| NECAB2 | -1.83644 | 3.61683E-06 |
| GGT5 | 1.5766 | 4.21195E-06 |
| EFCAB4B | 1.52589 | 4.27242E-06 |
| TLR2 | 1.58142 | 4.27452E-06 |
| C21orf63 | 1.53537 | 4.30722E-06 |
| GLTPD2 | -1.71616 | 5.05996E-06 |
| TMPRSS2 | -1.52104 | 5.44787E-06 |
| MRO | -2.43891 | 5.4527E-06 |
| MBOAT1 | 1.54611 | 5.70031E-06 |
| KLK1 | -2.708 | 6.20862E-06 |
| IDH1 | -1.50367 | 6.55865E-06 |
| ARRDC2 | 1.66068 | 6.57954E-06 |
| UBE2QL1 | -1.76895 | 6.76469E-06 |
| ALB | -4.11767 | 6.82617E-06 |
| ZYG11A | -1.92637 | 6.83448E-06 |
| SAMD3 | 1.58295 | 7.06467E-06 |
| GPR98 | -2.62258 | 7.19656E-06 |
| MLKL | 1.56773 | 7.21744E-06 |
| SLC2A5 | -1.91328 | 7.25168E-06 |
| KLF4 | 1.56399 | 7.33393E-06 |
| ITPRIPL2 | 1.55643 | 7.34185E-06 |
| APOL6 | 1.55686 | 7.34937E-06 |
| LOC153328 | -1.54066 | 7.35108E-06 |
| SPATA17 | -1.58696 | 7.36371E-06 |
| IRF7 | 1.57063 | 7.78387E-06 |
| CYP3A4 | -1.68477 | 8.04902E-06 |
| CIITA | 1.59697 | 8.05175E-06 |
| PRKCDBP | 1.52369 | 8.25806E-06 |
| CTSL2 | -2.2103 | 9.30206E-06 |

(continued)

| IFTA vs TX | Fold Changes | p-values |
|---|---|---|
| C12orf35 | 1.61211 | 9.52663E-06 |
| GATSL3 /// TBC1D10A | -1.59015 | 1.02924E-05 |
| CCR5 | 1.61292 | 1.04302E-05 |
| LOC91316 | 1.55332 | 1.09782E-05 |
| LHFP | 1.61605 | 1.10787E-05 |
| ITGAL | 1.65885 | 1.22092E-05 |
| SLC12A3 | -2.04892 | 1.22155E-05 |
| PLAUR | 1.60949 | 1.23043E-05 |
| PPAP2C | 1.63507 | 1.23102E-05 |
| IER5 | 1.52273 | 1.23557E-05 |
| CYP3A7 | -2.0504 | 1.28185E-05 |
| SLC4A1 | -1.81494 | 1.34578E-05 |
| EBF1 | 1.52946 | 1.35965E-05 |
| IL16 | 1.51006 | 1.50211E-05 |
| HRG | -1.90476 | 1.51876E-05 |
| PRKCB | 1.59664 | 1.56904E-05 |
| SYCE1L | 1.61979 | 1.58566E-05 |
| PARP8 | 1.63188 | 1.59697E-05 |
| FAM65B | 1.56435 | 1.69584E-05 |
| PTGER3 | -1.89681 | 1.7065E-05 |
| FIBIN | 1.62824 | 1.74203E-05 |
| RNF182 | 1.50841 | 1.82387E-05 |
| ANGPTL3 | -2.04101 | 1.82518E-05 |
| PHACTR3 | 1.51835 | 1.84497E-05 |
| FKBP11 | 1.64574 | 1.84525E-05 |
| CARD6 | 1.50388 | 1.84747E-05 |
| CD1D | 1.59602 | 1.85397E-05 |
| GMFG | 1.64971 | 1.85486E-05 |
| AFM | -3.09543 | 1.90192E-05 |
| LAYN | 1.50713 | 1.91771E-05 |
| BIN2 | 1.5738 | 2.00422E-05 |
| HADH | -1.53494 | 2.01328E-05 |
| CLDN3 | 1.58139 | 2.01595E-05 |
| IL8 | 1.6304 | 2.06309E-05 |
| CHST15 | 1.57068 | 2.08254E-05 |
| HLA-DOB | 1.57448 | 2.08412E-05 |
| ALDH3A2 | -1.51338 | 2.24457E-05 |
| G6PC | -2.06203 | 2.31973E-05 |

(continued)

| IFTA vs TX | Fold Changes | p-values |
|---|---|---|
| LOC153684 | 1.51481 | 2.35676E-05 |
| BTN3A3 | 1.58385 | 2.36092E-05 |
| LOC96610 | 1.54288 | 2.37697E-05 |
| PRSS2 /// PRSS3 | -1.55684 | 2.39309E-05 |
| AADAT | -1.50725 | 2.45144E-05 |
| ATP6V1C2 | -1.87663 | 2.51529E-05 |
| FAM84A | 1.63054 | 2.72254E-05 |
| MYADM | 1.57406 | 2.72605E-05 |
| LOC727944 | -2.1898 | 2.74724E-05 |
| MOSC2 | -1.62916 | 2.95301E-05 |
| SLCO3A1 | 1.55284 | 2.95888E-05 |
| RSPH1 | 1.64647 | 2.97493E-05 |
| C1orf192 | 1.60412 | 2.99924E-05 |
| NNT | -1.64647 | 3.17285E-05 |
| SEL1L3 | 1.61562 | 3.17683E-05 |
| C9orf103 | -1.61381 | 3.20544E-05 |
| CD74 | 1.62637 | 3.27408E-05 |
| JUN | 1.63578 | 3.27609E-05 |
| RGS7 | -1.53976 | 3.27856E-05 |
| AOAH | 1.58066 | 3.28623E-05 |
| FAM24B | -1.53027 | 3.30462E-05 |
| NCKAP1L | 1.59544 | 3.3182E-05 |
| DCDC2 | 1.52668 | 3.46016E-05 |
| PLCL1 | -1.5323 | 3.47883E-05 |
| SERPINA6 | -1.72919 | 3.5546E-05 |
| CPNE6 | -1.52074 | 3.67948E-05 |
| LPCAT1 | 1.62907 | 3.68576E-05 |
| ADM | -1.53891 | 3.74019E-05 |
| ELMOD1 | -1.64162 | 3.79781E-05 |
| MSL1 | 1.56313 | 3.84376E-05 |
| PLA2G12B | -1.7228 | 3.89562E-05 |
| TUBB6 | 1.61007 | 4.06407E-05 |
| CDC42EP5 | 1.61246 | 4.08613E-05 |
| TLN2 | -1.51459 | 4.21746E-05 |
| TAP2 | 1.59627 | 4.28878E-05 |
| EMP3 | 1.52713 | 4.48564E-05 |
| VEGFA | -1.50122 | 4.7167E-05 |
| SIRPG | 1.57626 | 5.08523E-05 |

(continued)

| IFTA vs TX | Fold Changes | p-values |
|---|---|---|
| HCK | 1.62706 | 5.08921E-05 |
| ALAD | -1.51752 | 5.34836E-05 |
| GK3P | -1.67708 | 5.42274E-05 |
| CCR2 | 1.5253 | 5.72632E-05 |
| GSTA3 | -1.5681 | 5.78607E-05 |
| FYB | 1.55028 | 5.88739E-05 |
| ACADSB | -1.50559 | 6.10124E-05 |
| FOLH1B | -1.9831 | 6.11789E-05 |
| CALML3 | -1.94826 | 6.21615E-05 |
| LOC100289727 /// NCF1 /// NCF1B /// NCF1C | 1.58313 | 6.39151E-05 |
| PIGR | 1.50262 | 6.4456E-05 |
| SLC16A10 | -1.85159 | 6.50594E-05 |
| ANXA3 | 1.66158 | 6.59979E-05 |
| GK | -1.88206 | 6.68014E-05 |
| MX1 | 1.59966 | 6.80462E-05 |
| SORCS1 | -1.61133 | 6.84525E-05 |
| DNMT3L | -1.9539 | 7.10165E-05 |
| ARHGAP28 | -1.56725 | 7.16985E-05 |
| APOH | -2.43413 | 7.25988E-05 |
| RHCG | -1.86366 | 7.39257E-05 |
| C15orf59 | -1.54017 | 7.44282E-05 |
| MX2 | 1.65683 | 7.78195E-05 |
| IFI27 | 1.51629 | 7.82297E-05 |
| FOLH1 | -1.96392 | 8.12122E-05 |
| TMEM207 | -2.29284 | 8.39494E-05 |
| GPR65 | 1.63506 | 9.08757E-05 |
| CAMK1D /// LOC283070 | 1.52061 | 9.22035E-05 |
| HDAC9 | 1.56346 | 9.31902E-05 |
| WT1 | -1.52969 | 9.37583E-05 |
| CMPK2 | 1.60157 | 9.46544E-05 |
| SERPINH1 | 1.65676 | 9.67787E-05 |
| CAPN6 | 1.62011 | 0.00010357 |
| FLNA | 1.50644 | 0.00010568 |
| PRSS3 | -1.52475 | 0.000108476 |
| DKK3 | 1.51087 | 0.000117202 |
| RUNX3 | 1.54606 | 0.000128211 |
| LOC100132891 | 1.5901 | 0.000128519 |
| AQP11 | -1.57835 | 0.00013752 |

(continued)

| IFTA vs TX | Fold Changes | p-values |
|---|---|---|
| LOC388588 | -1.5056 | 0.000138318 |
| HS6ST2 | -1.80972 | 0.000140022 |
| GABBR1 | 1.58018 | 0.000140037 |
| CRTAM | 1.52125 | 0.000140069 |
| SUSD2 | -1.51355 | 0.000142327 |
| DPP4 | -1.67197 | 0.000145216 |
| SFXN2 | -1.6002 | 0.000145357 |
| C12orf64 | -2.01929 | 0.000155923 |
| PROZ | -1.80974 | 0.000157604 |
| FCGR3B | 1.55481 | 0.00015864 |
| ACSL4 | 1.50161 | 0.000161869 |
| DUSP5 | 1.54175 | 0.000163447 |
| GPR18 | 1.60959 | 0.000166523 |
| ESPL1 | -1.7324 | 0.000171456 |
| ATP6V1G3 | -1.77982 | 0.000172096 |
| DNAJC3 | 1.58767 | 0.00017247 |
| S100A9 | 1.54746 | 0.000178741 |
| CD27 | 1.64479 | 0.000180879 |
| MUC1 | 1.63077 | 0.000186774 |
| GALNT3 | 1.51347 | 0.000188942 |
| MTHFD2 | 1.57956 | 0.00019287 |
| TTC36 | -1.52589 | 0.000195258 |
| P2RY8 | 1.53511 | 0.000195337 |
| EPB41L3 | -1.52238 | 0.00020416 |
| ANK2 | -1.64784 | 0.000204429 |
| LOC100131781 | -2.9471 | 0.000204454 |
| ABCC2 | -1.68245 | 0.000207988 |
| HEPACAM2 | -1.84159 | 0.000209517 |
| NTM | 1.51173 | 0.000212033 |
| KLRC1 /// KLRC2 | 1.63195 | 0.000220071 |
| KRT19 | 1.55043 | 0.000225436 |
| KYNU | -1.8178 | 0.000228908 |
| VEPH1 | -1.62722 | 0.000229217 |
| SPTLC1 | -1.64623 | 0.000231038 |
| TARP | 1.62228 | 0.000243027 |
| ARSF | -1.50712 | 0.000250355 |
| PNPLA3 | -1.62818 | 0.000251061 |
| AMDHD1 | -1.6455 | 0.000254162 |

(continued)

| IFTA vs TX | Fold Changes | p-values |
|---|---|---|
| TBC1D10C | 1.5601 | 0.000256812 |
| SELP | 1.52502 | 0.000258767 |
| RCSD1 | 1.57125 | 0.000261223 |
| TMEM178 | -1.58308 | 0.000263195 |
| SLC6A19 | -1.82272 | 0.000275501 |
| P2RY14 | 1.59513 | 0.000279279 |
| APOC3 | -1.53424 | 0.000300535 |
| ACOX2 | -1.62014 | 0.000301637 |
| FHL2 | 1.52656 | 0.000311563 |
| ALOX5AP | 1.60741 | 0.000314792 |
| C15orf48 | 1.65767 | 0.000314854 |
| C10orf128 | 1.59052 | 0.000317657 |
| PRELP | 1.60495 | 0.000320644 |
| CELF2 | 1.52413 | 0.000324376 |
| ATP6V0D2 | -1.75411 | 0.000325699 |
| MAP7 | -1.79697 | 0.000334732 |
| SLC23A3 | -1.54687 | 0.000334785 |
| NPR3 | -1.79863 | 0.000368992 |
| CRISPLD2 | 1.53183 | 0.000389441 |
| RALYL | -1.74543 | 0.00039422 |
| CTH | -1.55662 | 0.000396559 |
| SLC4A9 | -1.52135 | 0.000411691 |
| GEM | 1.54158 | 0.000427531 |
| SELE | 1.57885 | 0.00043229 |
| WNT5A | 1.52321 | 0.000435362 |
| ARPC3 | 1.66106 | 0.000439626 |
| CYBB | 1.51692 | 0.00045065 |
| RGS1 | 1.56699 | 0.000457779 |
| PRNP | -2.36306 | 0.000488807 |
| IGSF11 | -1.63702 | 0.000509764 |
| GK /// GK3P | -1.55526 | 0.000515407 |
| LOC1518 | -1.51858 | 0.000515767 |
| SLC5A11 | -1.60913 | 0.000521049 |
| RMND1 | -1.55838 | 0.000540732 |
| HPGD | -1.78558 | 0.00054091 |
| DHDH | -1.71486 | 0.000544292 |
| NAT8B | -1.55811 | 0.000549575 |
| SAR1B | -1.64073 | 0.000558645 |

(continued)

| IFTA vs TX | Fold Changes | p-values |
|---|---|---|
| C4orf31 | -1.85662 | 0.000571234 |
| RDH12 | -2.01518 | 0.000572799 |
| SAMSN1 | 1.60145 | 0.000591216 |
| C1S | 1.50415 | 0.000607667 |
| FLJ42875 | -1.60547 | 0.000608022 |
| CCDC3 | 1.57889 | 0.000623944 |
| DDN | -1.50715 | 0.000626257 |
| GNB5 | -1.55044 | 0.000634195 |
| SOST | -2.11137 | 0.000644151 |
| LPPR1 | -1.82253 | 0.000649757 |
| CFH | 1.6367 | 0.000669139 |
| MME | -1.64596 | 0.000674042 |
| LOC100287237 | 1.56531 | 0.000764834 |
| SRGN | 1.54084 | 0.00076549 |
| KNG1 | -1.62639 | 0.000769626 |
| AGXT2L1 | -2.02162 | 0.00077628 |
| GBP1 | 1.60205 | 0.000778436 |
| SPON1 | 1.56225 | 0.000788929 |
| CFH /// CFHR1 | 1.52603 | 0.000790894 |
| LY75 | 1.61206 | 0.000791747 |
| HIBADH | -1.79186 | 0.000811983 |
| MAPT | -1.60132 | 0.000852554 |
| TACSTD2 | 1.52097 | 0.000874577 |
| PTTG1 | 1.51809 | 0.00091356 |
| BASP1 | 1.54985 | 0.000923589 |
| GPRC5A | 1.52408 | 0.000927775 |
| PRKX /// PRKY | 1.54463 | 0.000970822 |
| MTTP | -1.69593 | 0.000995856 |
| TNKS2 | 1.51761 | 0.001002234 |
| NR1D2 | 1.55627 | 0.001020233 |
| PRODH2 | -1.74304 | 0.001020309 |
| FAM134C | 1.5638 | 0.001050607 |
| C1orf186 | 1.60148 | 0.001110359 |
| MYL9 | 1.55261 | 0.001139867 |
| TPD52 | -1.70752 | 0.001169747 |
| FAM151A | -1.97601 | 0.001205059 |
| ZGPAT | -1.57946 | 0.001246363 |
| ACOT7 | -1.528 | 0.001258945 |

(continued)

| IFTA vs TX | Fold Changes | p-values |
|---|---|---|
| FH | -1.68361 | 0.00125963 |
| SORD | -1.56419 | 0.001267712 |
| PAH | -1.90254 | 0.001272156 |
| MUC13 | -1.69478 | 0.001290676 |
| NUDT6 | -1.5431 | 0.001345321 |
| SLC28A2 | -1.52768 | 0.001385666 |
| OSTalpha | -1.60696 | 0.001399651 |
| TMEM174 | -1.65713 | 0.001454055 |
| TNC | 1.60863 | 0.001486039 |
| PBLD | -1.55582 | 0.001556271 |
| AGXT | -1.75973 | 0.001590847 |
| DDAH1 | -1.6755 | 0.001592385 |
| CHI3L1 | -1.65385 | 0.001602868 |
| FOS | 1.59586 | 0.001625845 |
| BAX | 1.60078 | 0.001631275 |
| CES1 | 1.63045 | 0.001669311 |
| TYRP1 | -1.82362 | 0.001697884 |
| SLC10A2 | -1.73028 | 0.001716763 |
| TD02 | 1.63563 | 0.001726221 |
| NEURL1B | 1.56331 | 0.001726312 |
| CTXN3 | -1.79282 | 0.001743693 |
| DMGDH | -1.51241 | 0.001749636 |
| NFAT5 | 1.59118 | 0.001753736 |
| TUBAL3 | -1.51042 | 0.001804062 |
| IGFBP6 | 1.58017 | 0.001901916 |
| DIO1 | -1.59848 | 0.001910455 |
| SLC23A1 | -1.52549 | 0.001946197 |
| THY1 | -1.53788 | 0.001996268 |
| PSAT1 | -1.69764 | 0.002002125 |
| DAO | -1.60066 | 0.002066006 |
| PM20D1 | -1.71127 | 0.002086353 |
| CCL3 /// CCL3L1 /// CCL3L3 | 1.51353 | 0.002087392 |
| KMO | -1.60343 | 0.002283512 |
| IFI44L | 1.64407 | 0.0023117 |
| CRYAA | -1.80094 | 0.002325131 |
| OSTBETA | -1.58545 | 0.002365191 |
| LOC100288332 /// LOC100288583 /// NPIPL3 | 1.62547 | 0.002388231 |
| PLG | -1.78244 | 0.002461498 |

(continued)

| IFTA vs TX | Fold Changes | p-values |
|---|---|---|
| LPL | -1.66864 | 0.00246504 |
| CCR7 | 1.51168 | 0.002557407 |
| LRRC28 | -1.56336 | 0.002620124 |
| RRAD | 1.53482 | 0.002741312 |
| MUC15 | -1.59237 | 0.002741823 |
| IFI6 | 1.61934 | 0.002829231 |
| DDC | -1.7423 | 0.002861605 |
| CRHBP | -1.99859 | 0.002945064 |
| CYP2B6 /// CYP2B7P1 | -1.75265 | 0.002949884 |
| DNAJC12 | -1.64575 | 0.00299662 |
| HIRA | 1.51289 | 0.003063139 |
| CCL20 | 1.60658 | 0.003165424 |
| LOC145837 | -1.7347 | 0.003171816 |
| FGF1 | -1.64515 | 0.003348491 |
| ARHGDIB | 1.51724 | 0.003359731 |
| GPHN | -1.54106 | 0.003390414 |
| C9orf66 | -1.51569 | 0.003831676 |
| PTPRO | -1.52077 | 0.004034566 |
| UPP2 | -1.74057 | 0.00418584 |
| GSN | -1.6508 | 0.00438635 |
| SLC34A1 | -1.53144 | 0.004434079 |
| PCOLCE2 | -1.58643 | 0.004936761 |
| NELL2 | 1.50929 | 0.005435169 |
| IFT57 | -1.60347 | 0.005534224 |
| ASPN | -1.54129 | 0.005627179 |
| GPC5 | -1.62746 | 0.006502554 |
| TAC1 | 1.62375 | 0.00651702 |
| VAMP3 | -1.58292 | 0.006552971 |
| LPA /// PLG | -1.76434 | 0.006796348 |
| ADAMDEC1 | 1.50784 | 0.006940972 |
| CXCL13 | 1.55288 | 0.007006212 |
| GPR34 | 1.58029 | 0.007177428 |
| SLC7A13 | -2.2753 | 0.007781262 |
| NCRNA00182 | 1.65305 | 0.009416748 |
| CNN1 | 1.5022 | 0.009933538 |
| SRPX | 1.60599 | 0.010174115 |
| ITLN1 | -1.80647 | 0.01035312 |
| CYP4F2 /// CYP4F3 | -1.58922 | 0.01326078 |

(continued)

| IFTA vs TX | Fold Changes | p-values |
|---|---|---|
| UGT1A8 /// UGT1A9 | -1.69177 | 0.018037058 |
| CMYA5 | -1.51105 | 0.020198567 |
| OGN | -1.60803 | 0.022700207 |
| PDGFRL | -1.53918 | 0.02458871 |
| LEFTY1 | 1.66065 | 0.031306922 |
| MYH8 | -1.53175 | 0.038086016 |
| TNNC1 | -1.86111 | 0.042271383 |
| MFAP5 | -1.61249 | 0.043993201 |

**Clauses:**

1. A method of prognosing, detecting, diagnosing or monitoring a kidney transplant rejection or injury, or lack thereof in a subject, comprising:

(a) obtaining nucleic acids of interest, wherein the nucleic acids of interest comprise mRNA extracted from a sample from a subject or nucleic acids derived from the mRNA extracted from the sample from the subject;
(b) detecting expression levels in the subject of the at least five genes selected from the at least one of Tables 7, 8, 9, 10, and 11, using the nucleic acids of interest obtained in step (a); and
(c) prognosing, detecting, diagnosing or monitoring the kidney transplant rejection or injury, or lack thereof in the subject from the expression levels detected in step (c).

2. The method of clause 1, further comprising contacting the nucleic acids of interest with probes, wherein the probes are specific for the at least five genes selected in step (b).
3. The method of clause 1, wherein the sample from the subject is a biopsy sample.
4. The method of clause 1, wherein the subject has acute rejection (AR), acute dysfunction no rejection (ADNR), chronic allograft nephropathy (CAN), or well-functioning normal transplant (TX).
5. The method of clause 1, wherein for each of the at least five genes, step (c) comprises comparing the expression level of the gene in the subject to one or more reference expression levels of the gene associated with AR, ADNR, CAN, or TX.
6. The method of clause 5, wherein step (c) further comprises for each of the at least five genes assigning the expression level of the gene in the subject a value or other designation providing an indication whether the subject has AR, ADNR, CAN, or TX.
7. The method of clause 6, wherein the expression level of each of the at least five genes is assigned a value on a normalized scale of values associated with a range of expression levels in kidney transplant patients with AR, ADNR, CAN, or TX.
8. The method of clause 7, wherein the expression level of each of the at least five genes is assigned a value or other designation providing an indication that the subject has or is at risk of AR, ADNR, CAN, has well-functioning normal transplant, or that the expression level is uninformative.
9. The method of clause 6, wherein step (c) further comprises combining the values or designations for each of the genes to provide a combined value or designation providing an indication whether the subject has or is at risk of AR, ADNR, CAN, or has TX.
10. The method of clause 9, wherein the method is repeated at different times on the subject.
11. The method of clause 9, wherein the subject is receiving a drug, and a change in the combined value or designation over time provides an indication of the effectiveness of the drug.
12. The method of clause 1, wherein the subject has undergone a kidney transplant within 1 month, 3 months, 1 year, 2 years, 3 years or 5 years of performing step (a).
13. The method of clause 1, wherein step (b) is performed on at least 10, 20, 40, or 100 genes.
14. The method of clause 1, further comprising changing the treatment regime of the subject responsive to the prognosing, detecting, diagnosing or monitoring step.

15. The method of clause 14, wherein the subject has received a drug before performing the methods, and the changing the treatment regime comprises administering an additional drug, administering a higher dose of the same drug, administering a lower dose of the same drug or stopping administering the same drug.

16. The method of clause 1, wherein the subject is prognosed or diagnosed to have AR, have ADNR, have CAN, or have TX, and wherein the at least five genes are selected from the at least one of Tables 7, 8, 9, 10, and 11.

17. The method of clause 1, wherein expression levels are determined at the mRNA level or at the protein level.

18. The method of clause 1, wherein step (c) is performed by a computer.

19. An array, comprising a support or supports bearing a plurality of nucleic acid probes complementary to a plurality of mRNAs fewer than 5000 in number, wherein the plurality of mRNAs includes mRNAs expressed by at least five genes selected from the at least one of Tables 7, 8, 9, 10 and 11.

20. The array of clause 19, wherein the plurality of mRNAs are fewer than 1000 or fewer than 100 in number.

21. The array of clause 19, wherein the plurality of nucleic acid probes are attached to a planar support or to beads.

22. An array, comprising a support or supports bearing a plurality of ligands that specifically bind to a plurality of proteins fewer than 5000 in number, wherein the plurality of proteins includes at least five proteins encoded by genes selected from the at least one of Tables 7, 8, 9, 10, and 11.

23. The array of clause 22, wherein the plurality of proteins are fewer than 1000 or fewer than 100 in number.

24. The array of clause 22, wherein the plurality of ligands are attached to a planar support or to beads.

25. The array of clause 22, wherein the ligands are different antibodies, wherein the different antibodies bind to different proteins of the plurality of proteins.

26. A method of expression analysis, comprising determining expression levels of up to 5000 genes in a sample from a subject having a kidney transplant, wherein the genes include at least five genes selected from the at least one of Tables 7, 8, 9, 10, and 11.

27. The method of clause 26, wherein the expression levels of up to 100 or 1000 genes are determined.

28. The method of clause 26, wherein the expression levels are determined at the mRNA level or at the protein level.

29. The method of clause 26, wherein the expression levels are determined by quantitative PCR, hybridization to an array or RNA sequencing.

30. A method of screening a compound for activity in inhibiting or treating a kidney transplant rejection or injury, comprising:

    (a) administering the compound to a subject having or at risk of developing a kidney transplant rejection;
    (b) determining expression levels of at least five genes in the subject selected from the at least one of Tables 7, 8, 9, 10, and 11, and species variants thereof before and after administering the compound to the subject; and
    (c) determining whether the compound has activity in inhibiting or treating the kidney transplant rejection from a change in expression levels of the genes after administering the compound.

31. The method of clause 30, wherein the kidney transplant rejection or injury is AR, ADNR, or CAN.

32. The method of clause 30, wherein step (c) comprising for each of the at least five changes assigning a value or designation depending on whether the change in the expression level of the gene relative to one or more reference levels indicating presence or absence of the kidney transplant rejection.

33. The method of clause 30, further comprising determining a combined value or designation for the at least five genes from the values or designations determined for each gene.

34. The method of clause 30, wherein the subject is human or a nonhuman animal model of the kidney transplant rejection.

## Claims

1. A method of prognosing, detecting, diagnosing or monitoring a kidney transplant rejection or injury, or lack thereof in a subject, comprising:

    (a) obtaining nucleic acids of interest, wherein the nucleic acids of interest comprise RNA from a sample from a subject or cDNA reverse-transcribed from the RNA from the sample from the subject;
    (b) detecting expression levels in the subject of the at least five genes selected from the at least one of Tables 7, 8, 9, 10, and 11, using the nucleic acids of interest obtained in step (a); and
    (c) prognosing, detecting, diagnosing or monitoring the kidney transplant rejection or injury, or lack thereof in the subject from the expression levels detected in step (c).

2. The method of claim 1, further comprising contacting the nucleic acids of interest with probes, wherein the probes are specific for the at least five genes selected in step (b).

3. The method of claim 1, wherein the sample from the subject is a biopsy sample.

4. The method of claim 1, wherein the subject has acute rejection (AR), acute dysfunction no rejection (ADNR), chronic allograft nephropathy (CAN), or well-functioning normal transplant (TX).

5. The method of claim 1, wherein for each of the at least five genes, step (c) comprises comparing the expression level of the gene in the subject to one or more reference expression levels of the gene associated with AR, ADNR, CAN, or TX.

6. The method of claim 5, wherein step (c) further comprises for each of the at least five genes assigning the expression level of the gene in the subject a value or other designation providing an indication whether the subject has AR, ADNR, CAN, or TX.

7. The method of claim 6, wherein the expression level of each of the at least five genes is assigned a value on a normalized scale of values associated with a range of expression levels in kidney transplant patients with AR, ADNR, CAN, or TX.

8. The method of claim 7, wherein the expression level of each of the at least five genes is assigned a value or other designation providing an indication that the subject has or is at risk of AR, ADNR, CAN, has well-functioning normal transplant, or that the expression level is uninformative.

9. The method of claim 6, wherein step (c) further comprises combining the values or designations for each of the genes to provide a combined value or designation providing an indication whether the subject has or is at risk of AR, ADNR, CAN, or has TX.

10. The method of claim 9, wherein the method is repeated at different times on the subject.

11. The method of claim 9, wherein the subject is receiving a drug, and a change in the combined value or designation over time provides an indication of the effectiveness of the drug.

12. The method of claim 1, wherein the subject has undergone a kidney transplant within 1 month, 3 months, 1 year, 2 years, 3 years or 5 years of performing step (a).

13. The method of claim 1, wherein step (b) is performed on at least 10, 20, 40, or 100 genes.

14. The method of claim 1, wherein the subject is prognosed or diagnosed to have AR, have ADNR, have CAN, or have TX, and wherein the at least five genes are selected from the at least one of Tables 7, 8, 9, 10, and 11.

15. The method of claim 1, wherein expression levels are determined at the mRNA level or at the protein level, and/or wherein the expression levels of up to 100 or 1000 genes are determined.

# Figure 1

110. Obtain sample from a transplant recipient

120. Perform assay to determine gene expression level

130. Apply computer algorithm to the gene expression level

140. Classification based on the results

Class A    Class B    Class C

**Figure 2**

210.
Transplant
recipient

220.
Sample

230.
Assay

240.
Analysis

280.
Doctor

260.
Home

290.
Other use

250.
Internet

270.
Mobile App

**Figure 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 48116714
- US 2014054735 W
- US 62029038
- US 62001889
- US 62001902
- US 62001909
- US 063603 A1
- WO 9710365 A
- WO 9727317 A
- US 5578832 A
- US 5631734 A
- US 5210015 A, Gelfand
- US 5538848 A, Livak
- US 5863736 A, Haaland
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- WO 2008048970 A
- US 5458852 A
- WO 2007104537 A
- WO 2009060035 A
- US 20040126767 A
- WO 2004059293 A
- US 6785613 B
- WO 0238561 A
- WO 0382859 A
- WO 04052359 A
- WO 05066156 A

**Non-patent literature cited in the description**

- Academic Press Dictionary of Science and Technology. Academic Press, 1992
- Illustrated Dictionary of Immunology. 2002
- Oxford Dictionary of Biochemistry and Molecular Biology. Oxford University Press, 2000
- Encyclopaedic Dictionary of Chemistry. Anmol Publications Pvt. Ltd, 2002
- Dictionary of Microbiology and Molecular Biology. John Wiley & Sons, 2002
- Dictionary of Chemistry. 1999
- Dictionary of Pharmaceutical Medicine. Springer-Verlag Telos, 1994
- Dictionary of Organic Chemistry. Anmol Publications Pvt. Ltd, 2002
- A Dictionary of Biology (Oxford Paperback Reference). Oxford University Press, 2000
- **NIELSEN et al.** *Science,* 1991, vol. 254, 1497-1500
- Theory and Nucleic Acid Preparation. Molecular Biology: Hybridization With Nucleic Acid Probes. Elsevier, 1993
- **LOCKHART et al.** *Nature Biotechnology,* 1996, vol. 14, 1675-1680
- **LIPSCHUTZ et al.** *Nature Genetics Supplement,* 1999, vol. 21, 20-24
- **SCHENA et al.** *Science,* 1995, vol. 270, 467-470
- **DERISI et al.** *Nature Genetics,* 1996, vol. 14, 457-460
- **C.A. et al.** *Genome Research,* 1996, vol. 6, 986-994
- **GIBSON, U.E.M et al.** *Genome Research,* 1996, vol. 6, 995-1001
- **HOLLAND, P. M. et al.** *Proc. Natl. Acad. Sci. USA,* 1991, vol. 88, 7276-7280
- **LIVAK, K.J. et al.** *PCR Methods and Applications,* 1995, 357-362
- **ANGLICHEAU et al.** *PNAS,* 2009, vol. 106, 5330-5335
- **HALLORAN et al.** *Am. J. Transplant,* 2013, vol. 13 (11), 2865-74
- **HALLORAN et al.** *Am. J. Transplant,* 2013, vol. 13 (9), 2352-63
- **MANNON et al.** *Kidney International,* 1999, vol. 55, 1935-1944